# EUROPEAN PATENT APPLICATION

(11) **EP 1 800 684 A1**
(43) Date of publication of application: **27.06.2007**
(21) Application number: 05027823.3
(22) Date of filing: 20.12.2005
(51) Int. Cl.: A61K 31/661, A61K 31/675, A61K 31/683, A61K 31/685, A61P 31/00, A61P 31/04, A61P 31/06, A61P 31/08, A61P 31/10, A61P 31/12, A61P 31/14, A61P 31/16, A61P 31/18, A61P 31/20, A61P 31/22, A61P 33/00, A61P 33/02, A61P 33/04, A61P 33/06, A61P 33/08, A61P 33/12, A61P 35/00

(54) **Novel alkyl phospholipid derivatives and uses thereof**

(71) Applicant: Zentaris GmbH, 60314 Frankfurt/Main (DE)
(72) Inventor: Perrissoud, Daniel, 61348 Bad Homburg (DE); Pietras, Mathias, 61279 Grävenwiesbach (DE); Engel, Jürgen, Prof. Dr., 63755 Alzenau (DE)

(57) **Abstract**

The present invention provides novel alkyl phospholipid derivatives that are useful for treating various diseases including tumors and/or pathophysiological conditions in mammals, preferably humans, that are caused by microorganisms, in particular fungi, protozoa, bacteria and/or viruses. Such alkyl phospholipids can be employed as single drugs or in the course of combination therapies, in particular for the treatment of leishmaniasis, trypanosomiasis and/or malaria.

## Description

### Technical field

The invention relates to novel alkyl phospholipid derivatives that are useful in the treatment of various diseases and/or pathophysiological conditions in mammals caused by microorganisms, in particular fungi, protozoa, bacteria and/or viruses. Such alkyl phospholipids can be employed as single drugs or in combination therapies and may also exhibit anti-neoplastic properties.

### Prior art

Alkyl phospholipids (APL) as a substance class have been known for several decades to possess properties and exhibit biological activity that can advantageously be exploited for treatment in various medicinal indications.

A comprehensive overview of different effects and uses of for instance alkyl phosphocholines is given in Drugs of Today, Vol. 34, Suppl. F, 1998.

Further literature with regard to alkyl phospholipids, their various uses as well as relevant indications (including respective standard therapies) comprise the following.

EP 0 108 565 discloses alkyl phosphocholines that are claimed to possess anti-neoplastic properties. WO 87/03478 describes alkyl phospholipids for use as anti-tumor medicaments. US 5,219,866 describes octadecyl-[2-(N-methylpiperidino)-ethyl]-phosphate as useful for the treatment of cancer as well as a process for its preparation. US 6,172,050 and US 6,479,472 both disclose specific phospholipid derivatives and methods of using them as therapeutics, in particular against tumors. US 5,449,798 and US 5,958,906 are directed to phospholipid derivatives containing higher elements of the fifth group that are said to act as anti-neoplastic. US 6,093,704 describes the use of dopamine receptor antagonists in palliative tumor therapy reducing potential side effects of alkyl phosphocholines, such as miltefosine. WO 2004/012744 relates to the use of alkyl phosphocholines in combination with antitumor medicaments.

EP 0 108 565 discloses alkyl phosphocholines that are claimed among others to have anti-fungal properties. Lu et al. describe the use of the natural bisphosphocholine irlbacholine and synthetic analogues thereof as antifungal agents (Lu Q et al., J. Nat. Prod. 1999, 62(6):824-828). Ganendren and co-workers studied for compounds with structural similarities to phospholipid substrates as inhibitors of phospholipases from the fungal pathogen Cryptococcus neoformans (Ganendren R et al., Antimicrob. Agents Chemother. 2004, 48(5):1561-1569).

Protozoal diseases persist to be a worldwide burden. For most of these diseases curing therapies are not available. Leishmaniasis and Chagas disease are two prominent members of this class of diseases.

Leishmaniasis ranks third in the WHO neglected diseases list in terms of fatality numbers per year. With annually 60.000 cases of death only Malaria and tuberculosis cause more victims. It is estimated that worldwide 350 million people are at risk and currently 12 million people are infected. The disease is found in 88 countries of the new and old world with most infected people living in India, Bangladesh, Brazil and Sudan. Each year, 1-1.5 million new cases are reported. The DALY burden has been reported by WHO/TDR to be 860.000 for men and 1.200.000 for women.

Leishmaniasis is caused by protozoa of the genus Leishmania which are transmitted by sand flies (Phlebotomus sp. and Lutzomyia sp.). Two forms of the disease exist. Internal or visceral leishmaniasis is the most dangerous form and causes death within 6-12 month if untreated. The other form, called cutaneous leishmaniasis, leads to lesions in the skin and if untreated to ulcers. In cases were no spontaneous healing occurs it results in scars on body and face. Possible complications of untreated leishmaniasis comprise secondary infections of ulcer and development of the mucocutaneous form which can result in destruction of facial skin and mucosal parts.

The visceral form is found in the old (Leishmania species: L. donovani, L. infantum) and new (L. chagasi) world. It affects the subindian continent (India, Bangladesh and Nepal), parts of East Africa (Sudan and Ethiopia) and parts of South America (Brazil and Colombia). The number of new cases per year is 500.000 with a high rate of mortality. Visceral leishmaniasis is associated with fever, weight loss, enlargement of spleen and liver. If it is not treated it tends to be fatal.

The cutaneous form is widespread in both worlds. In the old world, Leishmania major and Leishmania tropica are the prominent pathogens. L. major is found in rural areas whereas L. tropica is found in urban areas. The main countries are Afghanistan, Pakistan and the whole Middle East, especially Iran, Iraq, Syria and Saudi-Arabia.

Currently, there is a tendency to leave the patients untreated because scars and painful ulcers are considered as non-life threatening. However, it is more likely to be the high risk of side effects caused by current treatment that could explain the wait- and-see position. In the new world the situation is more dangerous. Patients suffering from cutaneous leishmaniasis may develop the mucocutaneous form which leads to painful and disfiguring disruption of parts of the face. The disease is found all over Middie and South America with focal points in Venezuela, Peru, Bolivia and Guatemala.

The current standard therapy has to be given parenterally in hospitals and is highly toxic. AIDS and other immunosuppressive conditions such as malnutrition increase the risk. As a matter of fact, in many of the countries where visceral Leishmaniasis prevails they are also hot spots for AIDS and malnutrition. The current treatment options are limited. Since 50 years parenteral antimonials (sodium stiboglucanant, SSG, Pentostam^{™} and meglumine stiboglucanat Glucantime^{™}) have been the standard therapy for Leishmaniasis. The side effects are very serious including vomiting, nausea, diarrhoea and anorexia. Creatine values have to be observed and ECG values have to be monitored during the whole course of treatment and a regular ECG monitoring is required.

The alternative and second line treatment amphotericin B has its advantage in the absence of resistance. However, in addition to the high cost for hospitalization there is a higher drug price. Side effects are similarly serious with additional drug-induced fevers and the drug is only approved for visceral leishmaniasis. A further alternative to overcome side effects is the use of liposomal amphotericin B. The drug paromomycin currently undergoes clinical phase III trials and it is reported that it is efficient in 95% of the cases and generally well tolerable All treatments are parenteral and require a longer period of hospitalization.

Compared to Leishmaniasis, for Chagas disease the situation is much more problematic. Chagas disease, also called American trypanosomiasis, is caused by the parasite protozoon Trypanosoma cruzi. It is endemic in 21 countries of South and Latin America. Currently 16-18 million people are infected and 100 million people are at risk. The disease is transmitted via blood-sucking insects the parasites being transmitted from the gastric-enteral path while the insect is incorporating blood of its victim.

In human the disease starts with an acute phase followed by a life-long chronic phase. In the acute phase it is sometimes associated with fever, swelling of lymph glands, enlargement of the liver and spleen as well as local inflammations. Because the acute phase is often not correctly if at all diagnosed, the infection stays untreated. As a consequence, Chagas disease enters the chronic phase which is characterized by the parasite's penetration of the heart muscle, severe local inflammation and chronic heart disease. Usually, patients die early on because of these cardiac diseases.

[General literature on Chagas Disease: Guzman-Bracho C, Trends Parasitol. 2001, 17(8):372-376; Roberts A et al., J. Am. Acad. Nurse Pract. 2001, 13(4):152-153; Tarleton RL et al., Parasitol. Today. 1999, 15(3):94-99; Anez N et al., Mem. Inst. Oswaldo Cruz 2004, Rio de Janeiro, 99(8): 781-787; Second Report of the WHO Expert Committee, WHO technical report series 905, WHO 2002; Behbehani K, Bull World Health Organ. 1998, 76 (Suppl. 2):64-67; Umezawa ES et al., Lancet. 2001, 357(9258):797-799].

So far the treatment options have been limited. There are only two drugs approved for Chagas disease: Benznidazole (Radanil^{™}) and Nifurtimox (Lampit^{™}). They are very toxic and their use is limited to the acute phase of the disease only. Only poor evidence has been reported that at least one of the two drugs is of some efficiency in the chronic phase.

Currently, the drug of choice is Benznidazole which is given at 5-7 mg per kg bodyweight for 60 days. Side effect can be very severe and request an immediate notification to the physician. Very common side effects reported are convulsions (seizures), numbness, tingling pain, weakness in hands or feet, reddish discoloration of skin, abdominal or stomach pain, diarrhoea, nausea and vomiting. Rare side effects are fever or chills, pinpoint red spots on skin, skin rash, sore throat, unusual bleeding or bruising, confusion, dizziness, headache, restlessness, temporary loss of memory, trouble in sleeping, difficulty in concentrating, unusual tiredness or weakness.

The older substance Nifurtimox is much less in use than Benzidazole. It has to be given for 50 days at 8-10 mg per kg bodyweight. Known side effects are abdominal or stomach pain, dizziness, headache, loss of appetite, nausea, vomiting, weight loss, skin rash, chills or sore throat, clumsiness or unsteadiness, confusion, convulsions (seizures), decreased sexual drive or ability, fever, forgetfulness, irritability, mood or mental changes, muscle weakness, numbness, tingling, pain, weakness in hands or feet, trembling, trouble in sleeping, uncontrolled back-and-forth and/or rolling eye movements, unusual excitement, nervousness and restlessness.

[Literature on Chagas disease (standard) treatment: Coura JR et al., Mem. Inst. Oswaldo. Cruz 2002, Rio de Janeiro, 97(1): 3-24; Docampo R, Curr. Pharm. Des. 2001, 7(12):1157-1164; Kayser O et al., Pharm. Unserer Zeit. 1999, 28(4):177-185; Cerecetto H et al., Curr. Top. Med. Chem. 2002, 2(11):1187-1213; Urbina JA, Curr. Opin. Infect. Dis. 2001, 14(6):733-741; Paulino M et al., Mini-Reviews in Medicinal Chemistry 2005, 5: 499-519; Campos RF et al., Rev. Soc. Bras. Med. Trop. 2005, 38(2):142-146; Garcia S et al., Antimicrob. Agents Chemother. 2005, 49(4):1521-1528; Schenone H et al., Rev. Méd. Chile 2003; 131:1089-1090; Marcondes MC et al., Microbes Infect. 2000, 2(4):347-352; Corrales M et al., Antimicrob. Agents Chemother. 2005, 49(4):1556-1560; Urbina JA et al., Int. J. Antimicrob. Agents 2003, 21(1):39-48; Maya JD et al., Biochem. Pharmacol. 2003, 65(6):999-1006; Lockman JW et al., Curr. Med. Chem. 2005, 12(8):945-959].

The use of alkyl phospholipids in protozoal diseases, in particular Leishmaniasis and Chagas disease has also been reported. Miltefosine has been shown to be as high effective in the treatment of Leishmaniasis as currently used amphotericin B. It has been registered as the first oral drug in several countries for cutaneous and visceral leishmaniasis. However, there is still a need to improve therapy schemes and efficacy in the course of Leishmaniasis treatment. There is an increasing danger that due to the long metabolic half-life period of the drug and the long lasting therapy course of 28 days - which may not be completely followed by the patients - development of drug resistance will occur. Alkyl phospholipids have also been shown in preclinical test to be active in acute phase *in vivo* and *in vitro* against Trypanosoma cruzi.

[Literature on use of APL in Leishmaniasis and Chagas disease: Croft SL et al., Mol. Biochem. Parasitol. 2003, 126(2):165-172; Saraiva VB et al., Antimicrob. Agents Chemother. 2002, 46(11):3472-3477; de Castro SL et al., Mini-Reviews in Medicinal Chemistry 2004, 4:141-151; Berman J, Expert Opin. Pharmacother. 2005, 6(8):1381-1388; Bhattacharya SK et al., Clin. Infect. Dis. 2004, 38(2):217-221; Jha TK et al., N. Engl. J. Med. 1999, 341 (24):1795-1800; Jacobs S, N. Engl. J. Med. 2002, 347(22):1737-1738; Sundar S et al., N. Engl. J. Med. 2002, 347(22):1739-1746; Sindermann H et al., Clin. Infect. Dis. 2004, 39(10):1520-1523; Soto J et al., Clin. Infect. Dis. 2004, 38(9):1266-1272; Soto J et al., Clin. Infect. Dis. 2001, 33(7):E57-61; Sundar S et al., Pediatr. Infect. Dis. J. 2003, 22(5):434-438; Croft SL et al., J. Antimicrob. Chemother. 1996, 38(6):1041-1047; Santa-Rita RM et al., Acta Trop. 2000, 75(2):219-228; Sundar S et al., Lancet. 1998, 352 (9143): 1821-1823; Sundar S et al., Ann. Trop. Med. Parasitol. 1999, 93(6):589-597; Sundar S et al., Clin. Infect. Dis. 2000, 31(4):1110-1113; Lux H et al., Mol. Biochem. Parasitol. 2000, 111(1):1-14; US 5,980,915, US 6,521,879, US 6,506,393; US 2003/0216355].

As for protozoal diseases, in particular Leishmaniasis and Chagas disease, combination therapy approaches have only been rarely reported. The efficacy of picroliv in combination with miltefosine was described by Gupta and co-workers (Gupta S et al., Acta Trop. 2005, 94(1):41-47). A potential anti-proliferative synergy of lysophospholipid analogues and ketoconazole against Trypanosoma cruzi was discussed by Santa-Rita and collaborators (Santa-Rita RM et al., J. Antimicrob. Chemother. 2005, 55(5):780-784). Mechanism of action of anti-proliferative lysophospholipid analogues against the protozoan parasite Trypanosoma cruzi were elucidated by Lira et al. who postulated a potentiation of in vitro activity by the sterol biosynthesis inhibitor ketoconazole (Lira R et al., J. Antimicrob. Chemother. 2001, 47(5):537-546). Araujo and co-workers showed that a combination of benznidazole and ketoconazole enhances efficacy of chemotherapy of experimental Chagas disease (Araujo MS et al., J. Antimicrob. Chemother. 2000, 45(6):819-824).

However, alkyl phospholipid derivatives known in the prior art and uses thereof do show inherent disadvantages. In particular, standard drug medicamentation (APL and others) against fungal, protozoal, bacterial and/or viral diseases implicate several drawbacks, such as resistance of the microorganisms to be targeted, severe side effects caused by the high toxicity of the compounds to be applied and long courses of treatment.

### Description of the invention

The present invention has the object to provide novel alkyl phospholipid derivatives which can be employed for the treatment of diseases or pathophysiological conditions in mammals caused by microorganisms, in particular fungi, protozoa, bacteria and/or viruses. It is another object of the underlying invention to provide novel alkyl phospholipid derivatives that exhibit anti-neoplastic properties and can be used for treating tumors in mammals. A further object of the present invention is to provide novel combine tion therapies of alkyl phospholipid derivatives with suitable known drugs for the treatment of diseases or pathophysiological conditions in mammals caused by microorganisms.

The object of the invention has surprisingly been solved in one aspect by providing alkyl phospholipid derivatives according to formula I 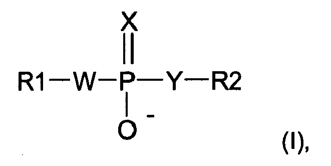 wherein:
W, X, Y independently are selected from the group consisting of: "oxygen atom, sulphur atom";
R1 is "-[(CR3R4)ₘ-Z]ₙ-R5";
R2 is "-(CR6R7)ₚ-R8";
R3 and R4 are independently from each other selected from the group consisting of "hydrogen atom; substituted or unsubstituted C1-C12alkyl, substituted or unsubstituted (C1-C12alkyl)_{q}-A-(C1-C18alkyl)ᵣ, -OH, substituted or unsubstituted -C(O)-(C8-C30alkyl), substituted or unsubstituted -OC(O)-(C8-C30alkyl), substituted or unsubstituted -NHCO-(C1-C12alkyl), substituted or unsubstituted -N(C1-C12alkyl)CO-(C1-C12alkyl)";
or optionally R3 and R4 together form a substituted or unsubstituted saturated, partially unsaturated or aromatic heterocyclic ring system of 3, 4, 5, 6, 7 or 8 ring atoms containing at least one heteroatom selected from the group consisting of: "oxygen atom, sulfur atom";
R5 is independently selected from the group consisting of: "substituted or unsubstituted C8-C30alkyl, substituted or unsubstituted -C(O)-(C8-C30alkyl), substituted or unsubstituted steroid moiety";
R6 and R7 are independently from each other selected from the group consisting of "hydrogen atom, -OH, halogen atom, -F, -Cl, -Br, -I, -CN, C1-C6alkyl";
or optionally R6 and R7 together form a substituted or unsubstituted saturated, partially unsaturated or aromatic ring system of 3, 4, 5, 6 or 7 carbon atoms;
or optionally if p is 1, "-(CR6R7)ₚ-" can also be a substituted or unsubstituted saturated, partially unsaturated or aromatic ring system of 3, 4, 5, 6 or 7 carbon atoms formed together by R6 and R7;
R8 is selected from the group consisting of: "-VR9R10R11; substituted or unsubstituted heterocycle", where the heterocycle is (i) a 5-, 6- or 7-membered saturated, partially unsaturated or aromatic monocyclic carbon atom ring system with at least one heteroatom selected from the group consisting of: "nitrogen atom, oxygen atom, sulphur atom, arsenic atom", and with the proviso that at least one heteroatom is a quaternary nitrogen atom or a quaternary arsenic atom, or (ii) a 7-, 8-, 9-, 10-, 11- or 12-membered saturated, partially unsaturated or aromatic bicyclic carbon atom ring system with at least one heteroatom selected from the group consisting of: "nitrogen atom, oxygen atom, sulphur atom, arsenic atom", and with the proviso that at least one heteroatom is a quaternary nitrogen atom or a quaternary arsenic atom, or (iii) a tropin moiety, where two or more ring atoms of the heterocycle can be additionally linked via an alkylene-bridge, and where the heterocycle if substituted is substituted with at least one R12, but if possible also 2, 3, 4, 5, 6, 7, 8, 9, 10, 11, 12, 13, 14, 15, 16, 17, 18, 19, 20 or more R12, which in case of two or more R12 are independently from each other identical, partly identical or different, and where the heterocycle may be attached with any of the ring atoms;
R9, R10, R11, R12 are independently from each other selected from the group consisting of: "hydrogen atom, substituted or unsubstituted C1-C18alkyl, substituted or unsubstituted C3-C8cycloalkyl, substituted or unsubstituted (C1-C12alkyl)ₛ-B-(C1-C12alkyl)ₜ-C-(C1-C12alkyl)ᵤ, substituted or unsubstituted aryl, substituted or unsubstituted heteroaryl, substituted or unsubstituted alkoxy, -OH, halogen, -F, -Cl, -Br, -I, =O, -C(O)O-(C1-C12alkyl), -C(O)O-(C3-C8cycloalkyl), -C(O)O-aryl, -C(O)O-heteroaryl, -C(O)O-heterocyclyl, -C(0)-(C1-C12alky)), -C(O)-(C3-C8cycloalkyl), -C(O)-aryl, -C(O)-heteroaryl, -C(O)-heterocyclyl", and optionally two substituents R12 can together form a substituted or unsubstituted saturated, partially unsaturated or aromatic ring system of 3, 4, 5, 6 or 7 carbon atoms;
Z is independently selected from the group consisting of "oxygen atom; sulphur atom";
V is independently selected from the group consisting of "nitrogen atom, arsenic atom";
A, B, C are independently from each other selected from the group consisting of "oxygen atom; sulphur atom; S(O₂)";
m independently is 1, 2 or 3;
n independently is 0, 1, 2, 3, 4, 5, 6, 7, 8, 9 or 10 and preferably is 0, 1, 2, or 3;
p independently is 0, 1, 2, 3, 4, 5 or 6, and preferably is 0, 1, 2 or 3;
q, r, s, t, u independently from each other are 0 or 1;
that can be used for the manufacture of a medicament for the treatment or prophylaxis of diseases and/or pathophysiological conditions in mammals that are caused by microorganisms.

If R1 is "-[(CR3R4)ₘ-Z]ₙ-R5" and n is ≥ 2, then for each "-[(CR3R4)ₘ-Z]" group Z, R3 and R4 can be identical, partly identical or different, for instance, "-CH₂-O-CHCH₃-S-CH₂-O-".

In a further preferred embodiment, alkyl phospholipid derivatives according to above formula (I) are provided, where
R1 is R5,
n is 0,
that can be used for the manufacture of a medicament for the treatment or prophylaxis of diseases and/or pathophysiological conditions in mammals that are caused by microorganisms.

In another preferred embodiment, alkyl phospholipid derivatives according to above formula (I) are provided, where
m is 2 or 3,
n is 1 or 2,
that can be used for the manufacture of a medicament for the treatment or prophylaxis of diseases and/or pathophysiological conditions in mammals that are caused by microorganisms.

In another aspect, the object of the invention has surprisingly been solved by providing alkyl phospholipid derivatives selected from the group consisting of:
"**Compound 1:** "Phosphoric acid 1-benzyl-1-methyl-piperidin-4-yl ester hexadecyl ester" (C₂₉H₅₂NO₄P; MW: 509,72) 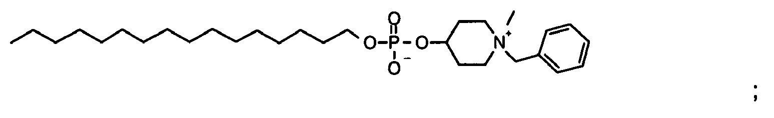
**Compound 2:** "Phosphoric acid 2-(1-methyl-piperidin-1-yl)-ethyl ester hexadecyl ester" (C₂₄H₅₀NO₄P; MW: 447,64) 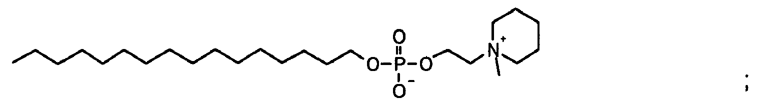
**Compound 3:** "Phosphoric acid 2-(1-aza-bicyclo[2.2.2]oct-1-yl)-ethyl ester hexadecyl ester" (C₂₅H₅₀NO₄P; MW: 459,66) 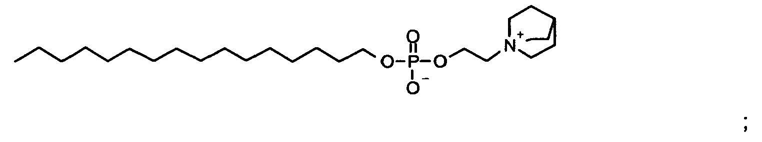
**Compound 4:** "Phosphoric acid 1-methyl-1-phenethyl-piperidin-4-yl ester hexadecyl ester" (C₃₀H₅₄NO₄P; MW: 523,74) 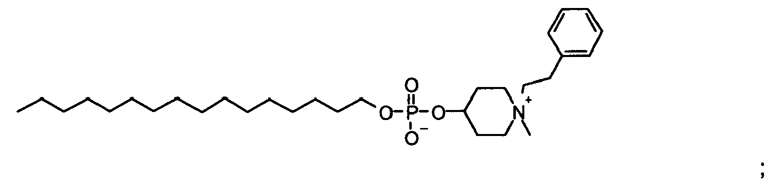
**Compound 5:** "Phosphoric acid 1,1-diethyl-piperidin-4-yl ester octadecyl ester" (C₂₇H₅₆NO₄P; MW: 489,73) 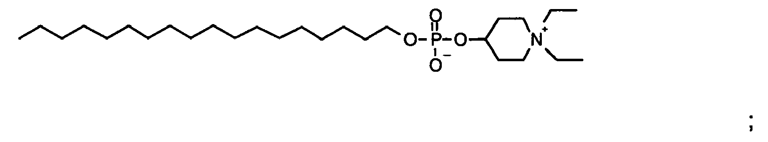
**Compound 6:** "Phosphoric acid 1,1-dimethyl-piperidin-4-yl ester 3-hexadecylsulfanyl-2-methoxymethyl-propyl ester" (C₂₈H₅₈NO₅PS; MW: 551,81) 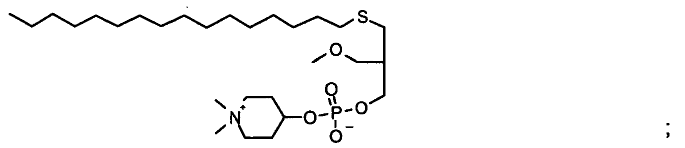
**Compound 7:** "Phosphoric acid 1,1-dimethyl-piperidin-4-yl ester 2-hexadecyloxymethyl-tetrahydro-furan-2-ylmethyl ester" (C₂₉H₅₈NO₆P; MW: 547,75) 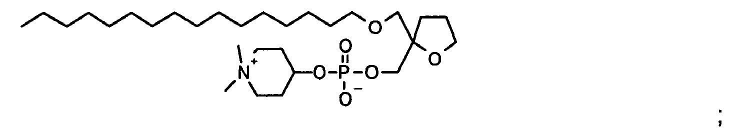
**Compound 8:** "Phosphoric acid 3-hexadecyloxy-propyl ester 2-trimethylammonium-ethyl ester" (C₂₄H₅₂NO₅P; MW: 465,65) 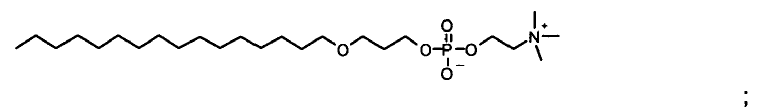
**Compound 9:** "Phosphoric acid 1,1-dimethyl-piperidin-2-ylmethyl ester octadecyl ester" (C₂₆H₅₄NO₄P; MW: 475,69) 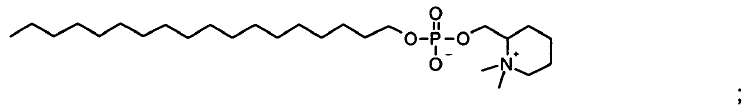
**Compound 10:** "Phosphoric acid 1,1-dimethyl-piperidin-4-yl ester 2-octadecyloxymethyl-tetrahydro-furan-2-ylmethyl ester" (C₃₁H₆₂NO₆P; MW: 575,81) 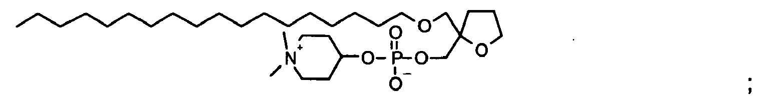
**Compound 11:** "Phosphoric acid 2-trimethylammonium-ethyl ester 2-hexadecyloxymethyl-tetrahydro-furan-2-ylmethyl ester" (C₂₇H₅₆NO₆P; MW: 521,71) 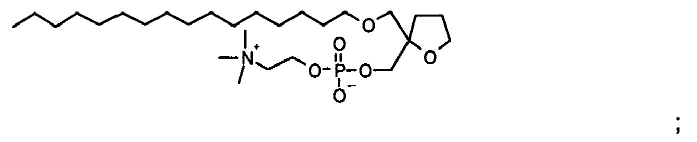
**Compound 12:** "Phosphoric acid 1,1-dimethyl-piperidin-4-yl ester 3-hexadecyloxy-2-methoxy-propyl ester" (C₂₇H₅₆NO₆P; MW: 521,71) 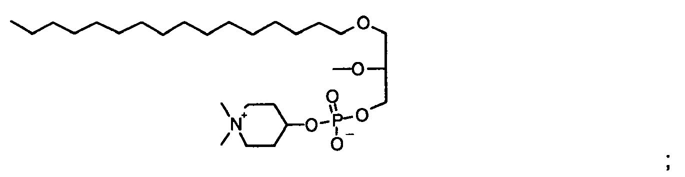
**Compound 13:** "Phosphoric acid 1,1-dimethyl-piperidin-4-yl ester 3-hexadecyloxy-2-methoxymethyl-propyl ester" (C₂₈H₅₈NO₆P; MW: 535,74) 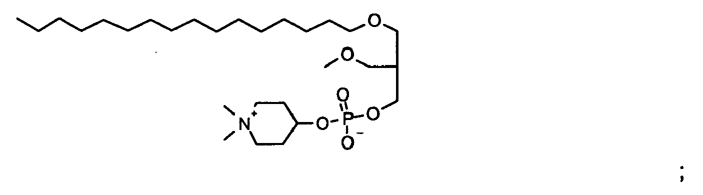
**Compound 14:** "Phosphoric acid 2-trimethylammonium-ethyl ester 2-hexadecyloxy-4-methanesulfonyl-butyl ester" (C₂₆H₅₆NO₇PS; MW: 557,77) 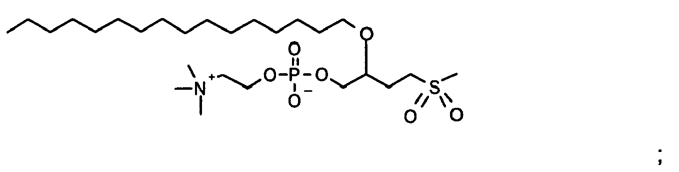
**Compound 15:** "Phosphoric acid 1,1-dimethyl-piperidin-4-yl ester 2-methoxy-3-octadecyloxy-propyl ester" (C₂₉H₆₀NO₆P; MW: 549,78) 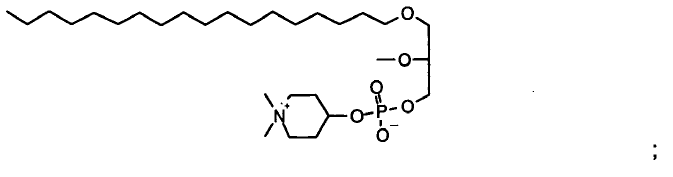
**Compound 16:** "Phosphoric acid 2-trimethylammonium-ethyl ester (Z)-icos-11-enyl ester" (C₂₅H₅₂NO₄P; MW: 461,66) 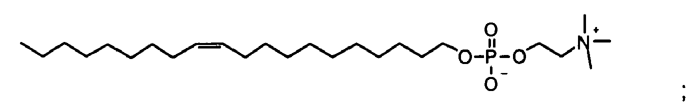
**Compound 17:** "Phosphoric acid 1,1-dimethyl-piperidin-4-yl ester (Z)-docos-13-enyl ester" (C₂₉H₅₈NO₄P; MW: 515,76) 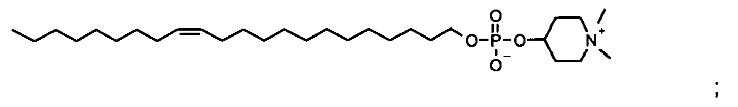
**Compound 18:** "Phosphoric acid 2-triethylammonium-ethyl ester tetradecyl ester" (C₂₂H₄₈NO₄P; MW: 421.61) 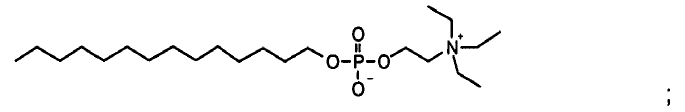
**Compound 19:** "Phosphoric acid (Z)-octadec-9-enyl ester 2-piperidin-1-yl-ethyl ester" (C₂₅H₅₀NO₄P; MW: 459.66) 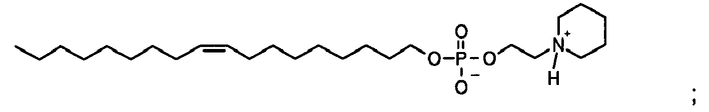
**Compound 20:** "Phosphoric acid 2-(2-decyloxy-ethoxy)-ethyl ester 2-(1-methylpiperidin-1-yl)-ethyl ester" (C₂₂H₄₆NOₛP; MW: 451.59) 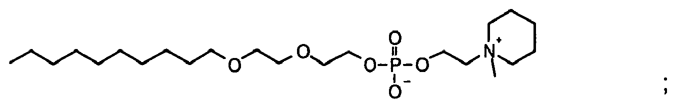
**Compound 22:** "Phosphoric acid octadecyl ester 2-(trimethyl-lambda*5*-arsanyl)-ethyl ester" (C₂₃H₅₀AsO₄P; MW: 496,54) 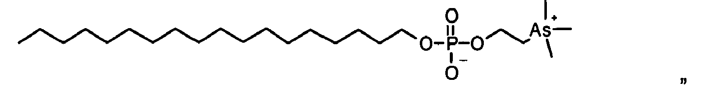
that can be used for the manufacture of a medicament for the treatment or prophylaxis of diseases and/or pathophysiological conditions in mammals that are caused by microorganisms.

Further examples of alkyl phospholipid derivatives that are within the scope and spirit of the present invention are:

| **Example** | **Structure** | **MW** |
|---|---|---|
| 21 | 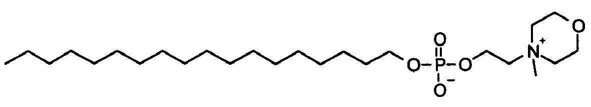 | 477,66507 |
| 23 | 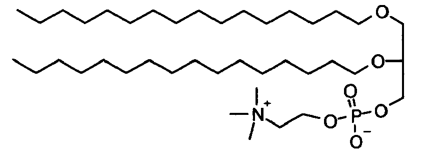 | 706,083252 |
| 24 | 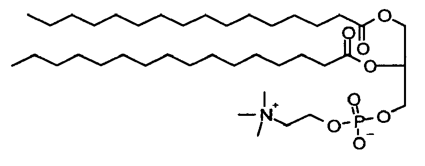 | 734,050354 |
| 25 | 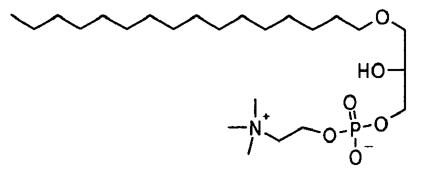 | 481,653564 |
| 26 | 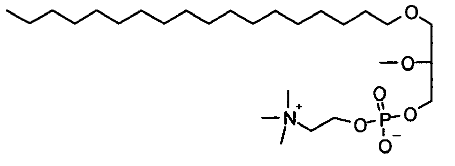 | 523,734192 |
| 27 | 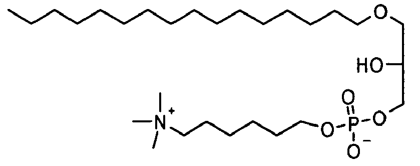 | 537,761108 |
| 28 | 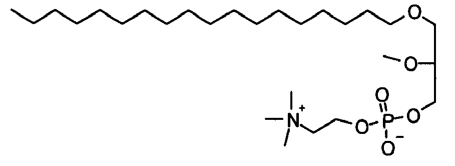 | 523,734192 |
| 29 | 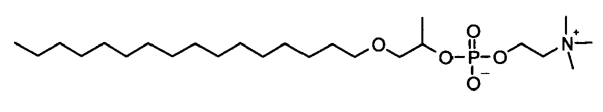 | 465,654022 |
| 30 | 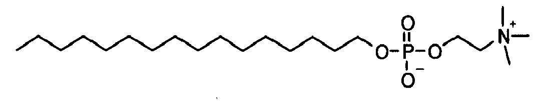 | 407,574097 |
| 31 | 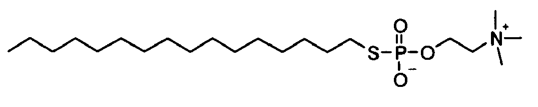 | 423,640686 |
| 32 | 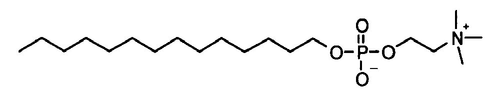 | 379,520416 |
| 33 | 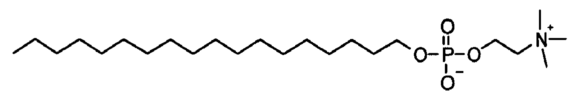 | 435,627838 |
| 34 | 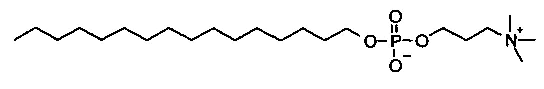 | 421,600983 |
| 35 | 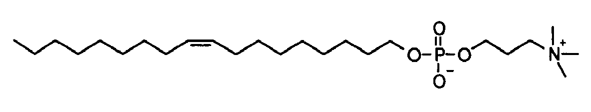 | 447,638855 |
| 36 | 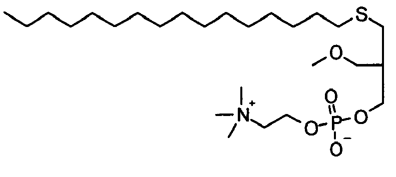 | 525, 773804 |
| 37 | 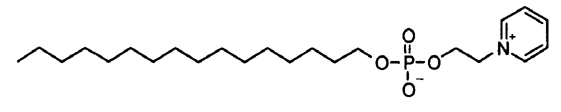 | 427,564392 |
| 38 | 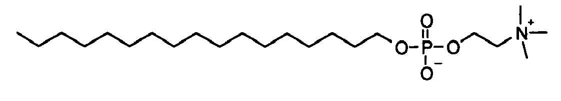 | 421,600983 |
| 39 | 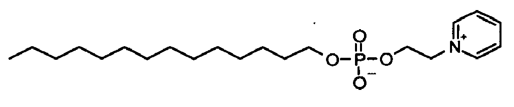 | 399,510681 |
| 40 | 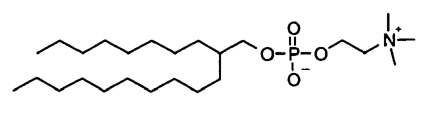 | 463,681641 |
| 41 | 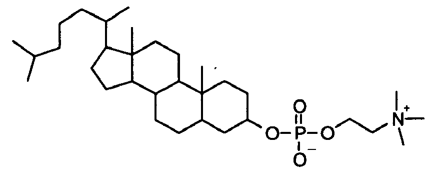 | 553,806274 |
| 42 | 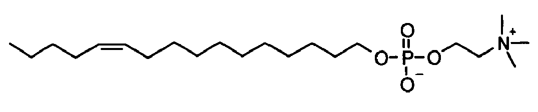 | 405,558319 |
| 43 | 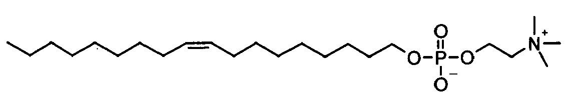 | 433,612 |
| 44 | 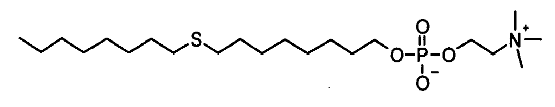 | 439,640137 |
| 45 | 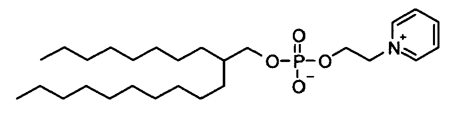 | 483,671844 |
| 46 | 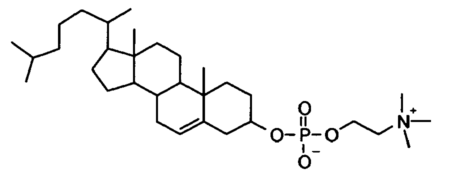 | 551,790405 |
| 47 | 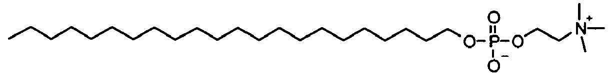 | 491,735291 |
| 48 | 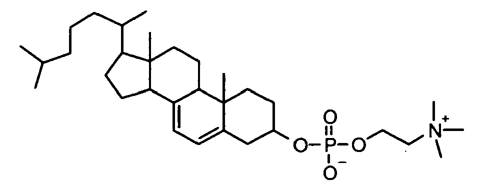 | 549,774536 |
| 49 | 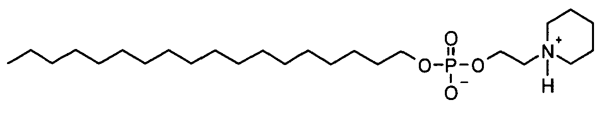 | 461,66568 |
| 50 | 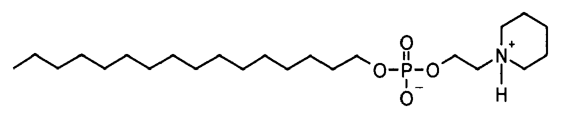 | 433,611969 |
| 51 | 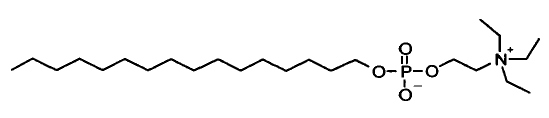 | 449,654663 |
| 52 | 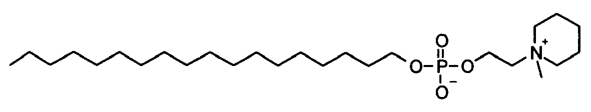 | 475,692505 |
| 53 | 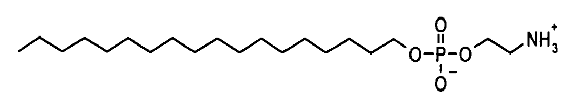 | 393,547272 |
| 54 | 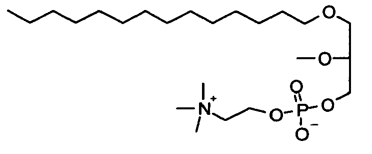 | 467,62674 |
| 55 | 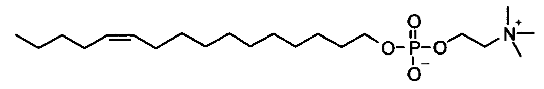 | 431,596069 |
| 56 | 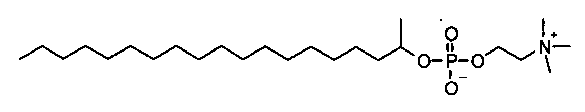 | 449,654694 |
| 57 | 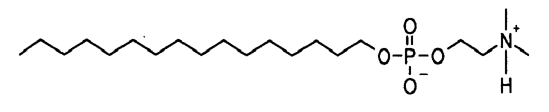 | 393,547241 |
| 58 | 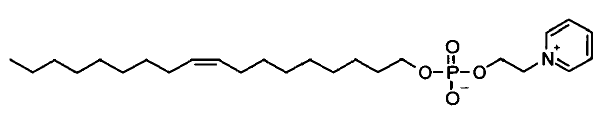 | 453,602142 |
| 59 | 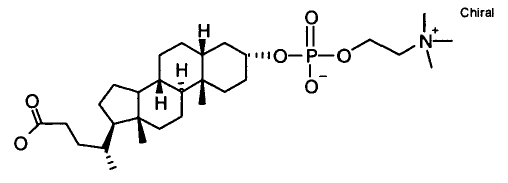 | 541,708496 |
| 60 | 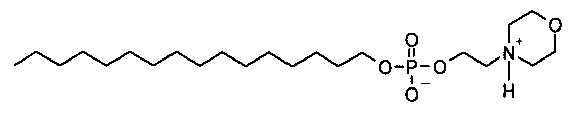 | 435,584473 |
| 61 | 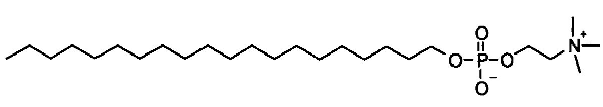 | 463,681549 |
| 62 | 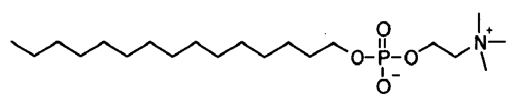 | 393,547241 |
| 63 | 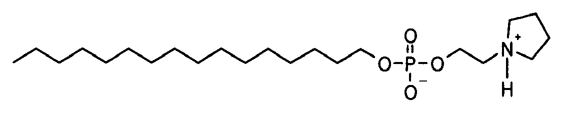 | 419,585083 |
| 64 | 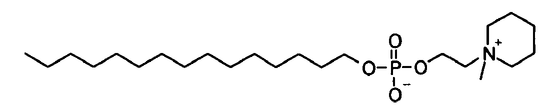 | 433,611938 |
| 65 | 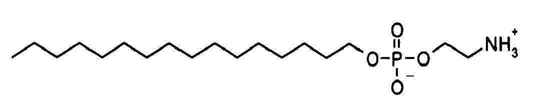 | 365,49353 |
| 66 | 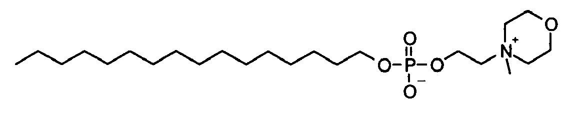 | 449,611328 |
| 67 | 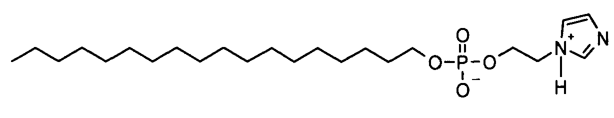 | 444,594849 |
| 68 | 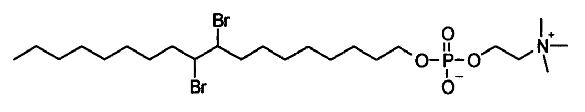 | 593,419861 |
| 69 | 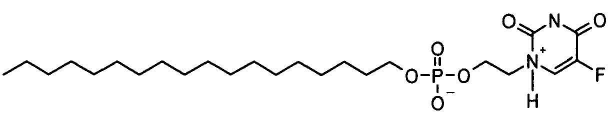 | 506,595093 |
| 70 | 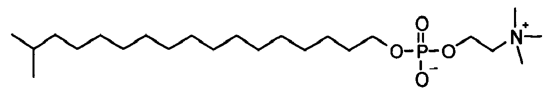 | 435,627838 |
| 71 | 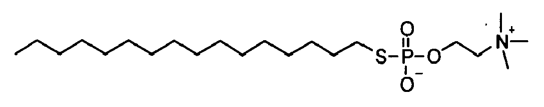 | 423,640686 |
| 72 | 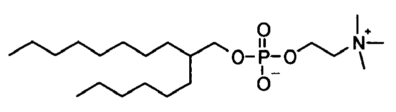 | 407,574158 |
| 73 | 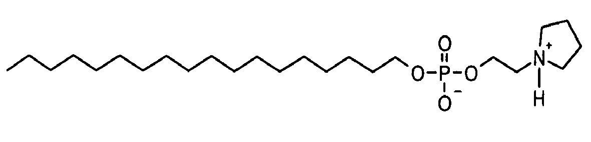 | 447,638794 |
| 74 | 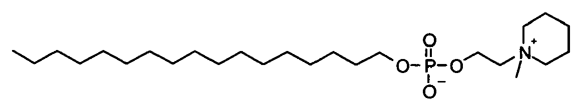 | 461,665649 |
| 75 | 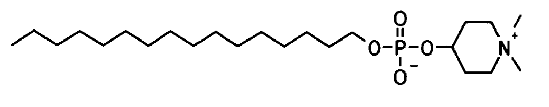 | 433,611969 |
| 76 | 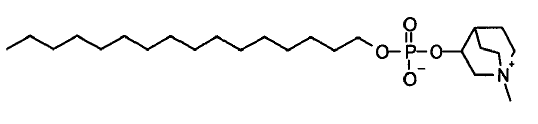 | 445,622894 |
| 77 | 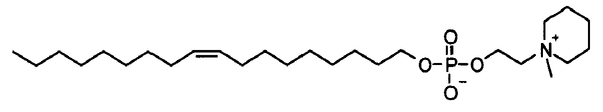 | 473,676636 |
| 78 | 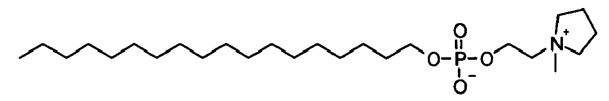 | 461,665649 |
| 79 | 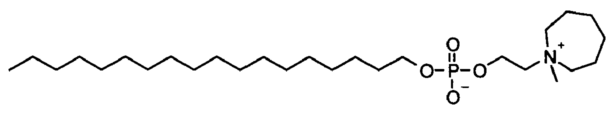 | 489,719421 |
| 80 | 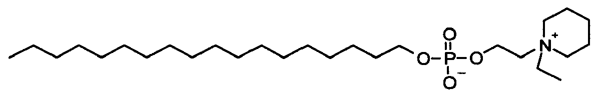 | 489,719421 |
| 81 | 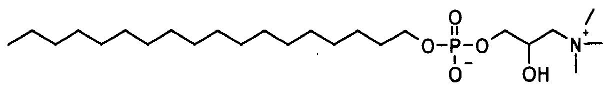 | 465,654053 |
| 82 | 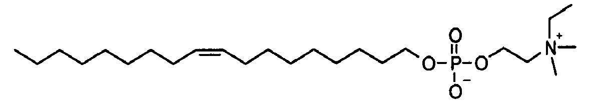 | 447,638885 |
| 83 | 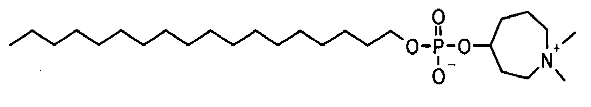 | 475,692535 |
| 84 | 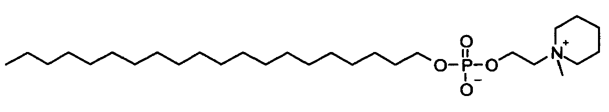 | 503,746307 |
| 85 | 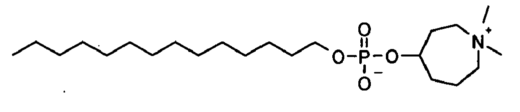 | 419,585114 |
| 86 | 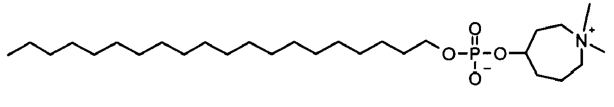 | 503,746246 |
| 87 | 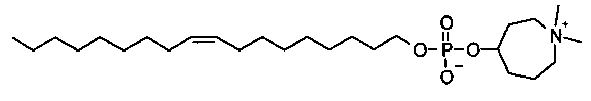 | 473,676758 |
| 88 | 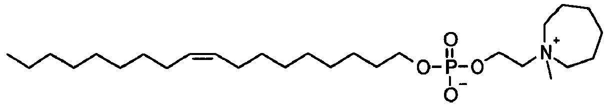 | 487,703522 |
| 89 | 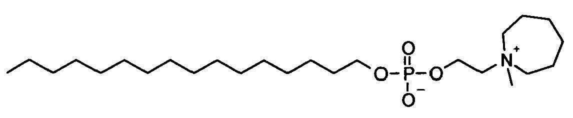 | 461,66568 |
| 90 | 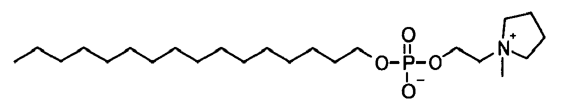 | 433,611938 |
| 91 | 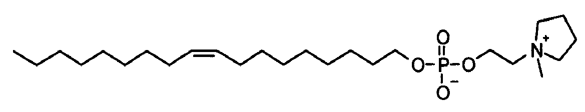 | 459,64978 |
| 92 | 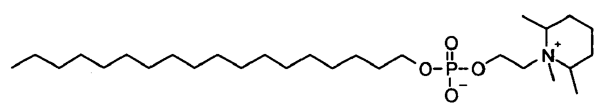 | 503,746277 |
| 93 | 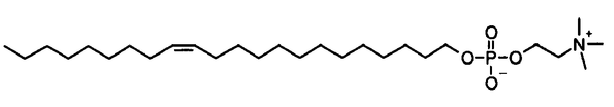 | 489,719574 |
| 94 | 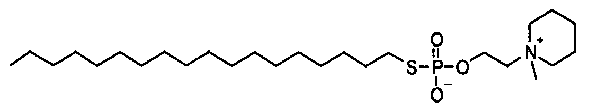 | 491,759094 |
| 95 | 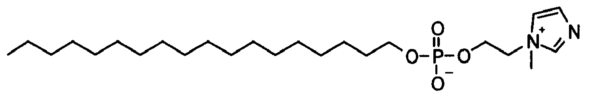 | 458,621704 |
| 96 | 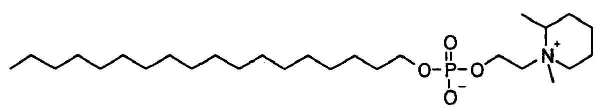 | 489,71936 |
| 97 | 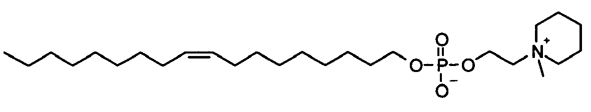 | 473,676636 |
| 98 | 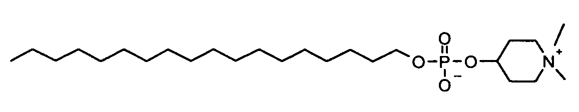 | 461,665802 |
| 99 | 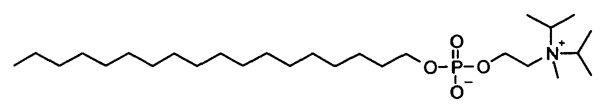 | 491,735291 |
| 100 | 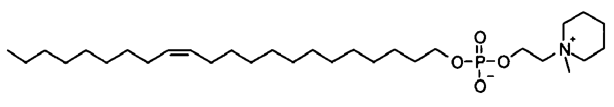 | 529,784241 |
| 101 | 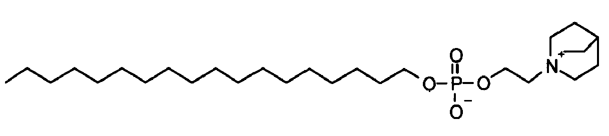 | 487,703522 |
| 102 | 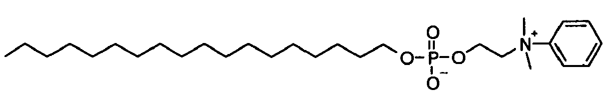 | 497,698669 |
| 103 | 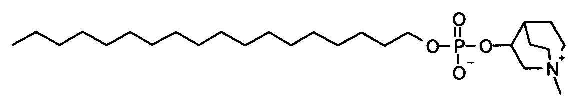 | 473,676636 |
| 104 | 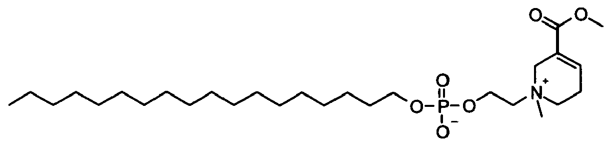 | 531,713318 |
| 105 | 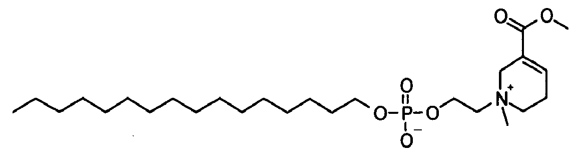 | 503,659576 |
| 106 | 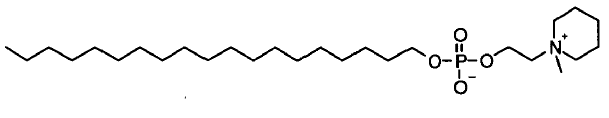 | 489,719421 |
| 107 | 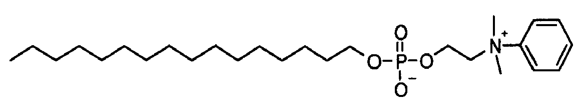 | 469,644897 |
| 108 | 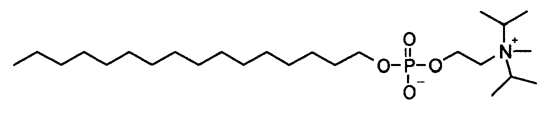 | 463,681519 |
| 109 | 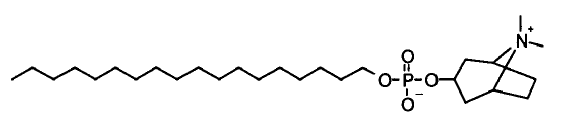 | 487,703522 |
| 110 | 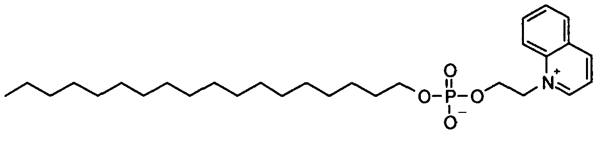 | 505,677917 |
| 111 | 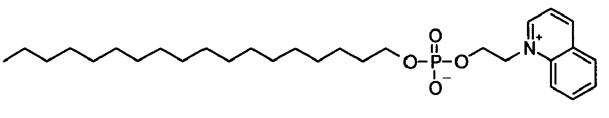 | 505,677856 |
| 112 | 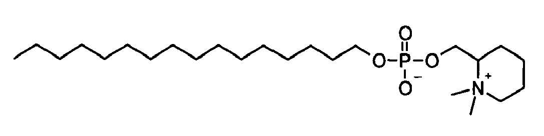 | 447,638794 |
| 113 | 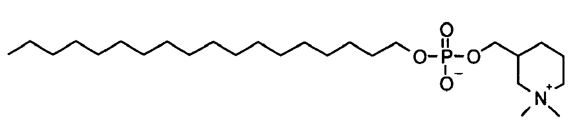 | 475,692535 |
| 114 | 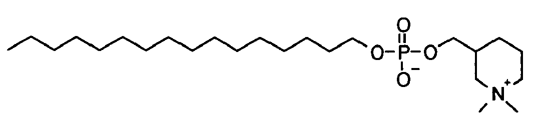 | 447,638794 |
| 115 | 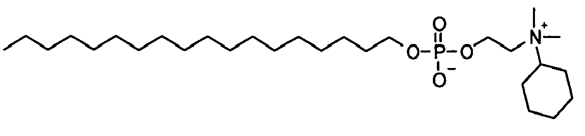 | 503,746277 |
| 116 | 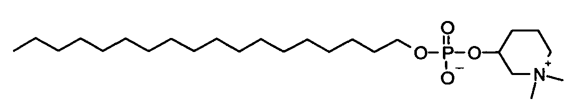 | 461,665649 |
| 117 | 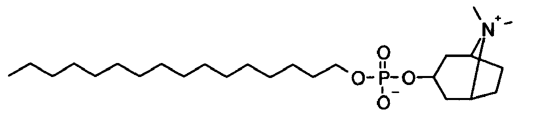 | 459,64978 |
| 118 | 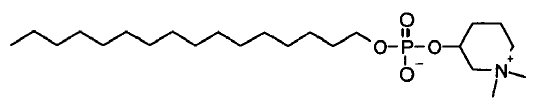 | 433,611938 |
| 119 | 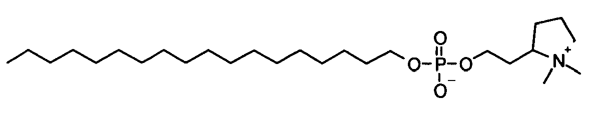 | 475,692535 |
| 120 | 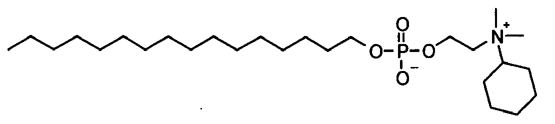 | 475,692535 |
| 121 | 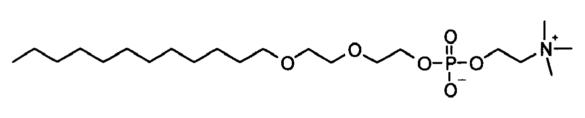 | 439,572906 |
| 122 | 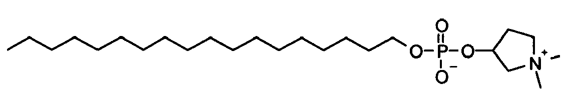 | 447,638824 |
| 123 | 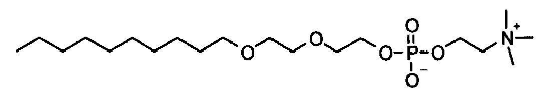 | 411,519165 |
| 124 | 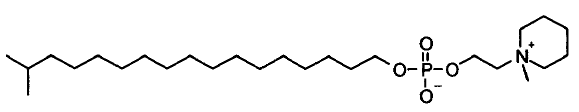 | 475,692535 |
| 125 | 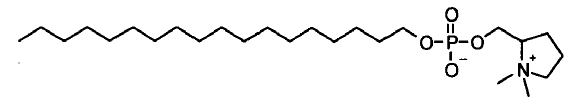 | 461,665649 |
| 126 | 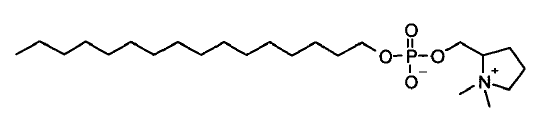 | 433,611938 |
| 127 | 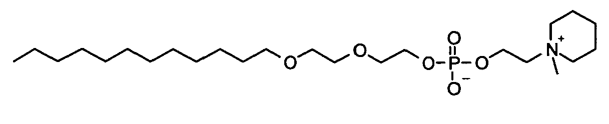 | 479,637604 |
| 128 | 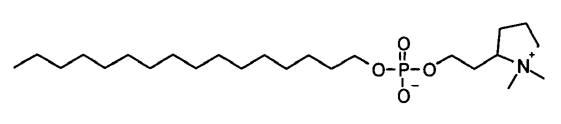 | 447,638794 |
| 129 | 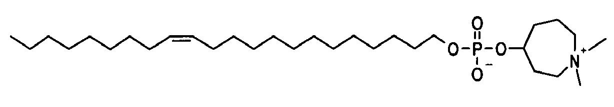 | 529,78418 |
| 130 | 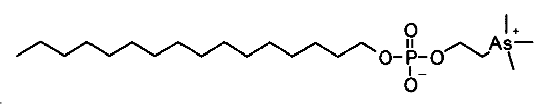 | 468,488861 |
| 131 | 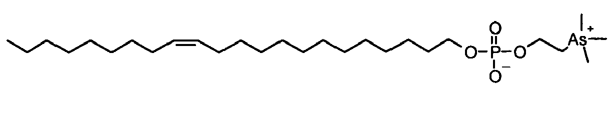 | 550,634399 |
| 132 | 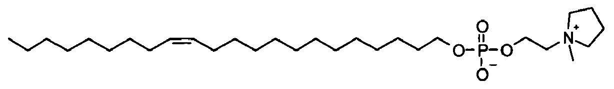 | 515,757324 |
| 133 | 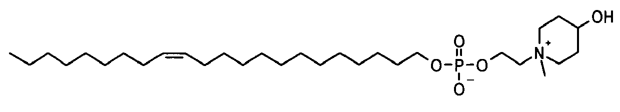 | 545,783569 |
| 134 | 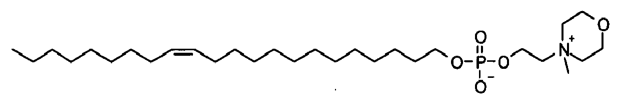 | 531,756714 |
| 135 | 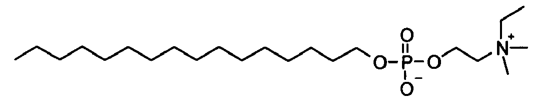 | 421,600983 |
| 136 | 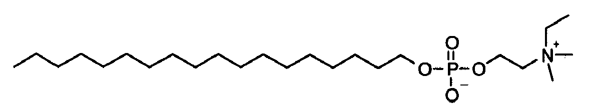 | 449,654694 |
| 137 | 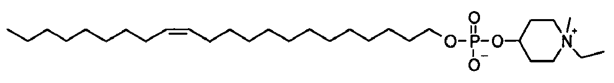 | 529,78418 |
| 138 | 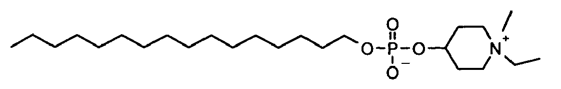 | 447,638794 |
| 139 | 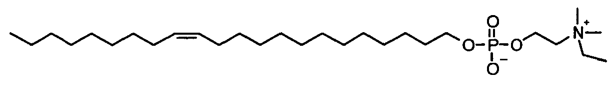 | 503,746429 |
| 140 | 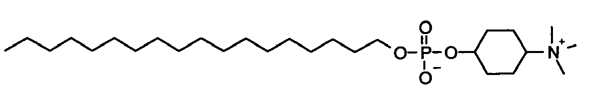 | 489,71936 |
| 141 | 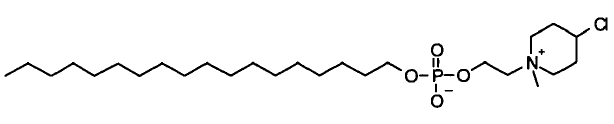 | 510,137329 |
| 142 | 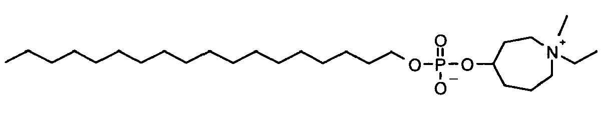 | 489,71936 |
| 143 | 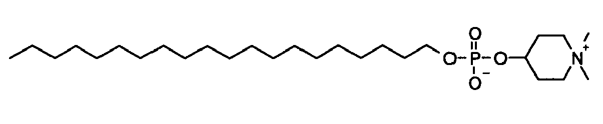 | 489,719513 |
| 144 | 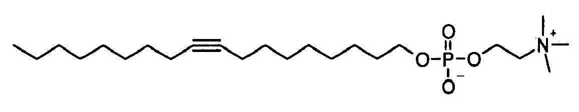 | 431,596283 |
| 145 | 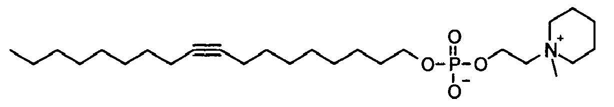 | 471,660889 |
| 146 | 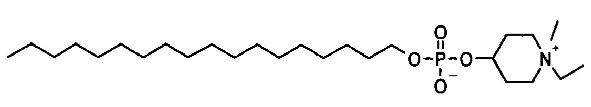 | 475,692505 |
| 147 | 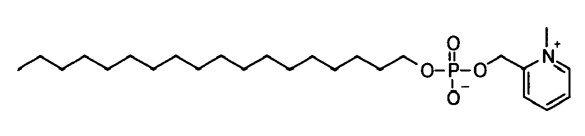 | 455,618042 |
| 148 | 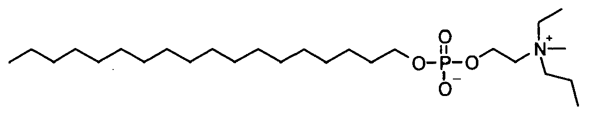 | 477, 708405 |
| 149 | 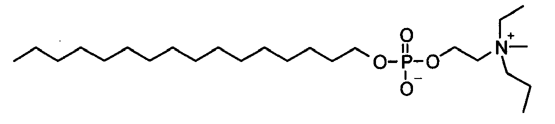 | 449,654663 |
| 150 | 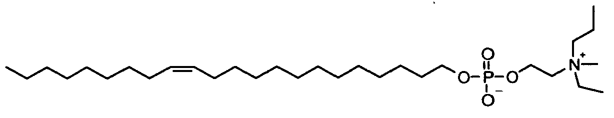 | 531,799988 |
| 151 | 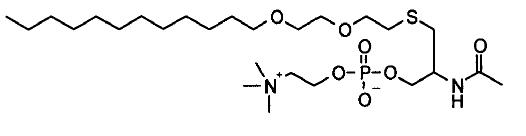 | 570,771484 |
| 152 | 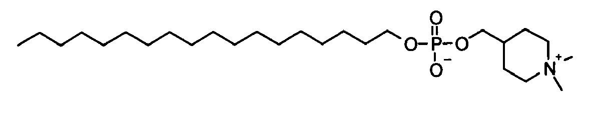 | 475,692688 |
| 153 | 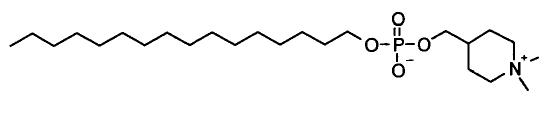 | 447,638916 |
| 154 | 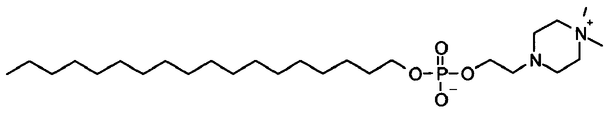 | 490,707306 |
| 155 | 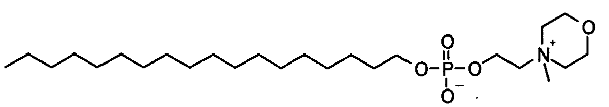 | 477,665039 |
| 156 | 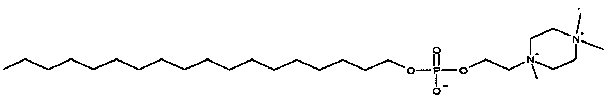 | 676,938599 |
| 157 | 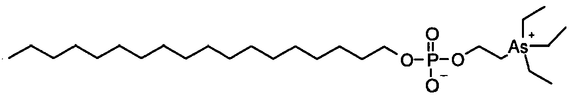 | 538,62323 |
| 158 | 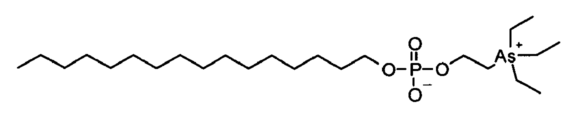 | 510,569458 |
| 159 | 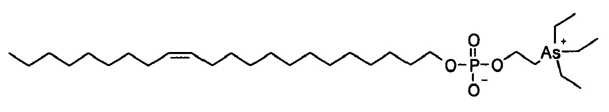 | 592,714722 |
| 160 | 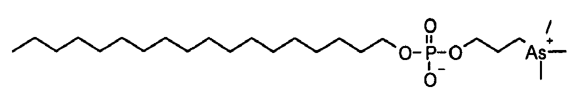 | 510,569641 |
| 161 | 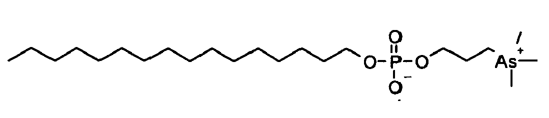 | 482,51593 |
| 162 | 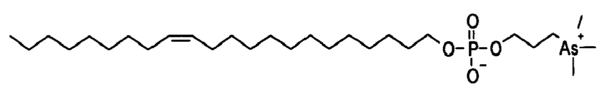 | 564,661255 |
| 163 | 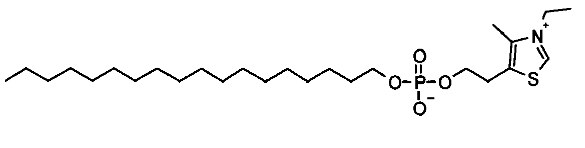 | 503,726746 |
| 164 | 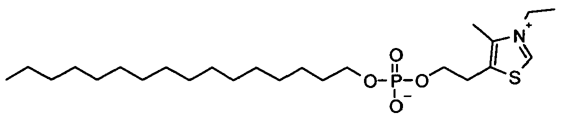 | 475,673035 |
| 165 | 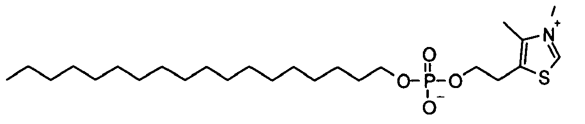 | 489,69986 |
| 166 | 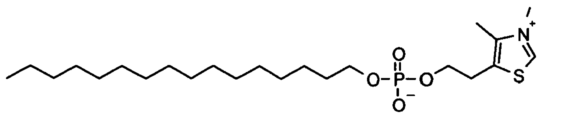 | 461,646179 |
| 167 | 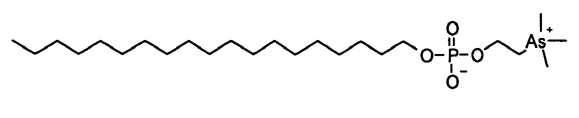 | 510,569458 |
| 168 | 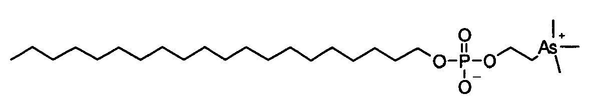 | 524,596375 |
| 169 | 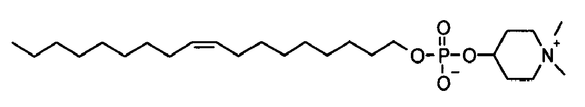 | 459,64978 |
| 170 | 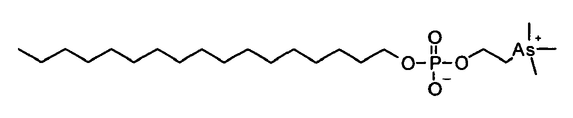 | 482,515686 |
| 171 | 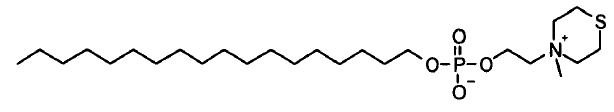 | 493, 731598 |
| 172 | 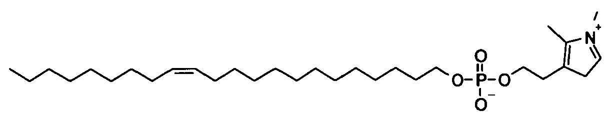 | 525,752441 |
| 173 | 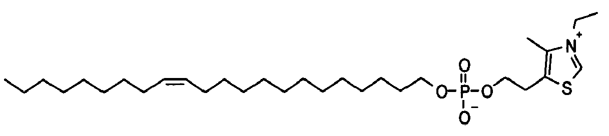 | 557,818298 |
| 174 | 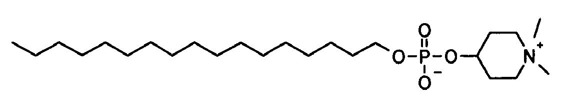 | 447,638824 |
| 175 | 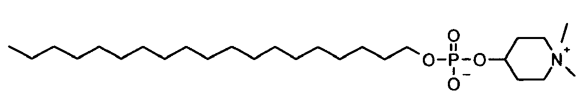 | 475,692505 |
| 176 | 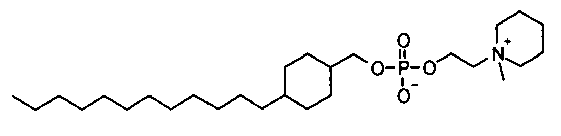 | 487,703644 |
| 177 | 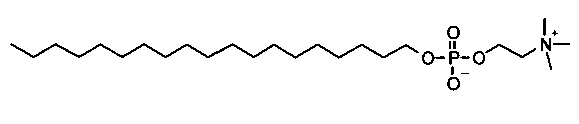 | 449,654694 |
| 178 | 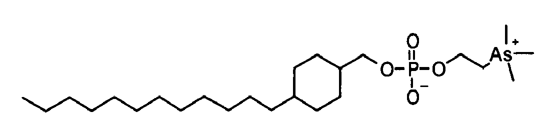 | 508,553864 |
| 179 | 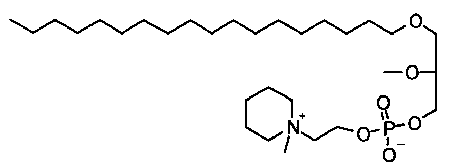 | 563,798828 |
| 180 | 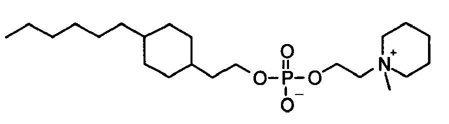 | 417,569336 |
| 181 | 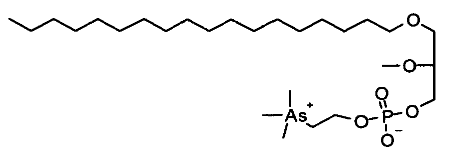 | 584,648865 |
| 182 | 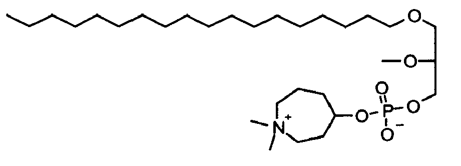 | 563,798828 |
| 183 | 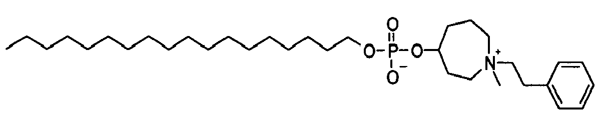 | 565,817139 |
| 184 | 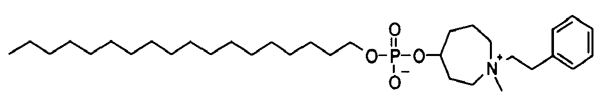 | 565,817139 |
| 185 | 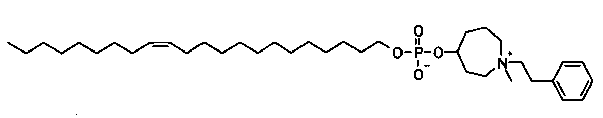 | 619,908691 |
| 186 | 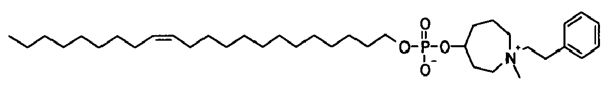 | 619,908691 |
| 187 | 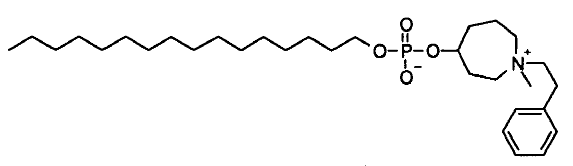 | 537,763428 |
| 188 | 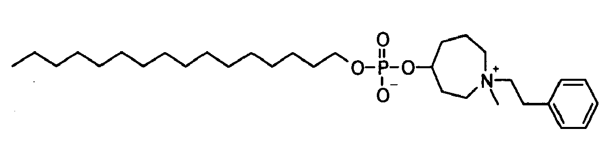 | 537,763428 |
| 189 | 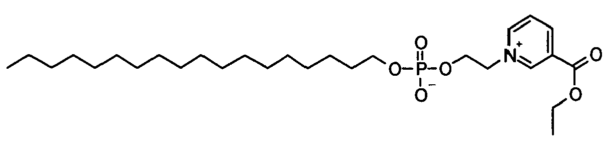 | 527,681519 |
| 190 | 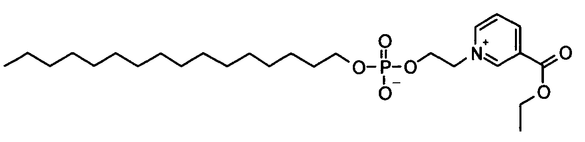 | 499,627808 |
| 191 | 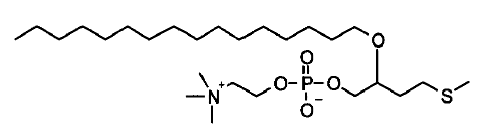 | 525,773804 |
| 192 | 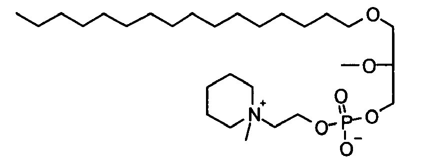 | 535,745117 |
| 193 | 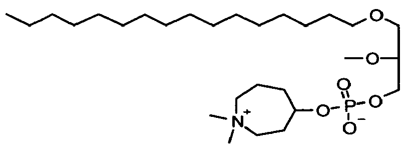 | 535,745117 |
| 194 | 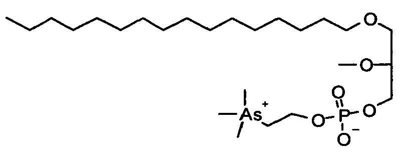 | 556,595154 |
| 195 | 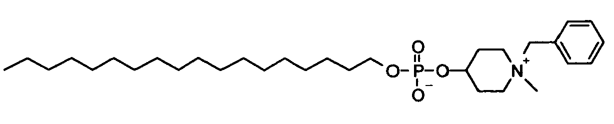 | 537,763306 |
| 196 | 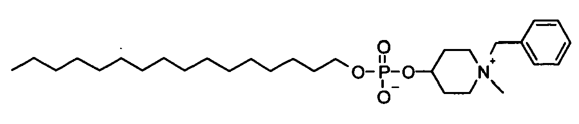 | 509,709595 |
| 197 | 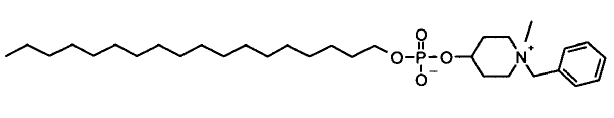 | 537, 763367 |
| 198 | 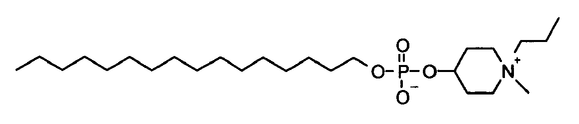 | 461,66568 |
| 199 | 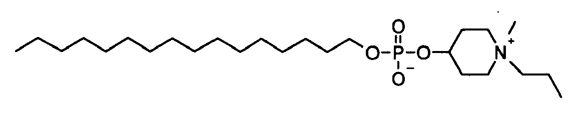 | 461,66568 |
| 200 | 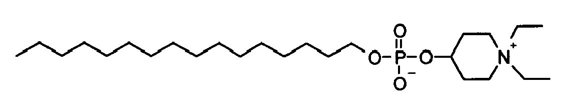 | 461,66568 |
| 201 | 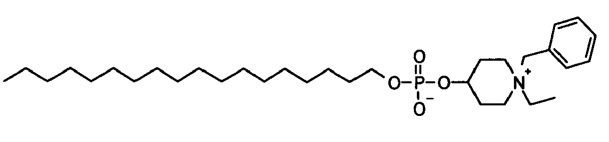 | 551,790222 |
| 202 | 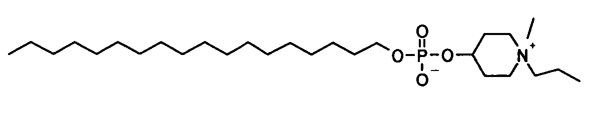 | 489,71936 |
| 203 | 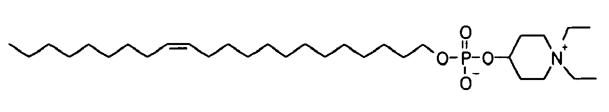 | 543,810974 |
| 204 | 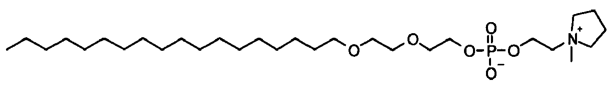 | 549,771973 |
| 205 | 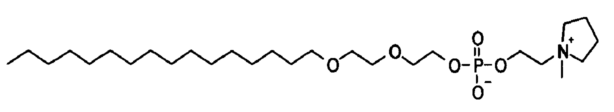 | 521,718201 |
| 206 | 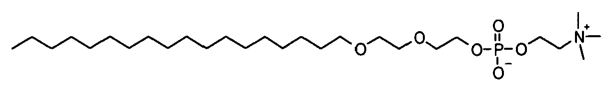 | 523, 73407 |
| 207 | 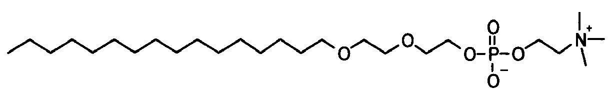 | 495,680298 |
| 208 | 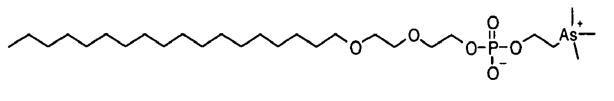 | 584,648804 |
| 209 | 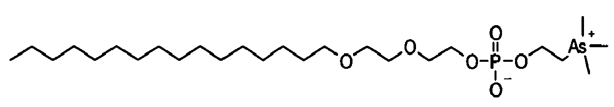 | 556,595154 |
| 210 | 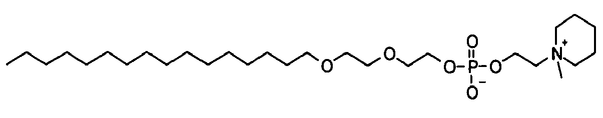 | 535,745117 |
| 211 | 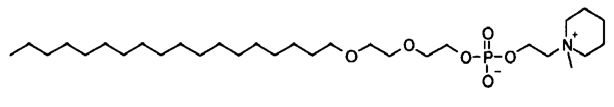 | 563,798828 |
| 212 | 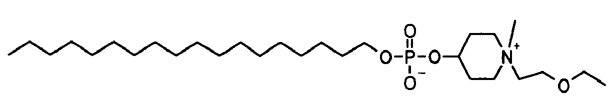 | 519,745667 |
| 213 | 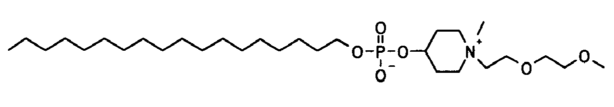 | 549,771973 |
| 214 | 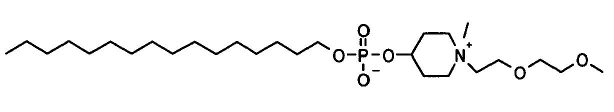 | 521,718262 |
| 215 | 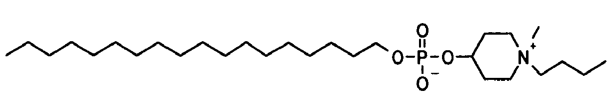 | 503,746307 |
| 216 | 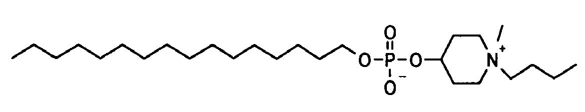 | 475,692596 |
| 217 | 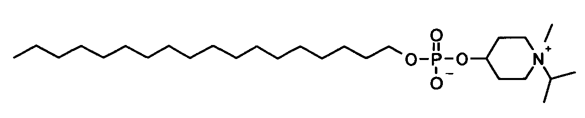 | 489,71936 |
| 218 | 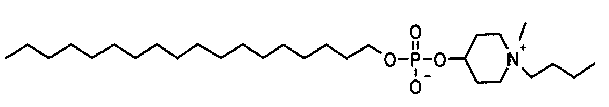 | 503,746307 |
| 219 | 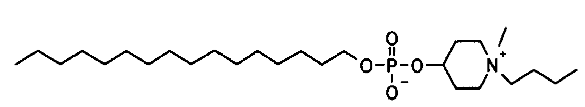 | 475,692596 |
| 220 | 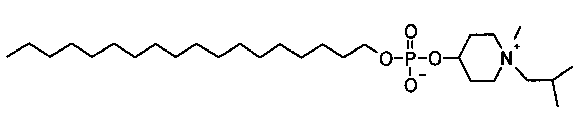 | 503,746246 |
| 221 | 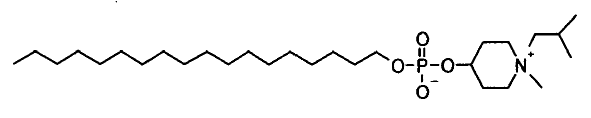 | 503,746246 |
| 222 | 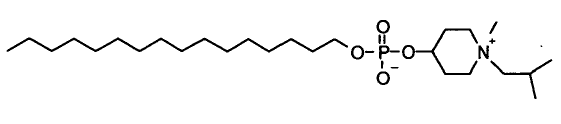 | 475,692535 |
| 223 | 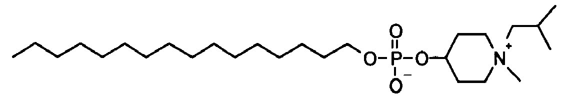 | 475,692535 |
| 224 | 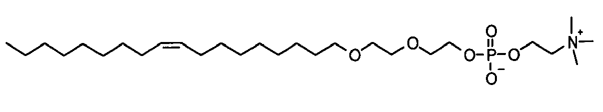 | 521,718323 |
| 225 | 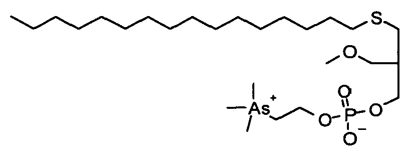 | 586,688599 |
| 226 | 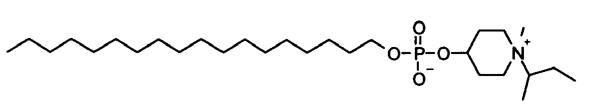 | 503,746246 |
| 227 | 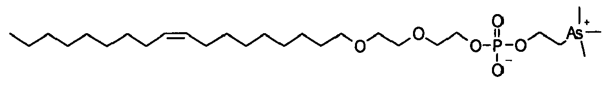 | 582,633179 |
| 228 | 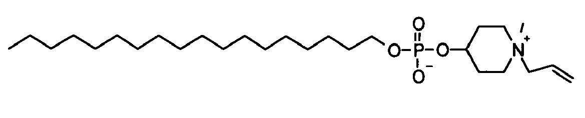 | 487,703461 |
| 229 | 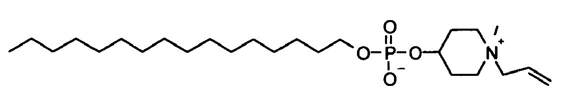 | 459,64978 |
| 230 | 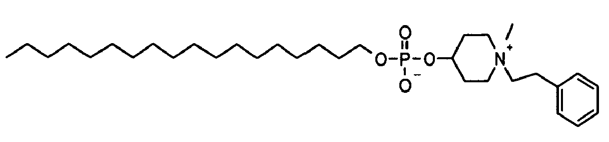 | 551,790222 |
| 231 | 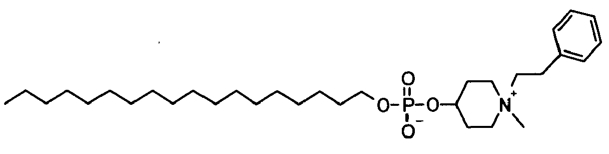 | 551,790222 |
| 232 | 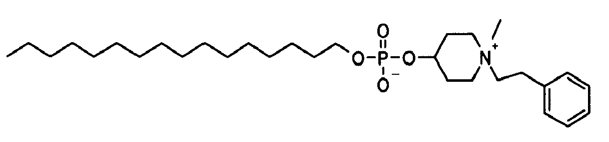 | 523, 736511 |
| 233 | 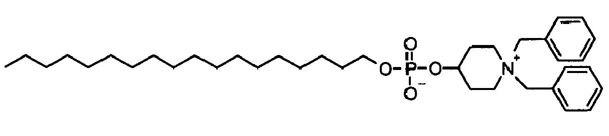 | 613,861023 |
| 234 | 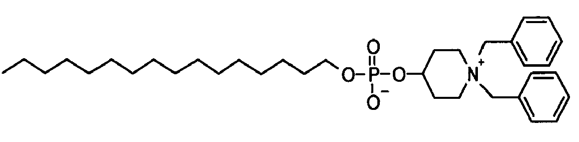 | 585,807373 |
| 235 | 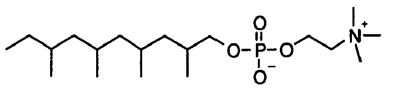 | 379,520386 |
| 236 | 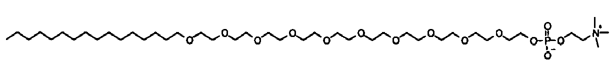 | 848,105347 |
| 237 | 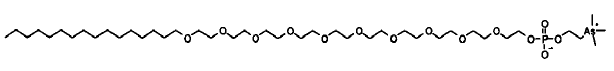 | 909,020203 |
| 238 | 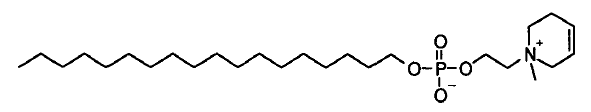 | 473,676636 |
| 239 | 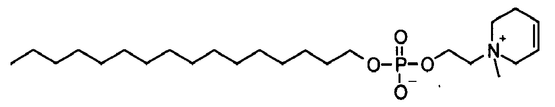 | 445,622894 |
| 240 | 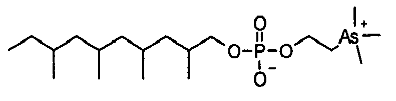 | 440,435242 |
| 241 | 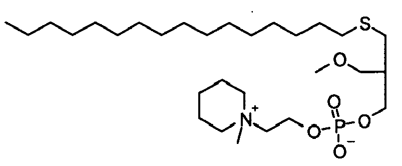 | 565,838501 |
| 242 | 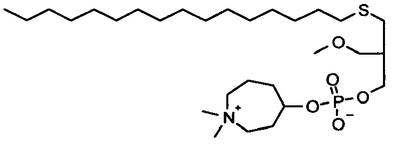 | 565,838501 |
| 243 | 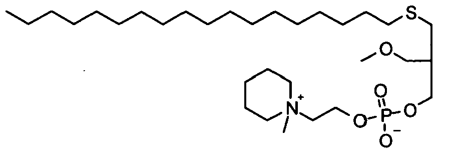 | 593,892273 |
| 244 | 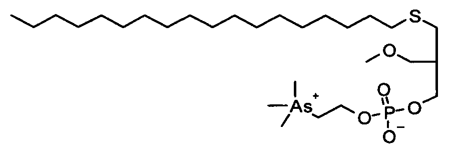 | 614, 742371 |
| 245 | 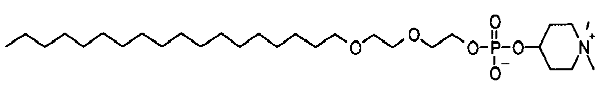 | 549,771973 |
| 246 | 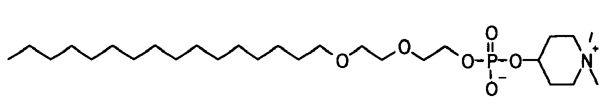 | 521,718201 |
| 247 | 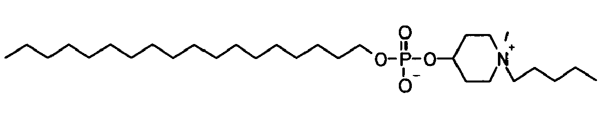 | 517,773132 |
| 248 | 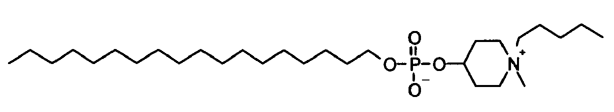 | 517,773132 |
| 249 | 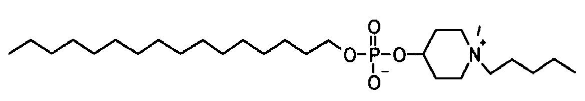 | 489,719452 |
| 250 | 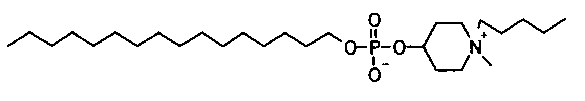 | 489,719452 |
| 251 | 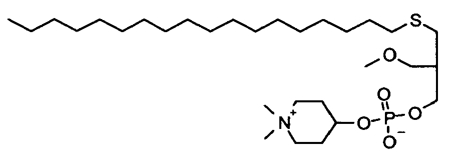 | 579,865356 |
| 252 | 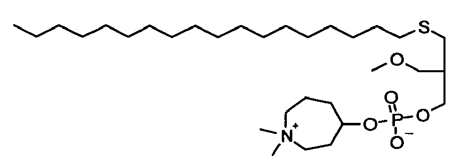 | 593,892212 |
| 253 | 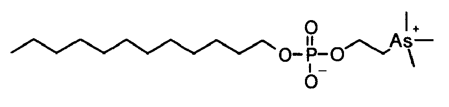 | 412,381439 |
| 254 | 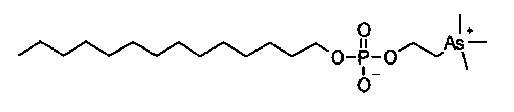 | 440,43515 |
| 255 | 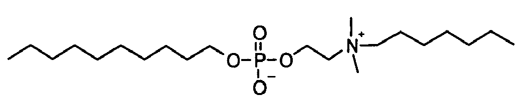 | 407,574127 |
| 256 | 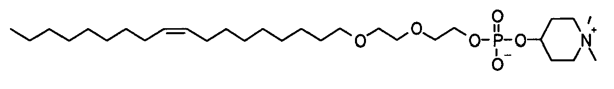 | 547,755981 |
| 257 | 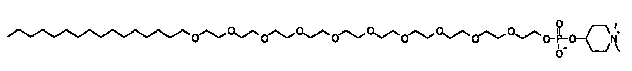 | 874,143127 |
| 258 | 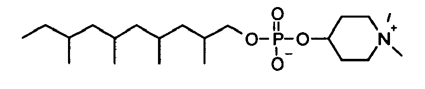 | 405,558289 |
| 259 | 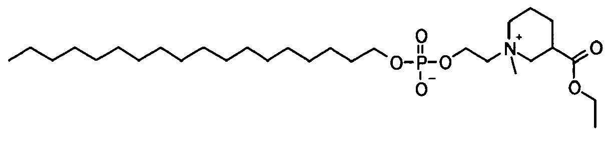 | 547,756042 |
| 260 | 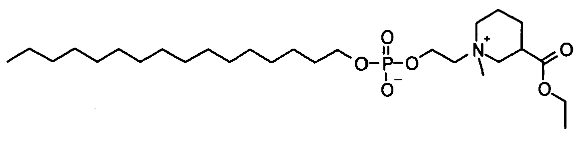 | 519,702332 |
| 261 | 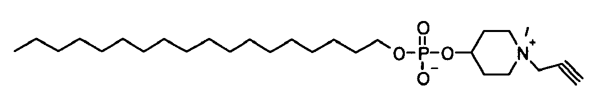 | 485,687592 |
| 262 | 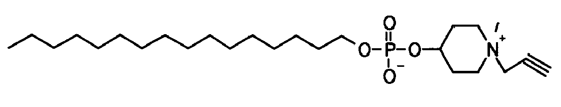 | 457,633911 |
| 263 | 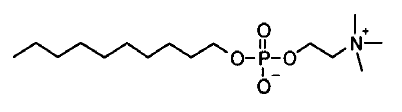 | 323,412994 |
| 264 | 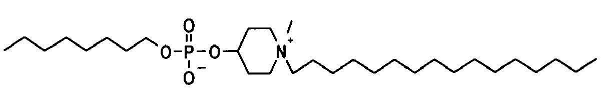 | 531,80011 |
| 265 | 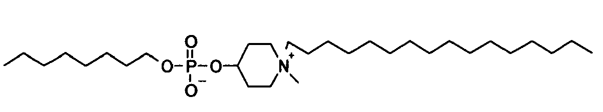 | 531,799988 |
| 266 | 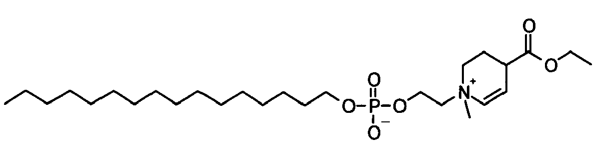 | 517,686462 |
| 267 | 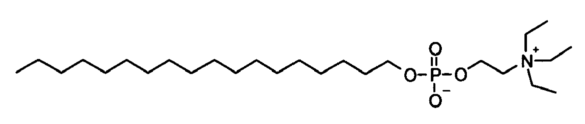 | 477,708374 |
| 268 | 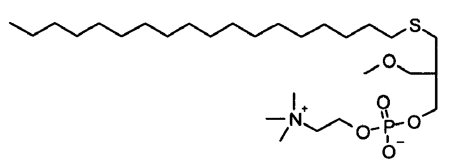 | 553,827515 |
| 269 | 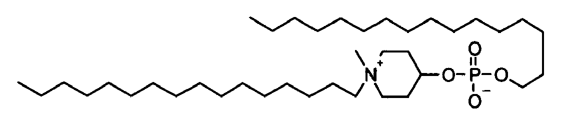 | 644,014954 |
| 270 | 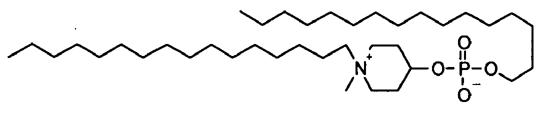 | 644,014954 |
| 271 | 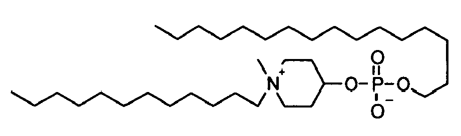 | 587,907532 |
| 272 | 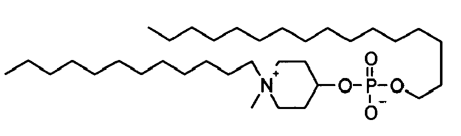 | 587,907532 |
| 273 | 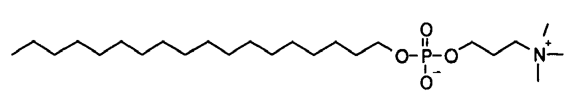 | 449,654785 |
| 274 | 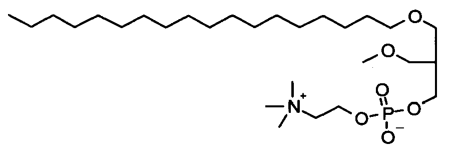 | 537,760986 |
| 275 | 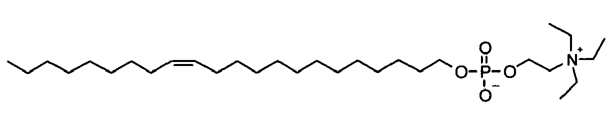 | 531,800049 |
| 276 | 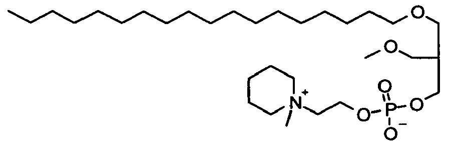 | 577,825684 |
| 277 | 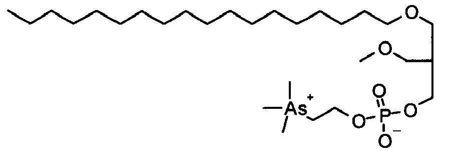 | 598,67572 |
| 278 | 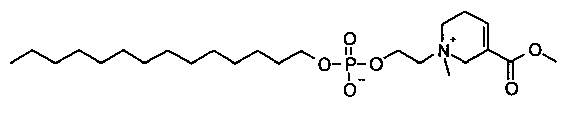 | 475,605835 |
| 279 | 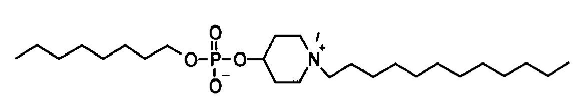 | 475,692566 |
| 280 | 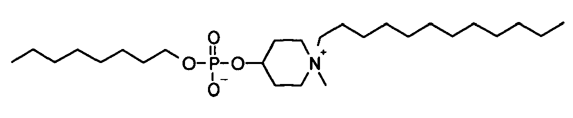 | 475,692657 |
| 281 | 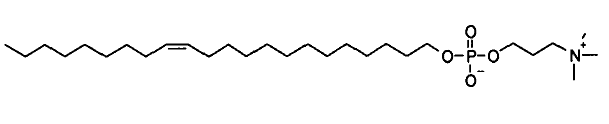 | 503,746246 |
| 282 | 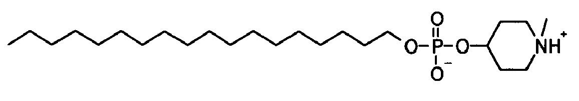 | 447,638855 |
| 283 | 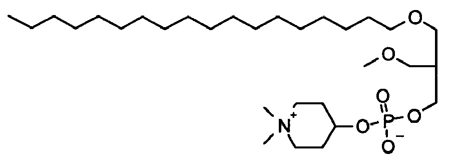 | 563,798828 |
| 284 | 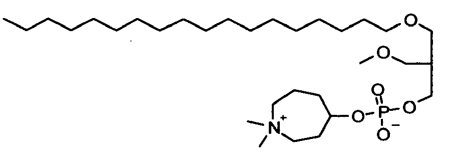 | 577,825684 |
| 285 | 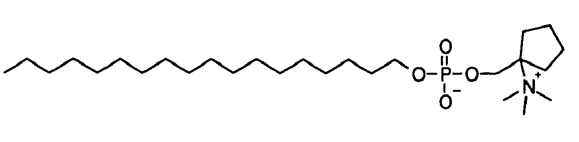 | 489,71936 |
| 286 | 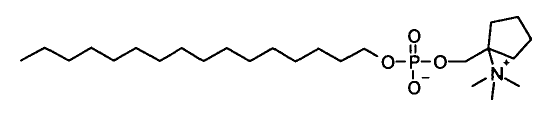 | 461,66568 |
| 287 | 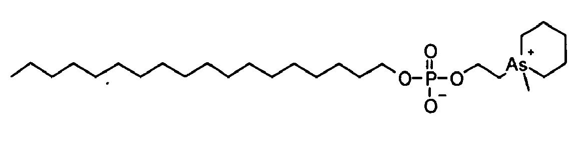 | 536,6073 |
| 288 | 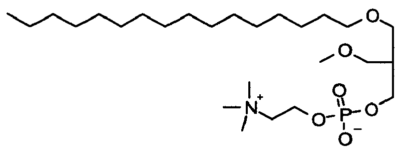 | 509,707245 |
| 289 | 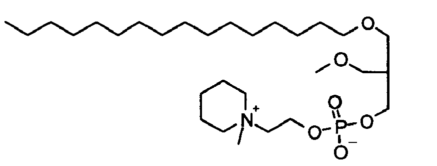 | 549,771973 |
| 290 | 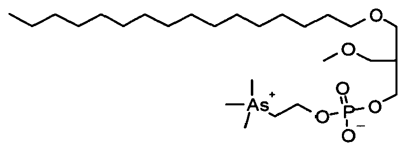 | 570,622009 |
| 291 | 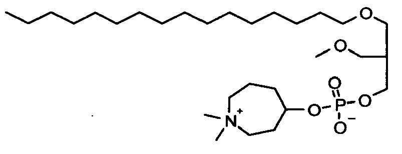 | 549,771973 |
| 292 | 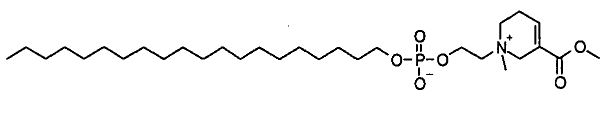 | 559,767029 |
| 293 | 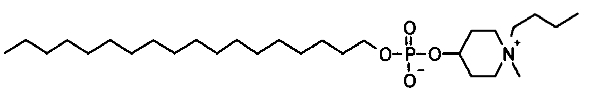 | 503, 746338 |
| 294 | 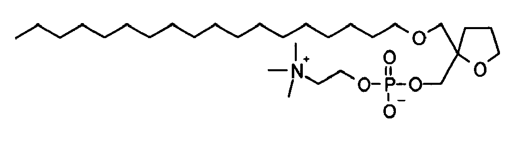 | 549,772034 |
| 295 | 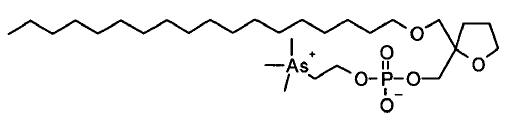 | 610,686768 |
| 296 | 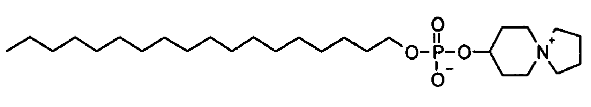 | 487,703583 |
| 297 | 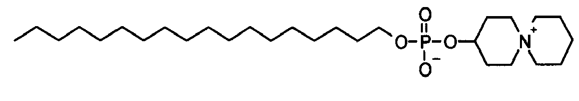 | 501, 730438 |
| 298 | 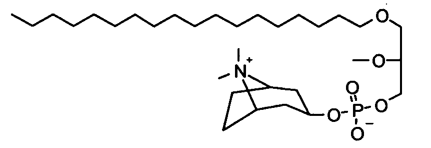 | 575,809875 |
| 299 | 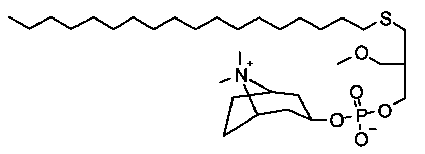 | 605,903198 |
| 300 | 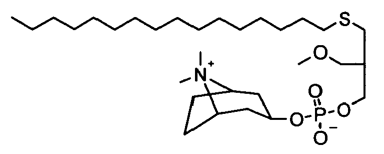 | 577,849548 |
| 301 | 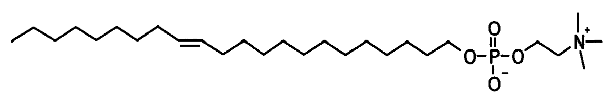 | 489,719391 |
| 302 | 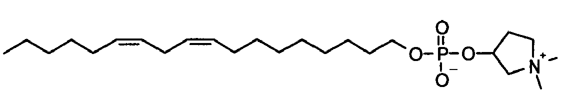 | 443,607056 |
| 303 | 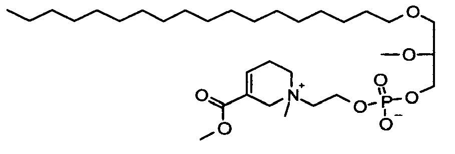 | 619,819519 |
| 304 | 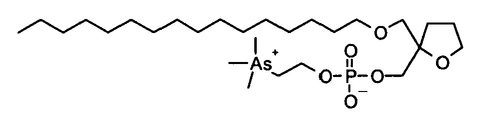 | 582,63324 |
| 305 | 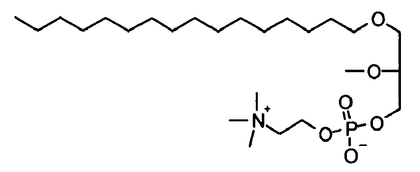 | 495,680389 |
| 306 | 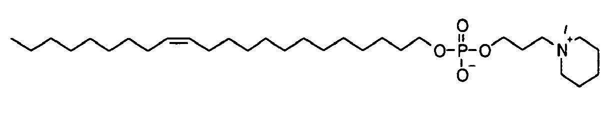 | 543,811035 |
| 307 | 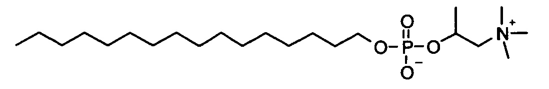 | 421,600983 |
| 308 | 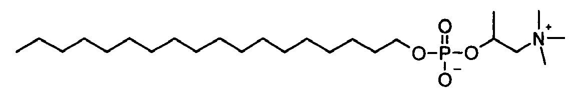 | 449,654663 |
| 309 | 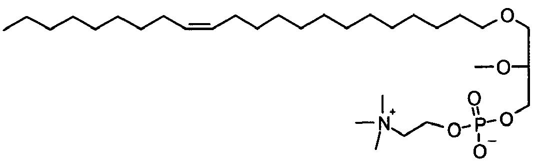 | 577,825562 |
| 310 | 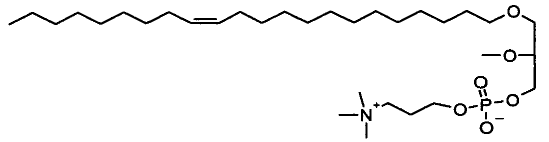 | 591,852417 |
| 311 | 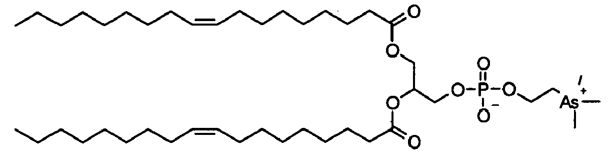 | 847,04071 |
| 312 | 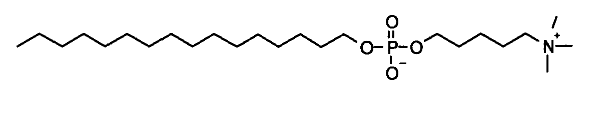 | 449,654785 |
| 313 | 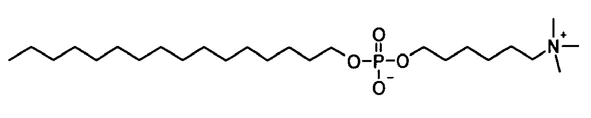 | 463,681641 |
| 314 | 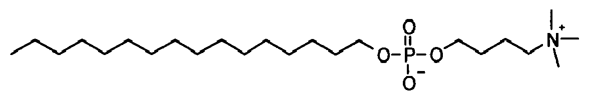 | 435,62793 |
| 315 | 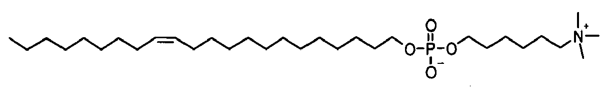 | 545, 826843 |
| 316 | 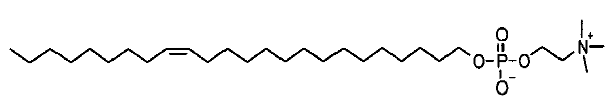 | 517,773315 |
| 317 | 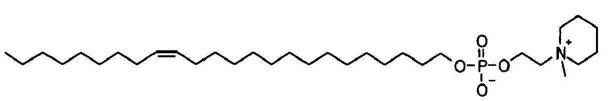 | 557,837891 |

In a preferred embodiment, all the above generically or explicitly disclosed alkyl phospholipid derivatives (including preferred subsets of formula I), hereinafter referred to as compounds of the invention, can be used for the manufacture of a medicament for the treatment or prophylaxis of diseases and/or pathophysiological conditions in mammals that are caused by microorganisms, where the microorganism is selected from the group consisting of "fungus, protozoon, bacterium and/or virus".

The terms indicated for explanation of the above compounds of the invention always, unless indicated otherwise in the description or in the claims, have the following meanings:

The term "unsubstituted" means that the corresponding radical, group or moiety has no substituents.

The term "substituted" means that the corresponding radical, group or moiety has one or more substituents. Where a radical has a plurality of substituents, and a selection of various substituents is specified, the substituents are selected independently of one another and do not need to be identical.

The term "alkyl" includes for the purposes of this invention acyclic and cyclic saturated, unsaturated or partially unsaturated hydrocarbons which may be straight-chain or branched or cyclic or also include cyclic hydrocarbons as part of a straight-chained or branched hydrocarbon system and which may contain one or more double and/or triple bonds. In this connection, C1-C6alkyl, C1-C12alkyl, C1-C18alkyl, C8-C30alkyl and the like refer to above definition having 1 to 6, 1 to 12, 1 to 18, 8 to 30, respectively, and the like carbon atoms.

Examples of suitable alkyl radicals are methyl, ethyl, n-propyl, isopropyl, n-butyl, isobutyl, sec-butyl, tert-butyl, n-pentyl, iso-pentyl, neo-pentyl, tert-pentyl, 2- or 3-methylpentyl, n-hexyl, 2-hexyl, isohexyl, n-heptyl, n-octyl, n-nonyl, n-decyl, n-undecyl, n-dodecyl, n-tetradecyl, n-hexadecyl, n-octadecyl, n-icosanyl, n-docosanyl, ethylenyl (vinyl), propenyl (-CH₂CH=CH₂; -CH=CH-CH₃, -C(=CH₂)-CH₃), butenyl, pentenyl, hexenyl, heptenyl, octenyl, octadienyl, octadecenyl, octadec-9-enyl, icosenyl, icos-11-enyl, (Z)-icos-11-enyl, docosnyl, docos-13-enyl, (Z)-docos-13-enyl, ethynyl, propynyl (-CH₂-C≡CH, -C≡C-CH₃), butynyl, pentynyl, hexynyl, heptynyl, octynyl, cyclopropyl, cyclobutyl, cyclopentyl, cyclohexyl, cycloheptyl, cyclooctyl, cyclodecyl, cyclohexenyl, cyclopentenyl, cyclooctadienyl.

The term "C3-C8cycloalkyl" stands for a saturated or partially unsaturated nonaromatic cyclic hydrocarbon group/radical, containing 3 to 8 carbon atoms that may optionally be linked via an alkyl group where the alkyl has the meaning as defined herein, preferably a (C3-C8)-cycloalkyl-(C1-C4)-alkyl radical. Such "C3-C8cycloalkly" radicals can be linked via any ring member.

Examples of suitable cycloalkyl radicals are cyclopropyl, cyclobutyl, cyclopentyl, cyclohexyl, cycloheptyl, cyclooctyl, cyclohexenyl, cyclopentenyl, cyclooctadienyl, cyclopropylmethyl, cyclohexylmethyl, cyclopentylethyl, cyclohexenylethyl.

The term "steroid moiety" for the purpose of the present invention refers to a basic steroid structure as for instance illustrated on page 131 in "Organische Chemie" by K. Peter C. Vollhardt (VCH Weinheim, 1. korrigierter Nachdruck, 1990, der 1. Auflage 1988, ISBN 3-527-26912-6). Such "steroid moiety" can be linked via any atom of the moiety.

The term "aryl" refers to aromatic hydrocarbon systems having 3 to 14, preferably 5 to 14, carbon atoms. The term "aryl" also includes systems in which the aromatic cycle is part of a bi- or polycyclic saturated, partially unsaturated and/or aromatic system, such as were the aromatic cycle is fused to an "aryl", "C3-C8cycloalkyl", "heteroaryl" or "heterocyclyl" group as defined herein via any desired and possible ring member of the aryl radical. Such "aryl" radicals can be linked via any ring member. Examples of "aryl" are inter alia phenyl, biphenyl, naphthyl and anthracenyl, but also indanyl, indenyl, or 1,2,3,4-tetrahydronaphthyl. The terms "aryl" is also intended to comprise radicals in which the aryl radical is linked via a C1-C18alkyl group, preferably C1-C12alkyl group. Most preferred are aryl-C1-C4alkyl radicals, preferably benzyl or phenylethyl radicals.

The term "heteroaryl" refers to a 5-, 6- or 7-membered cyclic aromatic radical which comprises at least 1, where appropriate also 2, 3, 4 or 5 heteroatoms, preferably nitrogen, oxygen and/or sulfur, where the heteroatoms are identical or different. The number of nitrogen atoms is preferably 0, 1, 2, or 3, and that of the oxygen and sulfur atoms is independently 0 or 1. The term "heteroaryl" also includes systems in which the aromatic cycle is part of a bi- or polycyclic saturated, partially unsaturated and/or aromatic system, such as were the aromatic cycle is fused to an "aryl", "C3-C8cycloalkyl", "heteroaryl" or "heterocyclyl" group as defined herein via any desired and possible ring member of the heteroaryl radical. Such "heteroaryl" radicals can be linked via any ring member. Examples of "heteroaryl" include pyrrolyl, thienyl, furyl, imidazolyl, thiazolyl, isothiazolyl, oxazolyl, oxadiazolyl, isoxazolyl, pyrazolyl, pyridinyl, pyrimidinyl, pyrazinyl, indolyl, quinolinyl, isoquinolinyl, imidazolyl, triazolyl, tetrazolyl, pyridazinyl, phthalazinyl, indazolyl, indolizinyl, quinoxalinyl, quinazolinyl, pteridinyl, carbazolyl, phenazinyl, phenoxazinyl, phenothiazinyl, acridinyl. The terms "heteroaryl" is also intended to comprise radicals in which the heteroaryl radical is linked via a C1-C18alkyl group, preferably C1-C12alkyl group. Most preferred are heteroaryl-C1-C4alkyl radicals, preferably indolyl-(C₁-C₄)-alkyl radicals, such as 1*H* indole-3-yl-methyl or 2-(1*H*-indole-3-yl)-ethyl.

The term "heterocyclyl" refers to a mono- or polycyclic system of 3 to 20, preferably 5 or 6 to 14 ring atoms comprising carbon atoms and 1, 2, 3, 4, or 5 heteroatoms, in particular nitrogen, oxygen and/or sulfur which are identical or different. The cyclic system may be saturated, mono- or polyunsaturated but may not be aromatic. In the case of a cyclic system consisting of at least two rings the rings may be fused or spiro- or otherwise connected. Such "heterocyclyl" radicals can be linked via any ring member. The term "heterocyclyl" also includes systems in which the heterocycle is part of a bi- or polycyclic saturated, partially unsaturated and/or aromatic system, such as where the heterocycle is fused to an "aryl", "C3-C8cycloalkyl", "heteroaryl" or "heterocyclyl" group as defined herein via any desired and possible ring member of the heterocycyl radical. Examples of "heterocyclyl" include pyrrolidinyl, thiapyrrolidinyl, piperidinyl, piperazinyl, oxapiperazinyl, oxapiperidinyl, oxadiazolyl, tetrahydrofuryl, imidazolidinyl, thiazolidinyl, tetrahydropyranyl, morpholinyl, tetrahydrothiophenyl, dihydropyranyl. The term "heterocyclyl" is also intended to comprise radicals in which the heterocyclyl group is linked via an C1-C18alkyl group, preferably C1-C12alkyl group. Most preferred are hetercyclyl-C1-C4alkyl radicals.

The term "alkoxy" refers to radicals in which a "alkyl", "C3-C8cycloalkyl", "aryl", "heteroaryl", and/or "heterocyclyl" group is linked via an oxygen atom (-O-group), where "alkyl", "C3-C8cycloalkyl", "aryl", "heteroaryl" and "heterocyclyl" have the meanings as defined herein.

The term "halogen", "halogen atom" or "halogen substituent" (Hal-) refers to one, where appropriate, a plurality of fluorine (F, fluoro), bromine (Br, bromo), chlorine (Cl, chloro), or iodine (I, iodo) atoms. The designations "dihalogen", "trihalogen" and "perhalogen" refer respectively to two, three and four substituents, where each substituent can be selected independently from the group consisting of fluorine, chlorine, bromine and iodine. "Halogen" preferably means a fluorine, chlorine or bromine atom.

The term "substituted" in connection with "steroid moiety", "alkyl", in particular C1-C6alkyl, C1-C12alkyl, C1-C18alkyl, C8-C30alkyl, "C3-C8cycloalkyl","aryl", "heteroaryl", "heterocyclyl", and "alkoxy", unless not explicitly otherwise defined in the description or in the claims, means the independent replacement/substitution of one ore more hydrogen atoms by a substituent independently selected from the group consisting of "-NO₂, -NO, -CN, -OH, halogen, F, Cl, Br, I, -NH₂, -NHNH₂, -N₃, -SH, -SO₃H, -COOH, -CONH₂, -CHO, -CHNOH, -NH-C(NH₂)=NH, -C(NH₂)=NH, -CF₃, -OCF₃, -OSO₃H, -P(O)(OH)₂, -P(O)(OH)₂, -NH-(C1-C12alkyl), -N(C1-C12alkyl)₂, -NH-aryl, -N(aryl)₂, -N-A5A6, -NH-C(NH₂)=N-(C1-C12alkyl),-C(NH₂)=N-(C1-C12alkyl), -NH-C(NH₂)=N-aryl, -C(NH₂)=N-aryl, -OP(O)(O-(C1-C12alkyl))₂, -OP(O)(O-aryl)₂, OP(O)(O-heteroaryl)₂, -OP(O)(O-A7)(O-A8), -O-aryl, -O-heteroaryl, -O-(C3-C8cycloalkyl), -O-heterocyclyl, -S-(C1-C12alkyl), -S-aryl, -S-heteroaryl, -S-(C3-C8cycloalkyl), -S-heterocyclyl, -SO-(C1-C12alkyl), -SO-aryl, -SO-heteroaryl, -SO-(C3-C8cycloalkyl), -SO-heterocyclyl, -SO₂-(C1-C12alkyl), -SO₂-aryl, -SO₂-heteroaryl, -SO₂-(C3-C8cycloalkyl), -SO₂-heterocyclyl, -SO₃-(C1-C12alkyl), -SO₃-aryl, -SO₃-heteroaryl, -SO₃-(C3-C8cycloalkyl), -SO₃-heterocyclyl, -OC(O)-(C1-C12alkyl), -OC(O)-(C3-C8cycloalkyl), -OC(O)-aryl, -OC(O)-heterocyclyl, -OC(O)-heteroaryl, -C(O)-(C1-C12alkyl), -C(O)-(C3-C8cycloalky), -C(O)-aryl, -C(O)-heterocyclyl, -C(O)-heteroaryl, -C(O)O-(C1-C12alkyl), -C(O)O-(C3-C8cycloalkyl), -(O)O-aryl, -C(O)O-heterocyclyl, -C(O)O-heteroaryl, -OC(O)NH-(C1-C12alkyl), -C(O)NH-(C3-C8cycloalkyl), -OC(O)NH-aryl, -OC(O)NH-heteroaryl, -OC(O)NH-heterocyclyl, -OC(O)N-A1A2, -OC(O)N-heterocyclyl, -C(O)NH-(C1-C12alkyl), -(O)NH-(C3-C8cycloalkyl), -C(O)NH-aryl, -C(O)NH-heteroaryl, -C(O)NH-heterocyclyl, -C(O)N-A3A4, -C(O)N-heterocyclyl, C1-C12alkyl, C3-C8cycloalkyl, aryl, heteroaryl, heterocyclyl" where A1, A2, A3, A4, A5, A6, A7, A8, A9, A10 independently are selected from the group consisting of "C1-C12alkyl, C3-C8cycloalkyl, aryl, heteroaryl, heterocyclyl".

For the purpose of the present invention, the terms indicated for explanation of the above group of microorganisms always, unless indicated otherwise in the description or in the claims, have the following meanings:

The term "fungus" or "fungi" is intended to comprise all known single-cell/unicellular and/or multi-cellular members of the kingdom of fungi, such as Chytridiomycota, Zygomycota, Glomeromycota, Ascomycota and/or Basidiomycota, as well as in addition Myxomycota, Oomycota and/or Hypochytriomycota.

Examples of such fungi are Absidia spp., Acremonium spp., Alternaria spp., Aspergillus spp., Bipolaris spp., Candida spp., Cladophialophora spp., Cladosporium spp., Coccidioides spp., Coniothyrium spp., Cryptococcus spp., Cunninghamella spp., Curvularia spp., Epidermophyton spp., Exophiala spp., Exserohilum spp., Fonsecaea spp., Fusarium spp., Histoplasma spp., Lacazia spp., Lasiodiplodia spp., Leptosphaeria spp., Madurella spp., Microsporum spp., Mucor spp., Mucorales spp., Neotestudina spp., Ochroconis spp., Onychocola spp., Paecilomyces spp., Paracoccidioides spp., Penicillium spp., Phialophora spp., Pseudallesheria spp., Pyrenochaeta spp., Rhizomucor spp., Rhizopus spp., Scedosporium spp., Scopulariopsis spp., Scytalidium spp., Sporothrix spp., Trichophyton spp. and/or Wangiella spp.

Further examples of such fungi are Absidia corymbifera, Acremonium falciforme, Acremonium recifei, Aspergillus flavus, Aspergillus fumigatus, Aspergillus glaucus, Aspergillus nidulans, Aspergillus niger, Aspergillus terreus, Bipolaris australiensis, Candida albicans, Candida glabrata, Candida krusei, Candida parapsilosis, Candida tropicalis, Cladophialophora bantiana, Cladophialophora carrionii, Coccidioides immitis, Coniothyrium fuckelii, Cryptococcus gattii, Cryptococcus neoformans, Cunninghamella bertholletitae, Epidermophyton floccosum, Exophiala jeanselmei, Exophiala spinifera, Fonsecaea compacta, Fonsecaea pedrosoi, Fusarium oxysporum, Fusarium solani, Histoplasma capsulatum capsulatum, Histoplasma capsulatum duboisii, Lacazia loboi, Lasiodiplodia theobromae, Leptosphaeria senegalensis, Madurella grisea, Madurella mycetomatis, Microsporum audouinii, Microsporum canis, Microsporum gypseum, Neotestudina rosatii, Ochroconis gallopava, Onychocola Canadensis, Paecilomyces lilacinus, Paracoccidioides brasiliensis, Phialophora parasitica, Phialophora repens, Phialophora verrucosa, Pseudallesheria boydii, Pyrenochaeta romeroi, Rhizomucor pusillus, Rhizopus arrhizus, Rhizopus oryzae, Scedosporium apiospermum, Scedosporium inflatum, Scedosporium prolificans, Scopulariopsis brevicaulis, Scytalidium dimidiatum, Sporothrix schenckii, Trichophyton mentagrophytes, Trichophyton rubrum, Trichophyton schoenleinii, Trichophyton tonsurans and/or Wangiella dermatitidis.

The term "protozoon" or "protozoa" is intended to comprise all known single-cell/unicellular and/or multi-cellular members of Sporozoa, Gregarinida, Coccida, Piroplasminda, Babesia, Microsporidia, Giardiinae, Trichomonadida, Diplomonadida, Hypermastigida, Trypanosoma, Entamoebidae, Kinetoplasta, Tryposomatidea, Tryposomatidae, Apicomplexa, Haemosporida, Plasmodiidae, Rhizopoda and/or Amoebina.

Examples of such protozoa are Acanthamoeba spp., Amoeba spp., Babesia spp., Balantidium spp., Cryptosporidium spp., Cyclospora spp., Dientamoeba spp., Echinamoeba spp., Endolimax spp., Entamoeba spp., Enterocytozoon spp., Giardia spp. , Hartmanella spp., Isospora spp., Jodamoeba spp., Lamblia spp., Leishmania spp., Microsporidium spp., Naegleria spp., Nosema spp., Paramecium spp., Paramoeba spp., Penumocystis spp., Plasmodium spp., Sarcocystis spp., Tetrahymena spp., Toxoplasma spp., Trichomonas spp. and/or Trypanosoma spp.

Further examples of such protozoa are Amoeba proteus, Babesia microti, Balantidium coli, Cryptosporidium parvum, Cyclospora cayetanensis, Dientamoeba fragilis, Endolimax nana, Entamoeba coli, Entamoeba gingivalis, Entamoeba hartmanni, Entamoeba histolytica, Enterocytozoon bieneusi, Enterocytozoon cuniculi, Giardia lamblia, Giardia lamblia intestinalis, Isospora belli, Jodamoeba butschlii, Lamblia intestinalis, Leishmania braziliensis braziliensis, Leishmania chagasi, Leishmania donovani, Leishmania infantum, Leishmania major, Leishmania mexicana, Leishmania mexicana amazonensis, Leishmania mexicana mexicana, Leishmania tropica, Leishmania Viannia, Leishmania Viannia braziliensis, Leishmania Viannia guyanensis, Leishmania Viannia panamensis, Leishmania Viannia peruviana, Naegleria fowleri, Nosema corneum, Plasmodium falciparum, Plasmodium malariae, Plasmodium ovale, Plasmodium vivax, Pneumocystis carinii, Sarcocystis bovihomins, Sarcocystis suihominis, Tetrahymena pyriformis, Toxoplasma gondii, Trichomonas vaginalis, Trypanosoma brucei, Trypanosoma brucei brucei, Trypanosoma brucei gambiense, Trypanosoma brucei rhodesiense, Trypanosoma cruzi, Trypanosoma cruzi cruzi, Trypanosoma cruzi marinkellei, Trypanosoma equinum, Trypanosoma equiperdum, Trypanosoma evansi, Trypanosoma theileri and/or Trypanosoma vivax.

The term "bacterium" or "bacteria" is intended to comprise all known aerobic bacteria, obligatory/fastidious anaerobic bacteria and facultative anaerobic bacteria. These can be either gram-positive or gram-negative or difficult to Gram stain, do also comprise spore forming bacteria and bacterial spores and can for instance members of Actinobacteria, Aquificae, Bacteroidetes/Chlorobi, Chlamydiae/Verrucomicrobia, Chloroflexi, Chrysiogenetes, Cyanobacteria, Deferribacteres, Deinococcus-Thermus, Dictyoglomi, Fibrobacteres/Acidobacteria, Firmicutes, Fusobacteria, Gemmatimonadetes, Nitrospirae, Omnibacteria, Planctomycetes, Proteobacteria, Spirochaetes, Thermodesulfobacteria, Thermomicrobia and/or Thermotogae.

Examples of such bacteria are Acinetobacter spp., Actinobacillus spp., Actinomyces spp., Aeromonas spp., Agrobacterium spp., Alcaligenes spp., Anaplasma spp., Aquifex spp., Bacillus spp., Bacteroides spp., Bifidobacterium spp., Bordetella spp., Borrelia spp., Bradyrhizobium spp., Branhamella spp., Brucella spp., Buchnera spp., Burkholderia spp., Campylobacter spp., Capnocytophaga spp., Cardiobacterium spp., Caulobacter spp., Chlamydia spp., Chlamydophila spp., Chlorobium spp., Citrobacter spp., Clostridium spp., Corynebacterium spp., Coxiella spp., Deinococcus spp., Ehrlichia spp., Eikenella spp., Enterobacter spp., Enterococcus spp., Erysipelothrix spp., Escherichia spp., Francisella spp., Fusobacterium spp., Gardnerella spp., Gemella spp., Haemophilus spp., Heliobacter spp., Kingella spp., Kitasatospora spp., Klebsiella spp., Lactobacillus spp., Legionella spp., Leptospira spp., Listeria spp., Mannheimia spp., Mesorhizobium spp., Moraxella spp., Morganella spp., Mycobacterium spp., Mycoplasma spp., Neisseria spp., Neorickettsia spp., Nitrosomonas spp., Nocardia spp., Oceanobacillus spp., Orientia spp., Paracoccus spp., Pasteurella spp., Peptostreptococcus spp., Plasmodium spp., Plesiomonas spp., Porphyromonas spp., Prevotella spp., Propionibacterium spp., Proteus spp., Providencia spp., Pseudomonas spp., Psychobacter spp., Ralstonia spp., Rhodobacter spp., Rhodococcus spp., Rickettsia spp., Salmonella spp., Serratia spp., Shewanella spp., Shigella spp., Spirillum spp., Staphylococcus spp., Stenotrophomonas spp., Streptobacillus spp., Streptococcus spp., Streptomyces spp., Synechococcus spp., Synechocystis spp., Tannerella spp., Thermoanaerobacter spp., Thermotoga spp., Treponema spp., Tropheryma spp., Ureaplasma spp., Veillonella spp., Vibrio spp., Wigglesworthia spp., Wolbachia spp., Xanthomonas spp., Xylella spp., Yersinia spp. and/or Zymomonas spp.

Further examples of such bacteria are Acinetobacter baumannii, Acinetobacter haemolyticus, Actinobacillus actinomycetemcomitans, Actinobacillus pleuropneumoniae, Actinomyces israelii, Aeromonas hydrophila, Agrobacterium tumefaciens, Alcaligenes xylosoxidans, Anaplasma phagocytophilum, Aquifex aeolicus, Bacillus anthracis, Bacillus cereus, Bacillus halodurans, Bacillus subtilis, Bacteroides fragilis, Bacteroides thetaiotaomicron, Bartonella bacilliformis, Bartonella henselae, Bifidobacterium longum, Bordetella bronchiseptica, Bordetella pertussis, Borrelia burgdorferi, Borrelia recurrentis, Bradyrhizobium japonicum, Branhamella catarrhalis, Brucella abortus, Brucella canis, Brucella melitensis, Brucella suis, Buchnera aphidicola, Burkholderia cepacia, Burkholderia mallei, Burkholderia pseudomallei, Campylobacter fetus, Campylobacter jejuni, Capnocytophaga granulosa, Capnocytophaga haemolytica, Cardiobacterium hominis, Caulobacter crescentus, Caulobacter vibrioides, Chlamydia muridarum, Chlamydia pneumoniae, Chlamydia psittaci, Chlamydia trachomatis, Chlamydophila caviae, Chlamydophila pneumoniae, Chlamydophila psittaci, Chlorobium tepidum, Citrobacter freundii, Clostridium acetobutylicum, Clostridium botulinum, Clostridium difficile, Clostridium novyi, Clostridium perfringens, Clostridium tetani, Corynebacterium diphtheriae, Corynebacterium efficiens, Corynebacterium glutamicum, Coxiella burnetii, Deinococcus radiodurans, Ehrlichia canis, Ehrlichia chaffensis, Eikenella corrodens, Enterobacter cloacae, Enterococcus faecalis, Enterococcus faecium, Erysipelothrix rhusiopathiae, Escherichia coli, Escherichia coli O157:H7, Francisella tularensis, Francisella tularensis tularensis, Fusobacterium necrophorum, Fusobacterium nucleatum, Gardnerella vaginalis, Gemella morbillorum, Haemophilus ducreyi, Haemophilus influenzae, Haemophilus parainfluenzae, Heliobacter pylori, Kingella kingii, Kitasatospora griseola, Klebsiella aerogenes, Klebsiella granulomatis, Klebsiella pneumoniae, Klebsiella pneumoniae ozaenae, Klebsiella pneumoniae pneumoniae, Klebsiella pneumoniae rhinoscleromatis, Lactobacillus aviarius, Lactobacillus plantarum, Legionella pneumophila, Leptospira interrogans, Leptospira noguchii, Listeria innocua, Listeria ivanovii, Listeria ivanovii ivanovii, Listeria monocytogenes, Mannheimia haemolytica, Mesorhizobium loti, Moraxella catarrhalis, Morganella morganii morganii, Mycobacterium abscessus, Mycobacterium africanum, Mycobacterium avium, Mycobacterium avium paratuberculosis, Mycobacterium bovis, Mycobacterium bovis bovis, Mycobacterium bovis caprae, Mycobacterium chelonae, Mycobacterium fortuitum, Mycobacterium intracellulare, Mycobacterium kansasii, Mycobacterium leprae, Mycobacterium malmoense, Mycobacterium marinum, Mycobacterium scrofulaceum, Mycobacterium smegmatis, Mycobacterium tuberculosis, Mycobacterium ulcerans, Mycoplasma genitalium, Mycoplasma hominis, Mycoplasma penetrans, Mycoplasma pneumoniae, Neisseria gonorrhoeae, Neisseria meningitidis, Neorickettsia sennetsu, Nitrosomonas europaea, Nocardia arthritidis, Nocardia asteriodes, Nocardia cyriacigeorgica, Oceanobacillus iheyensis, Orientia tsutsugamushi, Paracoccus zeaxanthinifaciens, Pasteurella haemolytica, Pasteurella multocida, Peptostreptococcus parvulus, Peptostreptococcus tetradius, Peptostreptococcus vaginalis, Plesiomonas shigelloides, Porphyromonas gingivalis, Prevotella intermedia, Prevotella melaninogenica, Propionibacterium acnes, Proteus mirabilis, Proteus vulgaris, Providencia alcalifaciens, Providencia friedericiana, Pseudomonas aeruginosa, Pseudomonas alcaligenes, Pseudomonas flurescens, Pseudomonas putida, Pseudomonas stutzeri, Pseudomonas syringae, Ralstonia solanacearum, Rhodobacter capsulatus, Rhodococcus equi, Rickettsia akari, Rickettsia prowazekii, Rickettsia rickettsii, Rickettsia typhi, Salmonella choleraesuis, Salmonella enterica, Salmonella enteritidis, Salmonella paratyphi, Salmonella typhi, Salmonella typhimurium, Serratia marcescens, Shewanella oneidensis, Shewanella putrefaciens, Shigella dysenteriae, Shigella flexneri, Shigella sonnei, Sinorhizobium meliloti (Rhizobium meliloti), Spirillum minus, Staphylococcus aureus, Staphylococcus epidermidis, Staphylococcus hemolyticus, Staphylococcus saprophyticus, Stenotrophomonas maltophilia, Streptobacillus moniliformis, Streptococcus agalactiae, Streptococcus faecalis, Streptococcus milleri, Streptococcus mutans, Streptococcus pneumoniae, Streptococcus pyogenes, Streptococcus salivarius, Streptococcus viridans, Streptomyces avermitilis, Streptomyces coelicolor, Streptomyces hygroscopicus, Streptomyces lividans, Streptomyces rishiriensis, Synechococcus elongates, Synechococcus leopoliensis, Tannerella forsynthensis, Thermoanaerobacter tengcongensis, Thermotoga maritime, Treponema carateum, Treponema denticola, Treponema endemicum, Treponema pallidum, Treponema petenue, Tropheryma whipplei, Ureaplasma urealyticum, Veillonella alcalescens alcalescens, Veillonella parvula atypica, Vibrio cholerae, Vibrio parahemolyticus, Vibrio vulnificus, Wigglesworthia glossinidia, Xanthomonas axonopodis, Xanthomonas campestris, Xanthomonas maltophilia, Xylella fastidiosa, Yersinia enterocolitica, Yersinia pestis, Yersinia pseudotuberculosis and/or Zymomonas mobilis.

The classification of bacteria into "gram-positive", "gram-negative" or "bacteria that are difficult to Gram stain" as well as the determination of the aerobic or anaerobic state can be easily performed by the skilled artisan on the basis of his expert knowledge, e.g. by appropriate staining and metabolic assays which represent only routine experimentation.

In this connection the term "gram-negative" in the course of the present invention is intended to comprise bacteria that cannot be Gram stained as well as those that are difficult to Gram stain. In contrast, the term "gram-positive" in connection with bacteria is intended for the purpose of the present invention to comprise all bacteria that can be Gram stained and/or are known to be gram-positive.

The term "virus" or "viruses" is intended to comprise all known DNA viruses, e.g. dsDNA viruses (double stranded DNA) and ssDNA viruses (single stranded DNA); RNA viruses, e.g. dsRNA viruses (double stranded RNA), (+)ssRNA viruses (positive single stranded RNA or mRNA like) and (-)ssRNA viruses (negative single-stranded RNA); as well as DNA and RNA reverse transcribing viruses (retroviruses), e.g. ssRNA-RT viruses (single stranded RNA) and dsDNA-RT viruses (double stranded DNA). These can be either coated or uncoated.

Examples of such viruses are Caudovirales, Myoviridae, Podoviridae, Siphoviridae, Ascoviridae, Adenoviridae, Asfiviridae, Baculoviridae, Corticoviridae, Fuselloviridae, Guttaviridae, Herpesviridae, Iridoviridae, Lipothrixviridae, Nimaviridae, Papillomaviridae, Phycodnaviridae, Plasmaviridae, Polyomaviridae, Poxviridae, Rudiviridae, Tectiviridae, Inoviridae, Microviridae, Geminiviridae, Circoviridae, Nanoviridae, Parvoviridae, Anellovirus, Birnaviridae, Chrysoviridae, Cystoviridae, Hypoviridae, Partitiviridae, Reoviridae, Totiviridae, Endornavirus, Nidovirales, Arteriviridae, Coronaviridae, Roniviridae, Astroviridae, Barnaviridae, Bromoviridae, Caliciviridae, Closteroviridae, Comoviridae, Dicistroviridae, Flaviviridae, Flexiviridae, Hepeviridae, Leviviridae, Luteoviridae, Marnaviridae, Narnaviridae, Nodaviridae, Picornaviridae, Potyviridae, Sequiviridae, Tetraviridae, Togaviridae, Tombusviridae, Tymoviridae, Benyvirus, Cheravirus, Furovirus, Hordeivirus, Idaeovirus, Machlomovirus, Ourmiavirus, Pecluvirus, Pomovirus, Sadwavirus, Sobemovirus, Tobamovirus, Tobravirus, Umbravirus, Mononegavirales, Bornaviridae, Filoviridae, Paramyxoviridae, Rhabdoviridae, Arenaviridae, Bunyaviridae, Orthomyxoviridae, Deltavirus, Ophiovirus, Tenuivirus, Varicosavirus, Metaviridae, Pseudoviridae, Retroviridae, Hepadnaviridae and/or Caulimoviridae.

Further examples of such viruses are adenovirus type 1, 2, 3, 5, 11, 21, adenovirus, alphavirus, arbovirus, arenavirus, borna disease virus, bunyavirus, calicivirus, California encephalitis virus, Colorado tick fever virus, coronavirus cowpox virus, coxsackie type A virus, coxsackie type B virus, coxsackie virus type A-16, A-24, Coxsackie virus type B1, B2, B3, B4, B5, cytomegalovirus (CMV), deltavirus, dengue virus, Ebola virus, echovirus, EEE virus, enterovirus type 7, 70, Epstein-Barr virus (EBV), filovirus, flavivirus, foot and mouth disease virus, FSME virus, hantavirus type Hantaan, Seoul, Dobrava (Belgrade), Puumala. Sin Nombre, Black Creek Canal, Bayou, New York-1, hantavirus, hepadnavirus, hepatitis A virus, hepatitis B virus, hepatitis C virus, hepatitis D virus, hepatitis E virus, hepatitis F virus, hepatitis G virus, herpes simplex virus (HSV), herpes simplex virus type 1 and 2 (HSV-1, HSV-2), herpesvirus, HIV, HIV-1, HIV-2, human papilloma virus (HPV), human T cell leukemia virus, human T-cell lymphotrophic virus type I and II (HTLV-I, -II), influenza virus, influenza virus type A (H5N1) and (H3N2), influenza virus type A, B, C, Japanese encephalitis virus, JC virus, juninvirus, Kaposi's sarcoma-associated virus, LaCross virus, Lassavirus, lentivirus, lymphocytic choriomeningitis virus, machupovirus, Marburg virus, measles virus, Molluscum virus, mumps virus, Norwalk virus, orfvirus, orthomyxovirus, papovavirus, parainfluenza virus type 1, 2, 3, parainfluenza virus, paramyxovirus type 1, 2, 3, 4, paramyxovirus, parvovirus B19, parvovirus, picornavirus, poliovirus, poxvirus, rabies virus, Rabies virus, reovirus, respiratory syncytial virus, rhabdovirus, rhinoviruses, rotavirus, Rubella virus, rubeola virus, rubivirus, SARS virus, Simian virus 40, SLE virus, togavirus, Torque teno virus, vaccinia virus, varicella zoster virus, variola virus, Vicia faba endornavirus, WEE virus, West Nile virus and/or Yellow fever virus.

The term "spp." in connection with any microorganism is intended to comprise for the purpose of the present invention all members of a given genus, including species, subspecies and others. The term "Trypanosoma spp." for instance is intended to comprise all members of the genus Trypanosoma, such as Trypanosoma cruzi, Trypanosoma brucei, Trypanosoma brucei brucei and Trypanosoma brucei gambiense.

For the purpose of the present invention, the term "treatment" is also intended to include prophylactic treatment or alleviation.

In a further preferred embodiment, the compounds of the invention, and in particular alkyl phospholipid derivative **compounds 1** to **20** and **22**, can be used for the manufacture of a medicament for the treatment or prophylaxis of diseases and/or pathophysiological conditions in mammals that are caused by microorganisms, where the microorganism is a fungus and selected from the group consisting of "Absidia spp., Acremonium spp., Alternaria spp., Aspergillus spp., Bipolaris spp., Candida spp., Cladophialophora spp., Cladosporium spp., Coccidioides spp., Coniothyrium spp., Cryptococcus spp., Cunninghamella spp., Curvularia spp., Epidermophyton spp., Exophiala spp., Exserohilum spp., Fonsecaea spp., Fusarium spp., Histoplasma spp., Lacazia spp., Lasiodiplodia spp., Leptosphaeria spp., Madurella spp., Microsporum spp., Mucor spp., Mucorales spp., Neotestudina spp., Ochroconis spp., Onychocola spp., Paecilomyces spp., Paracoccidioides spp., Penicillium spp., Phialophora spp., Pseudallesheria spp., Pyrenochaeta spp., Rhizomucor spp., Rhizopus spp., Scedosporium spp., Scopulariopsis spp., Scytalidium spp., Sporothrix spp., Trichophyton spp. and/or Wangiella spp." More preferably, the fungus is selected from the group consisting of "Absidia spp., Aspergillus spp., Bipolaris spp., Candida spp., Cryptococcus spp., Cunninghamella spp., Exophiala spp., Fusarium spp., Paecilomyces spp., Rhizopus spp. and/or Scedosporium spp." Most preferably, the fungus is selected from the group consisting of "Absidia corymbifera, Aspergillus flavus, Aspergillus fumigatus, Aspergillus terreus, Bipolaris australiensis, Candida albicans, Candida glabrata, Candida krusei, Candida parapsilosis, Candida tropicalis, Cryptococcus gattii, Cryptococcus neoformans, Cunninghamella bertholletitae, Exophiala jeanselmei, Exophiala spinifera, Fusarium solani, Paecilomyces lilacinus, Rhizopus oryzae, Scedosporium apiospermum and/or Scedosporium prolificans.

In another preferred embodiment, the compounds of the invention, and in particular alkyl phospholipid derivative **compounds 1** to **20**, can be used for the manufacture of a medicament for the treatment or prophylaxis of diseases and/or pathophysiological conditions in mammals that are caused by microorganisms, where the microorganism is a protozoon and selected from the group consisting of "Acanthamoeba spp., Amoeba spp., Babesia spp., Balantidium spp., Cryptosporidium spp., Cyclospora spp., Dientamoeba spp., Echinamoeba spp., Endolimax spp., Entamoeba spp., Enterocytozoon spp., Giardia spp., Hartmanella spp., Isospora spp., Jodamoeba spp., Lamblia spp., Leishmania spp., Microsporidium spp., Naegleria spp., Nosema spp., Paramecium spp., Paramoeba spp., Penumocystis spp., Plasmodium spp., Sarcocystis spp., Tetrahymena spp., Toxoplasma spp., Trichomonas spp. and/or Trypanosoma spp.". More preferably, the protozoon is selected from the group consisting of "Leishmania spp., Plasmodium spp., Toxoplasma spp. and/or Trypanosoma spp." Most preferably, the protozoon is selected from the group consisting of "Leishmania braziliensis braziliensis, Leishmania chagasi, Leishmania donovani, Leishmania infantum, Leishmania major, Leishmania mexicana, Leishmania mexicana amazonensis, Leishmania mexicana mexicana, Leishmania tropica, Leishmania Viannia, Leishmania Viannia braziliensis, Leishmania Viannia guyanensis, Leishmania Viannia panamensis, Leishmania Viannia peruviana, Plasmodium falciparum, Plasmodium malariae, Plasmodium ovale, Plasmodium vivax, Toxoplasma gondii, Trypanosoma brucei, Trypanosoma brucei brucei, Trypanosoma brucei gambiense, Trypanosoma brucei rhodesiense, Trypanosoma cruzi, Trypanosoma cruzi cruzi, Trypanosoma cruzi marinkellei, Trypanosoma equinum, Trypanosoma equiperdum, Trypanosoma evansi, Trypanosoma theileri and/or Trypanosoma vivax". Even more preferably, the alkyl phospholipid derivative is selected from the group consisting of: "**Compound 1, 3, 4, 5, 6, 7, 8, 10, 11, 12, 13, 14, 15, 18, 19** and/or **compound 20**".

In another preferred embodiment, the compounds of the invention, and in particular alkyl phospholipid derivative **compounds 1** to **20**, can be used for the manufacture of a medicament for the treatment or prophylaxis of diseases and/or pathophysiological conditions in mammals that are caused by microorganisms, where the microorganism is a bacterium and selected from the group consisting of "gram-positive bacterium and/or gram-negative bacterium". More preferably, the compounds of the invention, and in particular alkyl phospholipid derivative **compounds 1** to **20**, can be used for the manufacture of a medicament for the treatment or prophylaxis of diseases and/or pathophysiological conditions in mammals that are caused by microorganisms, where the microorganism is a bacterium and selected from the group consisting of "Acinetobacter spp., Actinobacillus spp., Actinomyces spp., Aeromonas spp., Agrobacterium spp., Alcaligenes spp., Anaplasma spp., Aquifex spp., Bacillus spp., Bacteroides spp., Bifidobacterium spp., Bordetella spp., Borrelia spp., Bradyrhizobium spp., Branhamella spp., Brucella spp., Buchnera spp., Burkholderia spp., Campylobacter spp., Capnocytophaga spp., Cardiobacterium spp., Caulobacter spp., Chlamydia spp., Chlamydophila spp., Chlorobium spp., Citrobacter spp., Clostridium spp., Corynebacterium spp., Coxiella spp., Deinococcus spp., Ehrlichia spp., Eikenella spp., Enterobacter spp., Enterococcus spp., Erysipelothrix spp., Escherichia spp., Francisella spp., Fusobacterium spp., Gardnerella spp., Gemella spp., Haemophilus spp., Heliobacter spp., Kingella spp., Kitasatospora spp., Klebsiella spp., Lactobacillus spp., Legionella spp., Leptospira spp., Listeria spp., Mannheimia spp., Mesorhizobium spp., Moraxella spp., Morganella spp., Mycobacterium spp., Mycoplasma spp., Neisseria spp., Neorickettsia spp., Nitrosomonas spp., Nocardia spp., Oceanobacillus spp., Orientia spp., Paracoccus spp., Pasteurella spp., Peptostreptococcus spp., Plesiomonas spp., Porphyromonas spp., Prevotella spp., Propionibacterium spp., Proteus spp., Providencia spp., Pseudomonas spp., Psychobacter spp., Ralstonia spp., Rhodobacter spp., Rhodococcus spp., Rickettsia spp., Salmonella spp., Serratia spp., Shewanella spp., Shigella spp., Spirillum spp., Staphylococcus spp., Stenotrophomonas spp., Streptobacillus spp., Streptococcus spp., Streptomyces spp., Synechococcus spp., Synechocystis spp., Tannerella spp., Thermoanaerobacter spp., Thermotoga spp., Treponema spp., Tropheryma spp., Ureaplasma spp., Veillonella spp., Vibrio spp., Wigglesworthia spp., Wolbachia spp., Xanthomonas spp., Xylella spp., Yersinia spp. and/or Zymomonas spp." Even more preferably, the bacterium is selected from the group consisting of "Bacteroides spp., Branhamella spp., Chlamydia spp., Escherichia spp., Haemophilus spp., Klebsiella spp., Mycobacterium spp., Mycoplasma spp., Proteus spp., Pseudomonas spp., Serratia spp., Staphylococcus spp. and/or Streptococcus spp.". Most preferably, the bacterium is selected from the group consisting of "Bacteroides fragilis, Branhamella catarrhalis, Chlamydia pneumoniae, Chlamydia psittaci, Chlamydia trachomatis, Escherichia coli, Haemophilus ducreyi, Haemophilus influenzae, Haemophilus parainfluenzae, Haemophilus ducreyi, Klebsiella granulomatis, Klebsiella pneumoniae, Klebsiella pneumoniae ozaenae, Klebsiella pneumoniae pneumoniae, Klebsiella pneumoniae rhinoscleromatis, Mycobacterium africanum, Mycobacterium avium, Mycobacterium avium paratuberculosis, Mycobacterium bovis, Mycobacterium bovis bovis, Mycobacterium bovis caprae, Mycobacterium chelonae, Mycobacterium fortuitum, Mycobacterium intracellulare, Mycobacterium kansasii, Mycobacterium leprae, Mycobacterium malmoense, Mycobacterium marinum, Mycobacterium scrofulaceum, Mycobacterium tuberculosis, Mycobacterium ulcerans, Mycoplasma genitalium, Mycoplasma hominis, Mycoplasma pneumoniae, Proteus mirabilis, Proteus vulgaris, Pseudomonas aeruginosa, Pseudomonas alcaligenes, Pseudomonas flurescens, Pseudomonas putida, Pseudomonas stutzeri, Serratia marcescens, Staphylococcus aureus, Staphylococcus epidermidis, Staphylococcus hemolyticus, Staphylococcus saprophyticus, Streptococcus agalactiae, Streptococcus faecalis, Streptococcus milleri, Streptococcus mutans, Streptococcus pneumoniae, Streptococcus pyogenes, Streptococcus salivarius and/or Streptococcus viridans". In an even more preferred embodiment, the compounds of the invention, and in particular alkyl phospholipid derivative compounds 1 to 20, are bactericidal (bacteria-killing) and/or bacteriostatic (bacterial growth/reproduction inhibiting). In this context, the compounds of the invention, and in particular alkyl phospholipid derivative compounds 1 to 20, can even be bactericidal to one or more certain bacterial geni, species, subspecies, strains etc. but only bacteriostatic to another or further bacterial geni, species, subspecies, strains etc. and vice versa.

In another preferred embodiment, the compounds of the invention, and in particular alkyl phospholipid derivative **compounds 1** to **20**, can be used for the manufacture of a medicament for the treatment or prophylaxis of diseases and/or pathophysiological conditions in mammals that are caused by microorganisms, where the microorganism is a virus and selected from the group consisting of "DNA virus; dsDNA virus, ssDNA virus; RNA virus; dsRNA virus; (+)ssRNA virus; (-)ssRNA virus; DNA/RNA reverse transcribing virus; ssRNA-RT virus and/or dsDNA-RT virus". More preferably, the compounds of the invention, and in particular alkyl phospholipid derivative **compounds 1** to **20**, can be used for the manufacture of a medicament for the treatment or prophylaxis of diseases and/or pathophysiological conditions in mammals that are caused by microorganisms, where the microorganism is a virus and selected from the group consisting of "adenovirus type 1, 2, 3, 5, 11, 21, adenovirus, alphavirus, arbovirus, arenavirus, borna disease virus, bunyavirus, calicivirus, California encephalitis virus, Colorado tick fever virus, coronavirus cowpox virus, coxsackie type A virus, coxsackie type B virus, coxsackie virus type A-16, A-24, Coxsackie virus type B1, B2, B3, B4, B5, cytomegalovirus (CMV), deltavirus, dengue virus, Ebola virus, echovirus, EEE virus, enterovirus type 7, 70, Epstein-Barr virus (EBV), filovirus, flavivirus, foot and mouth disease virus, FSME virus, hantavirus type Hantaan, Seoul, Dobrava (Belgrade), Puumala. Sin Nombre, Black Creek Canal, Bayou, New York-1, hantavirus, hepadnavirus, hepatitis A virus, hepatitis B virus, hepatitis C virus, hepatitis D virus, hepatitis E virus, hepatitis F virus, hepatitis G virus, herpes simplex virus (HSV), herpes simplex virus type 1 and 2 (HSV-1, HSV-2), herpesvirus, HIV, HIV-1, HIV-2, human papilloma virus (HPV), human T cell leukemia virus, human T-cell lymphotrophic virus type I and II (HTLV-I, -II), influenza virus, influenza virus type A (H5N1) and (H3N2), influenza virus type A, B, C, Japanese encephalitis virus, JC virus, juninvirus, Kaposi's sarcoma-associated virus, LaCross virus, Lassavirus, lentivirus, lymphocytic choriomeningitis virus, machupovirus, Marburg virus, measles virus, Molluscum virus, mumps virus, Norwalk virus, orfvirus, orthomyxovirus, papovavirus, parainfluenza virus type 1, 2, 3, parainfluenza virus, paramyxovirus type 1, 2, 3, 4, paramyxovirus, parvovirus B19, parvovirus, picornavirus, poliovirus, poxvirus, rabies virus, Rabies virus, reovirus, respiratory syncytial virus, rhabdovirus, rhinoviruses, rotavirus, Rubella virus, rubeola virus, rubivirus, SARS virus, Simian virus 40, SLE virus, togavirus, Torque teno virus, vaccinia virus, varicella zoster virus, variola virus, Vicia faba endornavirus, WEE virus, West Nile virus and/or Yellow fever virus". Even more preferably, the alkyl phospholipid derivative is selected from the group consisting of: "**Compound 1, 2, 3, 4, 5, 6, 7, 8, 10, 11, 12, 13, 14, 15, 16, 18, 19** and/or **compound 20**".

All stereoisomers of the compounds of the invention, and in particular alkyl phospholipid derivative **compounds 1 to 20** and **22**, are contemplated, either in a mixture or in pure or substantially pure form. The compounds of the invention, and in particular alkyl phospholipid derivative **compounds 1 to 20** and **22**, can have asymmetric centers at any of the carbon atoms. Consequently, they can exist in the form of their racemates, in the form of the pure enantiomers and/or diastereomers or in the form of mixtures of these enantiomers and/or diastereomers. The mixtures may have any desired mixing ratio of the stereoisomers.

Thus, for example, the compounds of the invention, and in particular alkyl phospholipid derivative **compounds 1 to 20** and **22**, which have one or more centers of chirality and which occur as racemates or as diastereomer mixtures can be fractionated by methods known per se into their optical pure isomers, i.e. enantiomers or diastereomers. The separation of the compounds of the invention can take place by column separation on chiral or nonchiral phases or by recrystallization from an optionally optically active solvent or with use of an optically active acid or base or by derivatization with an optically active reagent such as, for example, an optically active alcohol, and subsequent elimination of the radical.

Where possible, the compounds of the invention, and in particular alkyl phospholipid derivative **compounds 1 to 20** and **22**, may be in the form of the tautomers.

It is likewise possible for the compounds of the invention, and in particular alkyl phospholipid derivative **compounds 1 to 20** and **22**, to be in the form of any desired prodrugs such as, for example, esters, carbonates or phosphates, in which cases the actually biologically active form is released only through metabolism. Any compound that can be converted in vivo to provide the bioactive agent (i.e. compounds of the invention, and in particular alkyl phospholipid derivative **compounds 1 to 20** and **22**) is a prodrug within the scope and spirit of the invention.

Various forms of prodrugs are well known in the art and are described for instance in:
(i) The Practice of Medicinal Chemistry (Wermuth CG et al., Chapter 31, Academic Press 1996);
(ii) Design of Prodrugs (editor: Bundgaard H, Elsevier 1985); and
(iii) A Textbook of Drug Design and Development (Krogsgaard-Larson P and Bundgaard H, eds., Chapter 5: 113 - 191, Harwood Academic Publishers 1991).

Said references are incorporated herein by reference.

It is further known that chemical substances are converted in the body into metabolites which may where appropriate likewise elicit the desired biological effect - in some circumstances even in more pronounced form.

Any biologically active compound that was converted in vivo by metabolism from any of the compounds of the invention, and in particular alkyl phospholipid derivative **compounds 1 to 20** and **22**, is a metabolite within the scope and spirit of the invention.

The compounds of the invention, and in particular alkyl phospholipid derivative **compounds 1 to 20** and **22**, can, if they have a sufficiently basic group such as, for example, a secondary or tertiary amine, be converted with inorganic and organic acids into salts. The pharmaceutically acceptable salts of the compounds of the invention, and in particular alkyl phospholipid derivative **compounds 1 to 20** and **22**, are preferably formed with hydrochloric acid, hydrobromic acid, iodic acid, sulfuric acid, phosphoric acid, methanesulfonic acid, p-toluenesulfonic acid, carbonic acid, formic acid, acetic acid, sulfoacetic acid, trifluoroacetic acid, oxalic acid, malonic acid, maleic acid, succinic acid, tartaric acid, racemic acid, malic acid, embonic acid, mandelic acid, fumaric acid, lactic acid, citric acid, taurocholic acid, glutaric acid, stearic acid, glutamic acid or aspartic acid. The salts which are formed are, inter alia, hydrochlorides, chlorided, hydrobromides, bromides, iodides, sulfates, phosphates, methanesulfonates, tosylates, carbonates, bicarbonates, formates, acetates, sulfoacetates, triflates, oxalates, malonates, maleates, succinates, tartrates, malates, embonates, mandelates, fumarates, lactates, citrates, glutarates, stearates, aspartates and glutamates. The stoichiometry of the salts formed from the compounds of the invention may moreover be an integral or non-integral multiple of one.

The compounds of the invention, and in particular alkyl phospholipid derivative **compounds 1 to 20** and **22**, can, if they contain a sufficiently acidic group such as, for example, the carboxy, sulfonic acid, phosphoric acid or a phenolic group, be converted with inorganic and organic bases into their physiologically tolerated salts. Examples of suitable inorganic bases are ammonium, sodium hydroxide, potassium hydroxide, calcium hydroxide, and of organic bases are ethanolamine, diethanolamine, triethanolamine, ethylenediamine, t-butylamine, t-octylamine, dehydroabietylamine, cyclohexylamine, dibenzylethylene-diamine and lysine. The stoichiometry of the salts formed from the compounds of the invention can moreover be an integral or non-integral multiple of one.

It is likewise possible for the compounds of the invention, and in particular alkyl phospholipid derivative **compounds 1 to 20** and **22**, to be in the form of their solvates and, in particular, hydrates which can be obtained for example by crystallization from a solvent or from aqueous solution. It is moreover possible for one, two, three or any number of solvate or water molecules to combine with the compounds of the invention to give solvates and hydrates.

In a preferred embodiment, the compounds of the invention, and in particular alkyl phospholipid derivative **compounds 1 to 20** and **22**, are available in the form of their hydrates, with any number of water molecules combined/complexed to them, including integer and non-integer ratios, such as 1:0,5; 1:1; 1:1,5; 1:2; 1:2,5; 1:3; 1:3,5; 1:4 etc.

It is known that chemical substances form solids which exist in different order states which are referred to as polymorphic forms or modifications. The various modifications of a polymorphic substance may differ greatly in their physical properties. The compounds of the invention, and in particular alkyl phospholipid derivative **compounds 1 to 20** and **22**, can exist in various polymorphic forms and certain modifications may moreover be metastable. All these polymorphic forms of the compounds are to be regarded as belonging to the invention.

The compounds of the invention, and in particular alkyl phospholipid derivative **compounds 1 to 20** and **22**, are suitable for the treatment or prophylaxis of diseases and/or pathophysiological conditions in mammals that are caused by microorganisms, in particular where the microorganism is selected from the group consisting of "fungus, protozoon, bacterium and/or virus".

For the purpose of the present invention, all known diseases and/or pathophysiological conditions in mammals are intended to be comprised that are caused by fungi.

Examples of such fungal diseases and/or pathophysiological conditions are aspergillosis, blastomycosis, candidiasis, chromoblastomycosis, coccidioidomycosis, cryptococcosis, dermatomycosis, dermatophytosis, histoplasmosis, lobomycosis, mucormycosis, mycetoma, mycotic keratitis, oculomycosis, onychomycosis, otomycosis, paracoccidiomycosis, phaeohyphomycosis, piedra, pityriasis versicolor, rhinosporidiosis, sporotrichosis, tinea barbae, tinea capitis, tinea corporis, tinea cruris, tinea favosa, tinea nigra, tinea pedis, tinea unguium and/or zygomycosis as well as their different forms and subforms.

For the purpose of the present invention, all known diseases and/or pathophysiological conditions in mammals are intended to be comprised that are caused by protozoa.

Examples of such protozoal diseases and/or pathophysiological conditions are African trypanosomiasis, American trypanosomiasis, amoebiasis, amoebic dysentery, amoebic keratitis, amoebic meningoencephalitis, amoebic vaginitis, babesiosis, chagas disease, coccidiosis, cryptosporidiosis, cutaneous leishmaniasis, cyclosporiasis, dientamoebiasis, entamoebiasis, giardiasis, isosporiasis, lambliasis, leishmaniasis, malaria, malaria quartana, malaria tertiana, malaria tropica, microsporidosis, mucocutaneous leishmaniasis, pneumocystosis, sarcosporidosis, sleeping sickness, toxoplasmosis, trichomoniasis, trypanosomiasis and/or visceral leishmaniasis as well as their different forms and subforms. Preferably, diseases and/or pathophysiological conditions in mammals that are caused by protozoa are selected from the group consisting of "trypanosomiasis, American trypanosomiasis, chagas disease, leishmaniasis, cutaneous leishmaniasis, mucocutaneous leishmaniasis, visceral leishmaniasis, malaria, malaria tropica and/or toxoplasmosis as well as their different forms and subforms".

For the purpose of the present invention, all known diseases and/or pathophysiological conditions in mammals are intended to be comprised that are caused by bacteria.

Examples of such bacterial diseases and/or pathophysiological conditions are actimomycosis, acute epiglottitis, acute otitis media, acute purulent (septic) arthritis, acute purulent meningitis, anthrax, appendicitis, bacillary dysentery, bacteremia, black death, borderline leprosy, borreliose, botulism, breast abscesses, bronchitis, brucellosis, bubonic plague, carbuncles, cellulitis, cephalic tetanus, cerebritis, cervicitis, Cholera, conjunctivitis, cutaneous anthrax, cystitis, dermatitis, diarrhea, empyema, encephalitis, endocarditis, enteric fever, enteritis, enterocolitis, epididymitis, erysipelas, erysipelothricosis, exfoliation, extrapulmonary tubercolosis, food poisining, furuncles, gas gangrene, gastritis, gastroenteritis, gastrointestinal tract (GI) infections, gastrointestinal tubercolosis, genitourinary tubercolosis, glomerulonephritis, hematogenous or hymphohematogenous tuberculosis, impetigo, intra-abdominal infections, laryngitis, lepromatous leprosy, leprosy, leptospirosis, listeriosis, Localized tetanus, Lyme disease, mastitis, melioidosis, meningitis, meningoencephalitis, miliary tubercolosis, myonecrosis, nausea, necrotizing enteritis, neonatal listeriosis, neonatal sepsis, nocardiosis, ophthalmitis, osteomyelitis, osteomyelitis, otitis media, otitis, pancreatitis, parotitis, pericarditis, peritonitis, pertussis, Pestis, pharyngitis, plague, pneumonia, postpartum sepsis, primary listeremia, proctitis, prostatis, puerperal sepsis, pulmonary anthrax, pulmonary tubercolosis, Pustular or bullous skin subcutaneous abscesses, pyleonephritis, pyoderma, Rabbit/Deer Fly Fever, rat-bite fever, relapsing fever, rheumatic fever, rhinithis, rhomboencephalitis, salmonellosis, salpingitis, scalded skin syndrome, scarlet fever, sepsis, septic arthritis, septic thrombophlebitis, septicemia, shigellosis, sinusitis, skin infections, stitch abscesses, syphilis, tetanus, tick/recurrent/famine fever, tonsillitis, Toxic shock syndrome, tracheobronchitis, treponematosis, tubercolosis, tuberculoid leprosy, tuberculous lymphadenitis, tuberculous meningitis, tuberculous pericarditis, tuberculous peritonitis, tularemia, typhoid fever, ulcus, undulant/Malta/Mediterranean/Gibraltar Fever, urethritis, urinary tract infections (UTls), vomitting and/or wound infections as well as their different forms and subforms. Preferably, diseases and/or pathophysiological conditions in mammals that are caused by bacteria are selected from the group consisting of "acute otitis media, bronchitis, dermatitis, encephalitis, endocarditis, gastritis, gastroenteritis, gastrointestinal tract (GI) infections, laryngitis, meningitis, otitis media, pericarditis, pharyngitis, pneumonia, sepsis, sinusitis, skin infections, tuberculosis and/or urinary tract infections (UTls) as well as their different forms and subforms".

For the purpose of the present invention, all known diseases and/or pathophysiological conditions in mammals are intended to be comprised that are caused by viruses.

Examples of such viral diseases and/or pathophysiological conditions are acute febrile respiratory disease (AFRD), acute hemorrhagic conjunctivitis, acute hemorrhagic cystitis, acute pharyngoconjunctival fever (APC), acute posterior ganglionitis, acute respiratory disease (ARD), AIDS, arbovirus encephalitis, aseptic meningitis, borna disease, Bornholm disease (pleurodynia), breakbone/dandy fever, bronchiolitis, bronchitis, Burkitt's lymphoma, California encephalitis, Castleman's disease, cervical cancer, chickenpox, Chikungunya disease, Colorado tick fever, common cold, conjunctivitis, Cowpox, Creutzfeldt-Jakob disease, Croup, cytomegalic inclusion disease, dengue, dengue hemorrhagic fever, Devil's grip (pleurodynia), Eastern equine encephalitis, Ebola hemorrhagic fever, Ebola virus infection, encephalomyelitis, epidemic keratoconjunctivitis (EKC), epidemic nephrosonephritis, erythema infectiosum, fatal familial insomnia, fifth disease, flue, foot and mouth disease (hand-foot-mouth disease), gastroenteritis, geniculate zoster, genital herpes, genital warts, German measles, Gerstmann-Straussier-Scheinker disease, gingivostomatitis, Hantaan-Korean hemorrhagic fever, hantavirus hemorrhagic fever, hantavirus pulmonary syndrome (HPS), hemorrhagic fever with renal syndrome (HFRS), hepatitis A, hepatitis B, hepatitis C, hepatitis D, hepatitis E, herpangina, herpes labialis, herpes zoster, herpetic stomatitis, HIV infection, Hodgkin's disease, HTLV-I-associated myelopathy, hydrophobia, infectious myocarditis, infectious pericarditis, influenza, Japanese encephalitis, jungle (sylvatic) yellow fever, Junin Argentinian hemorrhagic fever, Kaposi's sarcoma, keratitis, keratoconjunctivitis, Korean Hemorrhagic Fever, kuru, LaCrosse encephalitis, laryngitis, laryngotracheobronchitis (types 1 and 2), Lassa hemorrhagic fever, leukemia, lymphocytic choriomeningitis, lymphoma, Machupo Bolivian hemorrhagic fever, Marburg hemorrhagic fever, Mayaro disease, measles, meningoencephalitis, Molluscum contagiosum, mononucleosis, mononucleosis-like syndrome, multifocal leukoencephalopathy, mumps, nasopharyngeal carcinoma, nausea, neonatal herpes, nephropathia epidemica, ophthalmic herpes zoster, orchitis, orf, parainfluenza, parotitis, pharyngitis, pharyngoconjunctival fever, pleurodynia, pneumonia, polio, poliomyelitis, progressive multifocal leukencephalopathy (PML), rabies, roseola infantum, rubella, rubella panencephalitis, sclerosing panencephalitis, severe acute respiratory syndrome (SARS), shingles (zoster), slapped cheek disease (erythema infectiosum), smallpox, soeola, St. Louis encephalitis, temporal lobe encephalitis, tracheobronchitis, transmissable spongiform encephalopathies, tropical spastic paraparesis, urban yellow fever, urethritis, varicella, verrucae, vomitting, warts, Western equine encephalitis, Yellow fever, zona and/or zoster.

In a preferred embodiment, the compounds of the invention, and in particular alkyl phospholipid derivative **compounds 1 to 20** and **22** can be used for the manufacture of a medicament for the treatment or prophylaxis of diseases and/or pathophysiological conditions in mammals that are caused by microorganisms, where the disease and/or pathophysiological condition is selected from the group consisting of "aspergillosis, blastomycosis, candidiasis, chromoblastomycosis, coccidioidomycosis, cryptococcosis, dermatomycosis, dermatophytosis, histoplasmosis, lobomycosis, mucormycosis, mycetoma, mycotic keratitis, oculomycosis, onychomycosis, otomycosis, paracoccidiomycosis, phaeohyphomycosis, piedra, pityriasis versicolor, rhinosporidiosis, sporotrichosis, tinea barbae, tinea capitis, tinea corporis, tinea cruris, tinea favosa, tinea nigra, tinea pedis, tinea unguium, zygomycosis; African trypanosomiasis, American trypanosomiasis, amoebiasis, amoebic dysentery, amoebic keratitis, amoebic meningoencephalitis, amoebic vaginitis, babesiosis, chagas disease, coccidiosis, cryptosporidiosis, cutaneous leishmaniasis, cyclosporiasis, dientamoebiasis, entamoebiasis, giardiasis, isosporiasis, lambliasis, leishmaniasis, malaria, malaria quartana, malaria tertiana, malaria tropica, microsporidosis, mucocutaneous leishmaniasis, pneumocystosis, sarcosporidosis, sleeping sickness, toxoplasmosis, trichomoniasis, trypanosomiasis, visceral leishmaniasis, actimomycosis, acute epiglottitis, acute otitis media, acute purulent (septic) arthritis, acute purulent meningitis, anthrax, appendicitis, bacillary dysentery, bacteremia, black death, borderline leprosy, borreliose, botulism, breast abscesses, bronchitis, brucellosis, bubonic plague, carbuncles, cellulitis, cephalic tetanus, cerebritis, cervicitis, Cholera, conjunctivitis, cutaneous anthrax, cystitis, dermatitis, diarrhea, empyema, encephalitis, endocarditis, enteric fever, enteritis, enterocolitis, epididymitis, erysipelas, erysipelothricosis, exfoliation, extrapulmonary tubercolosis, food poisining, furuncles, gas gangrene, gastritis, gastroenteritis, gastrointestinal tract (GI) infections, gastrointestinal tubercolosis, genitourinary tubercolosis, glomerulonephritis, hematogenous or hymphohematogenous tuberculosis, impetigo, intra-abdominal infections, laryngitis, lepromatous leprosy, leprosy, leptospirosis, listeriosis, Localized tetanus, Lyme disease, mastitis, melioidosis, meningitis, meningoencephalitis, miliary tubercolosis, myonecrosis, nausea, necrotizing enteritis, neonatal listeriosis, neonatal sepsis, nocardiosis, ophthalmitis, osteomyelitis, osteomyelitis, otitis media, otitis, pancreatitis, parotitis, pericarditis, peritonitis, pertussis, Pestis, pharyngitis, plague, pneumonia, postpartum sepsis, primary listeremia, proctitis, prostatis, puerperal sepsis, pulmonary anthrax, pulmonary tubercolosis, Pustular or bullous skin subcutaneous abscesses, pyleonephritis, pyoderma, Rabbit/Deer Fly Fever, rat-bite fever, relapsing fever, rheumatic fever, rhinithis, rhomboencephalitis, salmonellosis, salpingitis, scalded skin syndrome, scarlet fever, sepsis, septic arthritis, septic thrombophlebitis, septicemia, shigellosis, sinusitis, skin infections, stitch abscesses, syphilis, tetanus, tick/recurrent/famine fever, tonsillitis, Toxic shock syndrome, tracheobronchitis, treponematosis, tubercolosis, tuberculoid leprosy, tuberculous lymphadenitis, tuberculous meningitis, tuberculous pericarditis, tuberculous peritonitis, tularemia, typhoid fever, ulcus, undulant/Malta/Mediterranean/Gibraltar Fever, urethritis, urinary tract infections (UTIs), vomitting, wound infections, acute febrile respiratory disease (AFRD), acute hemorrhagic conjunctivitis, acute hemorrhagic cystitis, acute pharyngoconjunctival fever (APC), acute posterior ganglionitis, acute respiratory disease (ARD), AIDS, arbovirus encephalitis, aseptic meningitis, borna disease, Bornholm disease (pleurodynia), breakbone/dandy fever, bronchiolitis, bronchitis, Burkitt's lymphoma, California encephalitis, Castleman's disease, cervical cancer, chickenpox, Chikungunya disease, Colorado tick fever, common cold, conjunctivitis, Cowpox, Creutzfeldt-Jakob disease, Croup, cytomegalic inclusion disease, dengue, dengue hemorrhagic fever, Devil's grip (pleurodynia), Eastern equine encephalitis, Ebola hemorrhagic fever, Ebola virus infection, encephalomyelitis, epidemic keratoconjunctivitis (EKC), epidemic nephrosonephritis, erythema infectiosum, fatal familial insomnia, fifth disease, flue, foot and mouth disease (hand-foot-mouth disease), gastroenteritis, geniculate zoster, genital herpes, genital warts, German measles, Gerstmann-Straussler-Scheinker disease, gingivostomatitis, Hantaan-Korean hemorrhagic fever, hantavirus hemorrhagic fever, hantavirus pulmonary syndrome (HPS), hemorrhagic fever with renal syndrome (HFRS), hepatitis A, hepatitis B, hepatitis C, hepatitis D, hepatitis E, herpangina, herpes labialis, herpes zoster, herpetic stomatitis, HIV infection, Hodgkin's disease, HTLV-I-associated myelopathy, hydrophobia, infectious myocarditis, infectious pericarditis, influenza, Japanese encephalitis, jungle (sylvatic) yellow fever, Junin Argentinian hemorrhagic fever, Kaposi's sarcoma, keratitis, keratoconjunctivitis, Korean Hemorrhagic Fever, kuru, LaCrosse encephalitis, laryngitis, laryngotracheobronchitis (types 1 and 2), Lassa hemorrhagic fever, leukemia, lymphocytic choriomeningitis, lymphoma, Machupo Bolivian hemorrhagic fever, Marburg hemorrhagic fever, Mayaro disease, measles, meningoencephalitis, Molluscum contagiosum, mononucleosis, mononucleosis-like syndrome, multifocal leukoencephalopathy, mumps, nasopharyngeal carcinoma, nausea, neonatal herpes, nephropathia epidemica, ophthalmic herpes zoster, orchitis, orf, parainfluenza, parotitis, pharyngitis, pharyngoconjunctival fever, pleurodynia, pneumonia, polio, poliomyelitis, progressive multifocal leukencephalopathy (PML), rabies, roseola infantum, rubella, rubella panencephalitis, sclerosing panencephalitis, severe acute respiratory syndrome (SARS), shingles (zoster), slapped cheek disease (erythema infectiosum), smallpox, soeola, St. Louis encephalitis, temporal lobe encephalitis, tracheobronchitis, transmissable spongiform encephalopathies, tropical spastic paraparesis, urban yellow fever, urethritis, varicella, verrucae, vomitting, warts, Western equine encephalitis, Yellow fever, zona and/or zoster as well as their different forms and subforms" and preferably is selected from the group consisting of "trypanosomiasis, American trypanosomiasis, chagas disease, leishmaniasis, cutaneous leishmaniasis, mucocutaneous leishmaniasis, visceral leishmaniasis, malaria, malaria tropica, toxoplasmosis, acute otitis media, bronchitis, dermatitis, encephalitis, endocarditis, gastritis, gastroenteritis, gastrointestinal tract (Gl) infections, laryngitis, meningitis, otitis media, pericarditis, pharyngitis, pneumonia, sepsis, sinusitis, skin infections, tuberculosis and/or urinary tract infections (UTls) as well as their different forms and subforms".

In another aspect, the object of the invention has surprisingly been solved by providing the compounds of the invention, and in particular alkyl phospholipid derivatives selected from the group consisting of: "**Compound 1, 2, 3, 4, 5, 6, 7, 8, 9, 10, 11, 12, 13, 14, 15, 16, 17, 18, 19** and/or **compound 20**" for use for the manufacture of a medicament for the treatment of tumors in mammals.

The term "tumor" or "cancer" is intended for the purpose of the present invention to comprise all known mammalian benign and/or malign tumors, i.e. the various known tumors, cancers, neoplasms and/or ulcers of any tissue and/or organ in mammals.

As illustrated supra, the compounds of the invention, and in particular alkyl phospholipid derivative compounds 1 to 20 and 22 are useful for the treatment or prophylaxis of diseases and/or pathophysiological conditions in mammals as defined herein. They can be administered to various mammalian species, including human.

For the purpose of the present invention, all mammalian species are regarded as being comprised. In a preferred embodiment, such mammals are selected from the group consisting of "human, domestic animals, cattle, livestock, pets, cow, sheep, pig, goat, horse, pony, donkey, hinny, mule, hare, rabbit, cat, dog, guinea pig, hamster, rat, mouse". More preferably, such mammals are human.

In a further aspect of the present invention, the compounds of the invention, and in particular alkyl phospholipid derivative **compounds 1 to 20** and **22**, are used in combination with at least one additional pharmacologically active substance.

In a yet further aspect, the object of the invention has surprisingly been solved by using alkyl phospholipid derivatives selected from the group consisting of "miltefosine (hexadecylphosphocholine), perifosine (octadecyl-1,1-dimethyl-piperidino-4-yl-phosphate) and/or erucylphosphocholine [(13Z)-docosenylphosphocholine]" in combination with at least one additional pharmacologically active substance.

Depending on the purpose of the combined use, such additional pharmacologically active substance may be other alkyl phospholipid derivatives (the compounds of the invention, and in particular alkyl phospholipid derivative **compounds 1 to 20** and **22**, and/or known alkyl phospholipid derivatives, such as miltefosine, perifosine and/or erucylphosphocholine) and/or other "suitable therapeutic agents" useful in the treatment and/or prophylaxis of the aforementioned diseases and/or pathophysiological conditions. Selection and combination of the additional pharmacologically active substance(s) can be easily performed by the skilled artisan on the basis of his expert knowledge and depending on the purpose of the combined use and diseases and/or pathophysiological conditions targeted.

In a preferred embodiment, the compounds of the invention, and in particular alkyl phospholipid derivative **compounds 1 to 20** and **22**, are used for the treatment and/or prophylaxis of the aforementioned diseases and/or pathophysiological conditions in the form of a medicament, where such medicament comprises at least one additional pharmacologically active substance.

In another preferred embodiment, the compounds of the invention, and in particular alkyl phospholipid derivative **compounds 1 to 20** and **22**, are used for the treatment and/or prophylaxis of the aforementioned diseases and/or pathophysiological conditions in the form of a medicament, where the medicament is applied before and/or during and/or after treatment with at least one additional pharmacologically active substance.

In a further preferred embodiment, alkyl phospholipid derivatives selected from the group consisting of "miltefosine (hexadecylphosphocholine), perifosine (octadecyl-1,1-dimethyl-piperidino-4-yl-phosphate) and/or erucylphosphocholine [(13Z)-docosenylphosphocholine]" are used for the treatment and/or prophylaxis of the aforementioned diseases and/or pathophysiological conditions in the form of a medicament, where such medicament comprises at least one additional pharmacologically active substance.

In a yet further preferred embodiment, alkyl phospholipid derivatives selected from the group consisting of "miltefosine (hexadecylphosphocholine), perifosine (octadecyl-1,1-dimethyl-piperidino-4-yl-phosphate) and/or erucylphosphocholine [(13Z)-docosenylphosphocholine]" are used for the treatment and/or prophylaxis of the aforementioned diseases and/or pathophysiological conditions in the form of a medicament, where the medicament is applied before and/or during and/or after treatment with at least one additional pharmacologically active substance.

The above mentioned "suitable therapeutic agents" include benznidazole (N-Benzyl-2-nitroimidazol-1-yl-acetamide; CAS Registry Number: 22994-85-0); nifurtimox [3-Methyl-4-(5-nitrofurfuryl-idenamino)tetrahydro-1,4-thiazine-1 ,1-dioxide; CAS Registry Number: 23256-30-6]; amphotericin B [(1R, 3S, 5R, 6R, 9R, 11R, 15S, 16R, 17R, 18S, 19E, 21 E, 23E, 25E, 27E, 29E, 31 E, 33R, 35S, 36R, 37S)-33-[(3-Amino-3,6-dideoxy-beta-D-mannopyranosyl)oxy]-1, 3, 5, 6, 9, 11, 17, 37-octahydroxy-15,16,18-trimethyl-13-oxo-14,39-dioxabicyclo[33.3.1]nonatriaconta-19, 21, 23, 25, 27, 29, 31-heptaene-36-carboxylic acid; CAS Registry Number: 1397-89-3]; liposomal amphotericin B (Am-Bisome^{™}; Gilead Sciences, Inc., Astellas Pharma US, Inc.) and/or paromomycin [O-2-amino-2-deoxy-alpha-D-glucopyranosyl-(1-4)-O-[O-2,6-diamino-2,6-dideoxy-beta-L-idopyranosyl-(1-3)-beta-D-ribofuranosyl-(1-5)]-2-deoxy-D-streptamine; CAS Registry Number: 7542-37-2] and preferably, a "suitable therapeutic agents" is selected of the group consisting of these agents.

The above other therapeutic agents, when employed in combination with the compounds of the present invention, may be used, for example, in those amounts indicated in the Physicians' Desk Reference (PDR) or as otherwise determined by one of ordinary skill in the art.

In a preferred embodiment, the compounds of the invention, and in particular alkyl phospholipid derivative **compounds 1 to 20**, are used for the treatment and/or prophylaxis of the aforementioned diseases and/or pathophysiological conditions in the form of a medicament, where such medicament comprises an additional pharmacologically active substance selected from the group consisting of "benznidazole (N-Benzyl-2-nitroimidazol-1-yl-acetamide); nifurtimox [3-Methyl-4-(5-nitrofurfurylidenamino)tetrahydro-1,4-thiazine-1,1-dioxide]; amphotericin B [(1R, 3S, 5R, 6R, 9R, 11R, 15S, 16R, 17R, 18S, 19E, 21 E, 23E, 25E, 27E, 29E, 31 E, 33R, 35S, 36R, 37S)-33-[(3-Amino-3,6-dideoxy-beta-D-mannopyranosyl)oxy]-1, 3, 5, 6, 9, 11, 17, 37-octahydroxy-15,16,18-trimethyl-13-oxo-14,39-dioxabicyclo[33.3.1] nonatriaconta-19,21,23,25,27,29,31-heptaene-36-carboxylic acid]; liposomal amphotericin B and/or paromomycin [O-2-amino-2-deoxy-alpha-D-glucopyranosyl-(1-4)-O-[O-2,6-diamino-2,6-d ideoxy-beta-L-idopyranosyl-(1-3)-beta-D-ribofuranosyl-(1-5)]-2-deoxy-D-streptamine]" and an alkyl phospholipid derivative selected from the group consisting of "the compounds of the invention; and in particular alkyl phospholipid derivative **compounds 1, 2, 3, 4, 5, 6, 7, 8, 9, 10, 11, 12, 13, 14, 15, 16, 17, 18, 19** and/or **compound 20**". More preferably such diseases and/or pathophysiological conditions are selected from the group consisting of "trypanosomiasis, American trypanosomiasis, chagas disease, leishmaniasis, cutaneous leishmaniasis, mucocutaneous leishmaniasis, visceral leishmaniasis, malaria and/or malaria tropica as well as their different forms and subforms".

In another preferred embodiment, the compounds of the invention, and in particular alkyl phospholipid derivative compounds 1 to 20, are used for the treatment and/or prophylaxis of the aforementioned diseases and/or pathophysiological conditions in the form of a medicament, where the medicament is applied before and/or during and/or after treatment with at least one additional pharmacologically active substance, where such additional pharmacologically active substance is selected from the group consisting of "benznidazole (N-Benzyl-2-nitroimidazol-1-yl-acetamide); nifurtimox [3-Methyl-4-(5-nitrofurfuryl-idenamino)tetrahydro-1,4-thiazine-1,1-dioxide]; amphotericin B [(1R, 3S, 5R, 6R, 9R, 11R, 15S, 16R, 17R, 18S, 19E, 21E, 23E, 25E, 27E, 29E, 31E, 33R, 35S, 36R, 37S)-33-[(3-Amino-3,6-dideoxy-beta-D-mannopyranosyl)oxy]-1, 3, 5, 6, 9, 11, 17, 37-octahydroxy-15,16,18-trimethyl-13-oxo-14,39-dioxabicyclo[33.3.1] nonatriaconta-19,21,23,25,27,29,31-heptaene-36-carboxylic acid]; liposomal amphotericin B and/or paromomycin [O-2-amino-2-deoxy-alpha-D-glucopyranosyl-(1-4)-O-[O-2,6-diamino-2,6-dideoxy-beta-L-idopyranosyl-(-3)-beta-D-ribofuranosyl-(1-5)]-2-deoxy-D-streptamine]" and the alkyl phospholipid derivative is selected from the group consisting of "the compounds of the invention; and in particular alkyl phospholipid derivative **compounds 1, 2, 3, 4, 5, 6, 7, 8, 9, 10, 11, 12, 13, 14, 15, 16, 17, 18, 19** and/or **compound 20**". More preferably such diseases and/or pathophysiological conditions are selected from the group consisting of "trypanosomiasis, American trypanosomiasis, chagas disease, leishmaniasis, cutaneous leishmaniasis, mucocutaneous leishmaniasis, visceral leishmaniasis, malaria and/or malaria tropica as well as their different forms and subforms".

In a further preferred embodiment, alkyl phospholipid derivatives selected from the group consisting of "miltefosine (hexadecylphosphocholine), perifosine (octadecyl-1,1-dimethyl-piperidino-4-yl-phosphate) and/or erucylphosphocholine [(13Z)-docosenylphosphocholine]" are used for the treatment and/or prophylaxis of the aforementioned diseases and/or pathophysiological conditions in the form of a medicament, where such medicament comprises an additional pharmacologically active substance selected from the group consisting of "benznidazole (N-Benzyl-2-nitroimidazol-1-yl-acetamide); nifurtimox [3-Methyl-4-(5-nitrofurfuryl-idenamino)tetrahydro-1,4-thiazine-1,1-dioxide]; amphotericin B [(1R, 3S, 5R, 6R, 9R, 11R, 15S, 16R, 17R, 18S, 19E, 21E, 23E, 25E, 27E, 29E, 31 E, 33R, 35S, 36R, 37S)-33-[(3-Amino-3,6-dideoxy-beta-D-mannopyranosyl)oxy]-1, 3, 5, 6, 9, 11, 17, 37-octahydroxy-15,16,18-trimethyl-13-oxo-14,39-dioxabicyclo[33.3.1] nonatriaconta-19,21,23,25,27,29,31-heptaene-36-carboxylic acid]; liposomal amphotericin B and/or paromomycin [O-2-amino-2-deoxy-alpha-D-glucopyranosyl-(1-4)-O-[O-2,6-diamino-2,6-dideoxy-beta-L-idopyranosyl-( 1-3)-beta-D-ribofuranosyl-(1-5)]-2-deoxy-D-streptamine]" and an alkyl phospholipid derivative selected from the group consisting of "miltefosine (hexadecylphosphocholine), perifosine (octadecyl-1,1-dimethyl-piperidino-4-yl-phosphate) and/or erucylphosphocholine [(13Z)-docosenyl-phosphocholine]". More preferably such diseases and/or pathophysiological conditions are selected from the group consisting of "trypanosomiasis, American trypanosomiasis, chagas disease, leishmaniasis, cutaneous leishmaniasis, mucocutaneous leishmaniasis, visceral leishmaniasis, malaria and/or malaria tropica as well as their different forms and subforms". Even more preferably, such medicament comprises miltefosine and amphotericin B, liposomal amphotericin B and/or paromomycin and is used for the treatment of leishmaniasis, cutaneous leishmaniasis, mucocutaneous leishmaniasis and/or visceral leishmaniasis as well as their different forms and subforms. Even more preferably, such medicament comprises miltefosine and benznidazole and/or nifurtimox and is used for the treatment of trypanosomiasis, American trypanosomiasis and/or chagas disease as well as their different forms and subforms.

In a yet further preferred embodiment, alkyl phospholipid derivatives selected from the group consisting of "miltefosine (hexadecylphosphocholine), perifosine (octadecyl-1,1-dimethyl-piperidino-4-yl-phosphate) and/or erucylphosphocholine [(13Z)-docosenylphosphocholine]" are used for the treatment and/or prophylaxis of the aforementioned diseases and/or pathophysiological conditions in the form of a medicament, where the medicament is applied before and/or during and/or after treatment with at least one additional pharmacologically active substance, where such additional pharmacologically active substance is selected from the group consisting of "benznidazole (N-Benzyl-2-nitroimidazol-1-yl-acetamide); nifurtimox [3-Methyl-4-(5-nitrofurfurylidenamino)tetrahydro-1,4-thiazine-1,1-dioxide]; amphotericin B [(1R, 3S, 5R, 6R, 9R, 11R, 15S, 16R, 17R, 18S, 19E, 21E, 23E, 25E, 27E, 29E, 31E, 33R, 35S, 36R, 37S)-33-[(3-Amino-3,6-dideoxy-beta-D-manno-pyranosyl)oxy]-1, 3, 5, 6, 9, 11, 17, 37-octahydroxy-15, 16,18-trimethyl-13-oxo-14,39-dioxabicyclo[33.3.1] nonatriaconta-19,21,23,25,27,29,31-heptaene-36-carboxylic acid]; liposomal amphotericin B and/or paromomycin [O-2-amino-2-deoxy-alpha-D-glucopyranosyl-(1-4)-O-[O-2,6-diamino-2,6-dideoxy-beta-L-idopyranosyl-(1-3)-beta-D-ribofuranosyl-(1-5)]-2-deoxy-D-streptamine]" and the alkyl phospholipid derivative is selected from the group consisting of "miltefosine (hexadecylphosphocholine), perifosine (octadecyl-1,1-dimethyl-piperidino-4-yl-phosphate) and/or erucylphosphocholine [(13Z)-docosenyl-phosphocholine]". More preferably such diseases and/or pathophysiological conditions are selected from the group consisting of "trypanosomiasis, American trypanosomiasis, chagas disease, leishmaniasis, cutaneous leishmaniasis, mucocutaneous leishmaniasis, visceral leishmaniasis, malaria and/or malaria tropica as well as their different forms and subforms". Even more preferably, such medicament comprises miltefosine and is applied before and/or during and/or after treatment with amphotericin B, liposomal amphotericin B and/or paromomycin for the treatment of leishmaniasis, cutaneous leishmaniasis, mucocutaneous leishmaniasis and/or visceral leishmaniasis as well as their different forms and subforms. Even more preferably, such medicament comprises miltefosine and is applied before and/or during and/or after treatment with benznidazole and/or nifurtimox for the treatment of trypanosomiasis, American trypanosomiasis and/or chagas disease as well as their different forms and subforms.

In a preferred embodiment, the compounds of the invention, and in particular alkyl phospholipid derivative **compounds 1 to 20** and **22**, are used for the treatment and/or prophylaxis of the aforementioned diseases and/or pathophysiological conditions in the form of a pharmaceutical kit, where such pharmaceutical kit comprises at least one additional pharmacologically active substance, where such additional pharmacologically active substance is a substance as described herein, and where the alkyl phospholipid derivative and the additional pharmacologically active substance are applied as a pharmaceutical kit.

The compounds of the invention, and in particular alkyl phospholipid derivative **compounds 1 to 20** and **22**, are surprisingly characterized by their improved action and/or efficacy against the various fungi, protozoa, bacteria and/or viruses as well as in the treatment of respective diseases and/or pathophysiological conditions thereof. Due to their surprisingly strong action and/or efficacy, the compounds of the invention, and in particular alkyl phospholipid derivative **compounds 1 to 20** and **22**, may be advantageously administered at lower doses compared to other known but less potent APL or other relevant therapeutic agents while still achieving equivalent or even superior desired biological effects. In addition, such a dose reduction may translate into less or even no medicinal adverse effects.

Furthermore, the compounds of the invention, and in particular alkyl phospholipid derivative **compounds 1 to 20** and **22**, are surprisingly less cytotoxic than known APL or other relevant therapeutic agents while being at least of equivalent if not superior potency. Therefore, use of the compounds of the invention, and in particular alkyl phospholipid derivative **compounds 1 to 20** and **22**, in the treatment of the herein mentioned diseases and/or pathophysiological conditions - even at unreduced doses - may lead to less or even no medicinal adverse effects.

Moreover, combined use of the compounds of the invention, and in particular alkyl phospholipid derivative **compounds 1 to 20** and **22**, but also miltefosine, perifosine and/or erucylphosphocholine with known standard therapeutic agents (e.g. benznidazole, nifurtimox, amphotericin B, liposomal amphotericin B and/or paromomycin) in the treatment of herein mentioned diseases and/or pathophysiological conditions is surprisingly characterized by a superior efficacy compared to the (standard) treatment with the single therapeutic agents alone.

Besides, such combination treatment surprisingly allows a dose reduction of the additional therapeutic agents (e.g. benznidazole, nifurtimox, amphotericin B, liposomal amphotericin B and/or paromomycin) applied compared to their single use while being of equivalent if not superior efficacy. In addition, such a dose reduction may translate into less or even no medicinal adverse effects.

Furthermore, such combination treatment surprisingly allows a significant time reduction, i.e. shorter courses of treatment, which is advantageous for both patients and healthcare systems.

The alkyl phospholipid derivative compounds disclosed herein and/or where appropriate additional pharmacologically active substances can be administered in a known manner. The route of administration may thereby be any route which effectively transports the active compound to the appropriate or desired site of action, for example orally or non-orally, in particular topically, transdermally, pulmonary, rectally, intravaginally, nasally or parenteral or by implantation. Oral administration is preferred.

The alkyl phospholipid derivative compounds disclosed herein and/or where appropriate additional pharmacologically active substances are converted into a form which can be administered and are mixed where appropriate with pharmaceutically acceptable carriers or diluents. Suitable excipients and carriers are described for example in Ullman's Encyclopedia of Technical Chemistry, Vol. 4, (1953), 1-39; Journal of Pharmaceutical Sciences, Vol. 52 (1963), 918 et seq.; H. v. Czetsch-Lindenwald, "Hilfsstoffe für Pharmazie and angrenzende Gebiete"; Pharm. Ind. 2, 1961, 72 et seq.; Dr. H.P. Fiedler, "Lexikon der Hilfsstoffe für Pharmazie, Kosmetik and angrenzende Gebiete", Cantor KG, Aulendorf in WOrttemberg, 1971.

Oral administration can take place for example in solid form as tablet, capsule, gel capsule, coated tablet, granulation or powder, but also in the form of a drinkable solution. The alkyl phospholipid derivative compounds disclosed herein can for oral administration be combined with known and ordinarily used, physiologically tolerated excipients and carriers such as, for example, gum arabic, talc, starch, sugars such as, for example, mannitol, methylcellulose, lactose, gelatin, surface-active agents, magnesium stearate, cyclodextrins, aqueous or nonaqueous carriers, diluents, dispersants, emulsifiers, lubricants, preservatives and flavorings (e.g. essential oils). The alkyl phospholipid derivative compounds disclosed herein can also be dispersed in a microparticulate, e.g. nanoparticulate, composition.

Non-oral administration can take place for example by intravenous, subcutaneous, intramuscular injection of sterile aqueous or oily solutions, suspensions or emulsions, by means of implants or by ointments, creams or suppositories. Administration as sustained release form is also possible where appropriate. Implants may comprise inert materials, e.g. biodegradable polymers or synthetic silicones such as, for example, silicone rubber. Intravaginal administration is possible for example by means of vaginal rings. Intrauterine administration is possible for example by means of diaphragms or other suitable intrauterine devices. Transdermal administration is additionally provided, in particular by means of a formulation suitable for this purpose and/or suitable means such as, for example, patches.

The dosage may vary within a wide range depending on type and/or severity of the disease and/or pathophysiological condition, the mode of administration, the age, gender, bodyweight and sensitivity of the subject to be treated. It is within the ability of a skilled worker to determine a "pharmacologically effective amount" of an alkyl phospholipid derivative compound as disclosed herein and/or additional pharmacologically active substance. Administration can take place in a single dose or a plurality of separate dosages.

A suitable unit dose is, for example, from 0.001 mg to 100 mg of the active ingredient, i.e. at least one alkyl phospholipid derivative compounds as disclosed herein and, where appropriate, at least one additional pharmacologically active substance, per kg of a patient's bodyweight.

### Chemical synthesis

Synthesis of alkyl phospholipid derivative compounds as disclosed herein is a procedure well described in the prior art and known to the skilled artisan due to his expert knowledge. In this context reference is expressively made to the following patent literature as well as the literature cited therein: EP 0 108 565 A2; WO 87/03478; US 5,980,915; US 6,254,879; US 6,506,393; US 6,172,050; US 6,479,472; US 5,449,798; US 5,958,906.

In the following, further details of syntheses of specific head and tail groups are given. For the avoidance of doubt, it is explicitly stated that the different R residues named in the chemical synthesis section do not correspond to those defined above for formula I and specific subsets. Hence, they may differ or have the same meaning.

### A)

Some nitrogen containing head groups were synthesized by alkylation of an amine (see table 1 and general procedure 1).

**Table 1**

| **Compound** | **Nitrogen Containing Head Group** |
|---|---|
| **#3** | 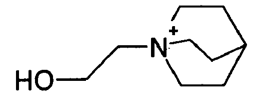 |
| **#19** | 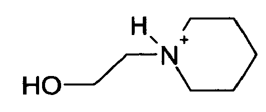 |

### General procedure 1:

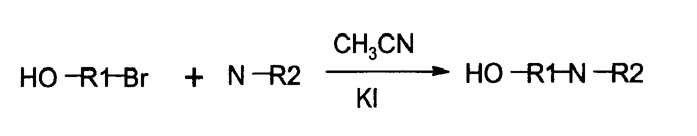

where R1 represents a choline derivative and R2 represent the herein disclosed substituted and/or unsubstituted alkyl radicals.

0.3 mol amine, 150 ml CH₃CN and catalytical amounts of Kl are placed in a reaction vessel. The alkoxy bromide is dropped to this mixture at room temperature, stirred for 15 min and then heated to reflux for 2d. After concentration in vacuo, the residue is crystallized from acetone. Yields vary from 10 to 50%.

When appropriate, other amine alkylating agents (e.g. benzyl bromide or phenetyl bromide) may also be used.

### B)

Other nitrogen containing head groups were synthesized by methylation (see table 2 and general procedure 2).

**Table 2**

| **Compound** | **Methylated Nitrogen Containing Head Group** |
|---|---|
| **#2, #20** | 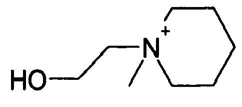 |
| **#1** | 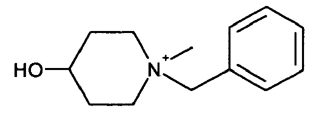 |
| **#4** | 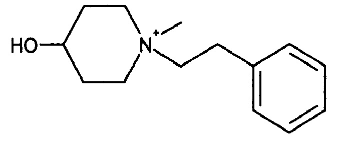 |
| **#6, #7, #10, #12, #13, #15, #17** | 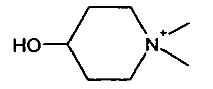 |
| **#8, #11, #14, #16** | 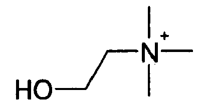 |
| **#9** | 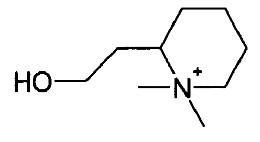 |

### General procedure 2:

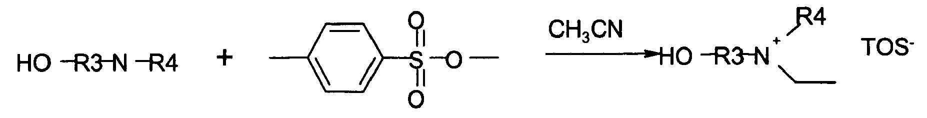

where R3 represents a choline derivative and R4 represent the herein disclosed substituted and/or unsubstituted alkyl radicals.

0,5 mol of the choline derivative and 125 ml CH₃CN are placed in a reaction vessel. 0,5 mol p-toluene sulfonic acid methyl ester in 125 ml CH₃CN are added dropwise while keeping reaction temperatures below 10°C. After stirring for 30 min at room temperatur, the mixture is heated to reflux for another 30 min and cooled to room temperatur. The resulting solid ("tosylate") is isolated (if applicable under inert atmosphere), crystallized from 125 ml isopropanol and dryed over P₂O₅ in vacuo. Yields vary from 40 to 60%.

Alternatively, methyl halogenids may also be used for methylation.

### C)

Other nitrogen containing head groups were synthesized by ethylation (see table 3 and general procedure 3).

**Table 3**

| **Compound** | **Nitrogen Containing Head Group** |
|---|---|
| **#5** | 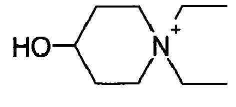 |
| **#18** | 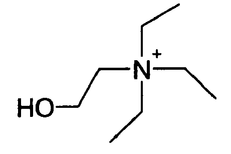 |

### General procedure 3:

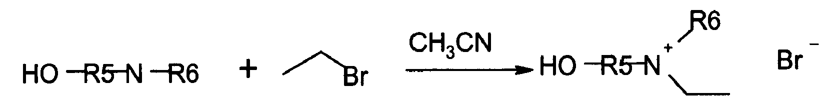

where R5 represents a choline derivative and R6 represents the herein disclosed substituted and/or unsubstituted alkyl radicals.

0,5 mol of the choline derivative and 125 ml CH₃CN are placed in a reaction vessel. 0,5 mol ethyl bromide in 125 ml CH₃CN are added dropwise while keeping reaction temperatures below 10°C. After stirring for 30 min at room temperatur, the mixture is heated to reflux for another 30 min and cooled to room temperatur. The resulting solid ("bromide") is isolated (if applicable under inert atmosphere), recrystallized from 125 ml isopropanol and dryed over P₂O₅ in vacuo. Yields vary from 40 to 60%.

Alternatively, ethyl iodine may also be used.

### D)

The various lipophilic tail groups, which are attached to the phosphate moiety, may be used as exemplified in the following ways (see table 4).

**Table 4**

| **Compound** | **Lipophilic Tail Group** |
|---|---|
| **#1,#2, #3, #4, #5, #9,** | Saturated alkyl chains: C16, C18 |
| **#16, #17, #18, #1**9 | Unsaturated alkylchains: C20, C22 |
| **#6, #12, #13, #15** | 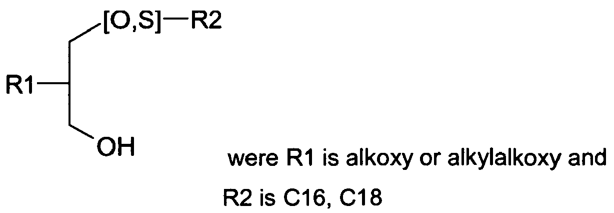 |
| **#14** | 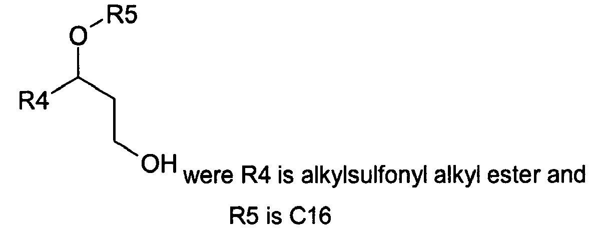 |
| **#7, #10, #11** | 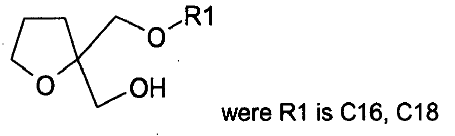 |
| **#8, #20** | Alkoxy-alkoxy chains |

### E)

Alkyl phospholipid derivative compounds 1-20 were synthesized according to the below mentioned general procedure 4, where "tosylate" denotes the nitrogen containing head group and "alcohol" denotes the lipophilic tail group.

### General procedure 4:

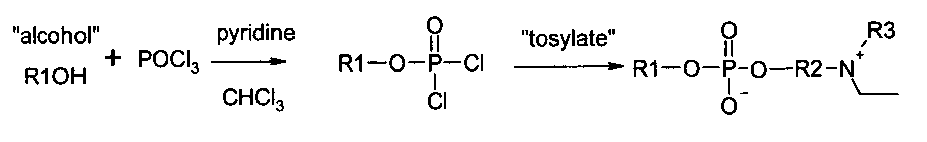

In a reaction vessel, 0.1 mol POCl₃ are placed in 50 ml Chloroform and cooled in an ice bath. A solution of 0.9 mol "alcohol" and 32 ml pyridine in 100ml CH₂Cl₂ is dropped to the POCl₃ solution while keeping the temperature between 5-12° C. After stirring for 30min at room temperature, 0.12 mol "tosylate" are added, cooled to 10° C and 40 ml pyridine is dropped to this solution. The mixture is stirred for 2.5 h at room temperature. 15 ml water are added (T< 20° C) and stirring is continued for another 30 min. The mixture is washed with 200 ml H₂O : MeOH (1:1 v/v), 200 ml 3% HCl : MeOH (1:1 v/v) and 200 ml H2O : MeOH (1:1 v/v). The organic phase is concentrated in vacuo (isopropanol is added in order to diminish foaming). The crude product is recrystallized from 200 ml methyl ethyl ketone. The resulting solid is heated in 150 ml EtOH, filtrated, cooled for 4 h to 5-7° C and again filtrated. 85 g Amberlite MB3 are added to the filtrate and stirred for 3 h at room temperature. After filtrating, the clear solution is concentrated in vacuo and recrystallized from 150ml methyl ethyl ketone. If applicable, the product is purified by column chromatography (CH₂Cl₂ / MeOH / NH₃ (25%) 80 : 25 : 5). Yields vary from 25 to 50%.

The contents of all cited references and patents are hereby incorporated by reference. The invention is explained in more detail by means of the following examples without, however, being restricted thereto.

### Examples

### I) Synthesis of alkyl phospholipid derivate compounds

### Example 1: Compound 1

### Phosphoric acid 1-benzyl-1-methyl-piperidin-4-yl ester hexadecyl ester

3.1g (6%) of compound 1 was obtained starting from hexadecan-1-ol and 1-benzylpiperidine-4-ol according to general procedure 4 after methylating 1-benzyl-piperidine-4-ol according to general procedure 2.

¹H-NMR (600MHz, CDCl₃-d1, 300K): δ = 7.59 (2H, d), 7.47-7.40 (3H, m), 4.78 (2H, broad-s), 4.55 (1H, broad-s), 3.86-3.77 (4H, m), 3.56 (2H, d), 3.13 (3H, s), 2.27 (2H, d), 2.13 (2H, t), 1.57 (2H, quintet), 1.31-1.18 (26H, m), 0.88 (3H, t) ppm

ESI-MS: found: m/z 510.4 [M+H], calculated: 510.7 g/mol

### Example 2: Compound 2

### Phosphoric acid hexadecyl ester 2-(1-methyl-piperidin-1-yl)-ethyl ester

5.8g (13%) of compound 2 was obtained starting from from hexadecan-1-ol and 2-piperidine-1-yl-ethanol according to general procedure 4 after methylating 2-piperidine-1-yl-ethanol according to general procedure 2.

¹H-NMR (600MHz, CDCl₃-d1, 300K): δ = 4.33 (2H, broad-s), 3.89-3.81 (4H, m), 3.70 (2H, m), 3.57-3.53 (2H, m), 3.37 (3H, s), 1.93-1.86 (4H, m), 1.74-1.68 (2H, m), 1.59 (2H, quintet), 1.35-1.19 (26H, m), 0.88 (3H, s) ppm

ESI-MS: found: m/z 448.3 [M+H], calculated: 448.6 g/mol

### Example 3: Compound 3

### Phosphoric acid 2-(1-aza-bicyclo[2.2.2]oct-1-yl)-ethyl ester hexadecyl ester

2,0g (5%) of compound 3 was obtained starting from starting from hexadecan-1-ol and 1-(2-hydroxy-ethyl)-chinuclidine according to general procedure 4 after alkylating chinuclidine according to general procedure 1.

¹H-NMR (600MHz, CDCl₃-d1, 300K): δ = 4.29 (2H, broad-s), 3.84 (2H, q), 3.73 (8H, m), 2.17 (1H, m), 1.60 (2H, quintet), 1.34-1.22 (26H, m), 0.88 (3H, t) ppm

ESI-MS: found: m/z 460.5 [M+H], calculated: 460.7 g/mol

### Example 4: Compound 4

### Phosphoric acid hexadecyl ester 1-methyl-1-phenethyl-piperidin-4-yl ester

6.3g (12%) of compound 4 was obtained starting from hexadecan-1-ol and 1-phenethyl-piperidine-4-ol according to general procedure 4 after methylating 1-phenethyl-piperidine-4-ol according to general procedure 2.

¹H-NMR (600MHz, CDCl₃-d1, 300K): δ = 7.34-7.22 (5H, m), 4.49 (1H, broad-s), 3.80 (2H, q), 3.77-3.58 (6H, m), 3.29 (3H, s), 3.10 (2H, dd), 2.24 (2H, m), 2.10 (2H, m), 1.59 (2H, quintet), 1.31-1.19 (26H, m), 0.88 (3H, t) ppm

ESI-MS: found: m/z 524.4 [M+H], calculated: 524.8 g/mol

### Example 5: Compound 5

### Phosphoric acid 1,1-diethyl-piperidin-4-yl ester octadecyl ester

6.9g (14%) of compound 5 was obtained starting from octadecan-1-ol and 1-ethylpiperidin-4-ol according to general procedure 4 after alkylating 1-ethyl-piperidin-4-ol according to general procedure 3.

¹H-NMR (600MHz, CDCl₃-d1, 300K): δ = 4.50 (1H, broad-s), 3.81 (2H, q), 3.71 (2H, m), 3.62 (2H, m), 3.51 (2H, q), 3.46 (2H, q), 2.25-2.09 (4H, m), 1.58 (2H, quintet), 1.38-1.21 (36H, m), 0.88 (3H, t) ppm

ESI-MS: found: m/z 490.6 [M+H]+ / calculated: 490.8 g/mol

### Example 6: Compound 8

### Phosphoric acid 3-hexadecyloxy-propyl ester 2-trimethyl-ammonium-ethyl ester

9.3g (20%) of compound 8 was obtained starting from 3-hexadecyloxy-propan-1-ol and 2-dimethylamino-ethanol according to general procedure 4 after methylating 2-dimethylamino-ethanol according to general procedure 2.

¹H-NMR (600MHz, CDCl₃-d1, 300K): δ = 4.32 (2H, broad-s), 3.93 (2H, q), 3.83 (2H,

bs), 3.50 (2H, t), 3.42 (9H, s), 3.39 (2H, t), 1.89 (2H, quintet), 1.54 (2H, quintet), 1.34-1.22 (26H, m), 0.88 (3H, t) ppm

ESI-MS: found: m/z 466.5 [M+H], calculated: 466.7 g/mol

Data on further examplified alkyl phospholipid derivative compounds are compiled in **Table 5** below:

**Table 5: Examplified alkyl phospholipid derivative compounds with synthesis schemes and MS data:**

| **No.** | **Synthesis schemes** | **[M+H] (calculated)** | **ESI-MS found (M+H)⁺** |
|---|---|---|---|
| **6** | 2,4 | 552.8 | 542.4 |
| **7** | 2,4 | 548.8 | 548.5 |
| **9** | 2,4 | 476.7 | 476.5 |
| **10** | 2,4 | 576.8 | 576.4 |
| **11** | 2,4 | 522.7 | 522.5 |
| **12** | 2,4 | 522.7 | 522.7 |
| **13** | 2,4 | 536.7 | 536.5 |
| **14** | 2,4 | 558.8 | 558.4 |
| **15** | 2,4 | 550.8 | 550.5 |
| **16** | 2,4 | 462.7 | 462.4 |
| **17** | 2,4 | 516.8 | 516.5 |
| **18** | 3,4 | 422.6 | 422.3 |
| **19** | 4 | 460.7 | 460.5 |
| **20** | 2,4 | 452.6 | 452.4 |
| **22** | | 496.5 | 497.4 |
| **27** | | 538.8 | 538.8 |
| **30** | | 408.6 | 408.4 |
| **33** | | 436.6 | 436.4 |
| **35** | | 448.6 | 448.4 |
| **40** | | 464.7 | 464.3 |
| **41** | | 554.9 | 554.6 |
| **42** | | 406.6 | 406.2 |
| **43** | | 434.6 | 434.4 |
| **47** | | 492.8 | 492.5 |
| **49** | | 462.9 | 462.3 |
| **51** | | 450.7 | 450.4 |
| **55** | | 432.6 | 432.3 |
| **56** | | 450.7 | 450.4 |
| **57** | | 394.5 | 394.6 |
| **60** | | 436.6 | 436.3 |
| **66** | | 450.6 | 450.4 |
| **67** | | 445.6 | 445.3 |
| **68** | | 594.4 | 594.2 |
| **70** | | 436.6 | 436.4 |
| **75** | | 434.6 | 434.5 |
| **76** | | 446.6 | 446.3 |
| **80** | | 490.7 | 490.5 |
| **81** | | 466.7 | 466.5 |
| **93** | | 490.7 | 490.6 |
| **95** | | 459.6 | 459.5 |
| **98** | | 462.7 | 462.3 |
| **99** | | 492.7 | 492.3 |
| **103** | | 474.7 | 474.5 |
| **106** | | 490.7 | 490.6 |
| **107** | | 470.6 | 470.4 |
| **108** | | 464.7 | 464.3 |
| **109** | | 488.7 | 488.4 |
| **112** | | 448.6 | 448.3 |
| **113** | | 476.5 | 476.5 |
| **115** | | 504.7 | 504.4 |
| **116** | | 462.7 | 462.4 |
| **117** | | 460.7 | 460.4 |
| **124** | | 476.7 | 476.4 |
| **133** | | 546.8 | 546.4 |
| **134** | | 532.8 | 532.5 |
| **136** | | 450.7 | 450.4 |
| **138** | | 448.7 | 448.3 |
| **139** | | 504.8 | 504.5 |
| **146** | | 476.7 | 476.5 |
| **154** | | 491.7 | 491.5 |
| **155** | | 478.3 | 478.3 |
| **169** | | 460.7 | 460.5 |
| **171** | | 494.7 | 494.3 |
| **176** | | 488.7 | 488.5 |
| **179** | | 564.8 | 564.3 |
| **192** | | 536.7 | 536.5 |
| **195** | | 538.8 | 538.5 |
| **197** | | 538.8 | 538.5 |
| **204** | | 550.1 | 550.5 |
| **211** | | 564.8 | 564.6 |
| **213** | | 550.8 | 550.4 |
| **215** | | 504.7 | 504.5 |
| **233** | | 614.9 | 614.4 |
| **236** | | 849.1 | 849.4 |
| **243** | | 594.9 | 594.4 |
| **257** | | 875.1 | 875.5 |
| **275** | | 532.8 | 532.5 |
| **296** | | 488.7 | 488.4 |
| **298** | | 576.8 | 576.3 |
| **305** | | 496.7 | 496.4 |
| **309** | | 578.8 | 578.5 |
| **314** | | 436.8 | 436.5 |

### II) Assays for assessing cytotoxicity against mammalian cell lines

Cytotoxicity of selected alkyl phospholipid derivative compounds against different mammalian cell lines was assessed as follows.

Human tumor cell lines KB/HeLa (ATCC CCL17, human cervix carcinoma), PC3 (ATCC CRL1435, human prostate carcinoma) and RKOp21 (human colon adenocarcinoma) cells were used in an automated XTT screening assay for the assessment of cytotoxicity.

The XTT assay quantifies cellular metabolic activity which correlates with cell viability and cell number. Test compounds (selected compounds of the invention and known substances as controls) are dissolved in culture medium at 600µM and added to the tumor cells in 10 different concentrations in a semi-logarithmic fashion starting from 100 µM as highest concentration.

48h later cellular metabolic activity of cells treated with test compounds is quantified by measurement of the absorbance at 490nm (conversion of XTT dye) in comparison to untreated control cells (100% viability).

KB/HeLa and PC3 cells were cultivated in RPMI medium supplemented with 10% heat inactivated fetal calf serum, 2 mM glutamine, 100 U/ml penicillin, and 100 µg/ml streptomycin. RKOp21 cells were grown in DMEM supplemented with 10% heat inactivated fetal calf serum, 2 mM glutamine, 100 U/ml penicillin, and 100 µg/ml streptomycin. All cells were cultured at 37°C in 5% CO₂ humidified air.

On the first day of the experiment cells were harvested from exponential phase cultures by trypsination, counted and plated into 96 well flat-bottom micro titer plates depending on the cell line as listed below:

| Cell Line | Number / Well | Volume / Well |
|---|---|---|
| KB/HeLa | 2500 | 125 |
| PC3 | 6000 | 125 |
| RKOp21 | 6000 | 125 |

After a 24h recovery to allow the cells to resume exponential growth, the test compounds were diluted immediately prior to use and transferred in 25µl volume to the wells.

Following 2 days of continuous drug exposure 50µl of freshly prepared XTT-PMS solution (2% 8.383mg/ml PMS in PBS + 98% 1 mg/ml XTT in RPMI 1640 without phenol red) was added to the wells. To measure the proportion of living cells, the cells were incubated for 3 h with XTT-PMS reagent at 37°C, 5% CO2, humidified atmosphere allowing to form the formazan salt. The amount of soluble formazan salt produced by cellular reduction of XTT is quantified by measuring the absorbance at 490nm using the Biomek^{®} 2000 ELISA plate reader.

%-inhibition (cytotoxicity) of each test compound was calculated in relation to untreated control cells. Inhibition curves were generated and IC₅₀ values calculated by using GraphPad Prism.

In the following **table 6** cytotoxicity results (IC₅₀ values) obtained for selected alkyl phospholipid derivative compounds (**compounds 20, 19, 17, 2**) are presented in comparison to examples of the prior art (perifosine, miltefosine).

**Table 6: Cytotoxicity assay results for mammalian RKOp21, KB/HELA and PC3 cell lines (IC₅₀ values for a number of selected exemplary compounds)**

| **Compound** | **RKOp21** | **PC3** | **KB/HELA** |
|---|---|---|---|
| | [IC₅₀ (µM)] | [IC₅₀ (µM)] | [IC₅₀ (µM)] |
| **20** | >100 | >100 | >100 |
| **19** | >100 | >100 | >100 |
| **17** | 32,47 | 18,28 | 16,59 |
| **2** | 40,93 | 19,64 | 5,02 |
| **perifosine** | 3,31 | 3,22 | 2,37 |
| **miltefosine** | 18,04 | 9,39 | 8,66 |

The result presented in **table 6** clearly demonstrate the favorable lower cytotoxicity of the selected compounds of the invention compared to prior art compounds perifosine and miltefosine.

### III) Anti-fungal activity assay

Anti-fungal activity of selected alkyl phospholipid derivative compounds against different fungi was assessed by means of anti-fungal susceptibility tests as follows.

The antifungal activities of the test compounds (compounds of the invention and known prior art compounds) were measured according to standard microdilution methods (Cryptococcus/Aspergillus: Ghannoum et al., Journal of Clinical Microbiology 1992, 30 (11): 2881-2886; Candida: Reference method for broth dilution susceptibility testing of yeast: proposed standard, NCCLS document M27-P, National Committee for Clinical Laboratory Standards 1992, Villanova, Pa).

A serial dilution series starting from 64 µg/mL test compound per well down to 0.125 µg/mL test compound per well was applied for each test compound on each fungus. The MIC of each compound was defined as the concentration which produces no visible growth after 48 hours (Candida) and 72 hours (Cryptococcus, Aspergillus) of culture at 35° C.

The following fungi were assessed:
**A)** *Candida albicans* (ATCC 10231)
**B)** *Candida albicans* (ATCC 90028)
**C)** *Candida albicans* (strain TS3, Shrikantha et al., Journal of Bacteriology 2000, 182(6):1580-1591)
**D)** *Candida parapsilosis* (ATCC 22019)
**E)** *Cryptocoocus neoformans* (ATCC 90112)
**F)** *Cryptocoocus neoformans* (strain H99; Ganendren et al., Antimicrobial Agents and Chemotherapy, 2004, 48(5):1561-1569)
**G)** *Cryptocoocus gattii* (ATCC 32608)
**H)** *Aspergillus fumigatus* (ATCC 204305)
**I)** *Aspergillus fumigatus* (strain 322-384)

**Table 7** displays the results (MIC values in µg/mL) of the anti-fungal activity assay obtained for selected alkyl phospholipid derivative compounds (**compounds 1, 2, 3, 5, 8**) in comparison to prior art example erucylphosphocholine (ErPC).

**Table 7: Susceptibilities of different fungal species (MICs in µg/mL) to selected alkyl phospholipid derivative compounds (compounds no. 1, 2, 3, 5, 8 and 22)**

| **fungus** | **no.1** | **no. 2** | **no. 3** | **no. 5** | **no. 8** | **no. 22** | **ErPC** |
|---|---|---|---|---|---|---|---|
| **A** | 2 | 1 | 1 | 2 | 0.75 | 0.5 | > 64 |
| **B** | 1 | 1 | 1 | 2 | 0.75 | 1 | > 64 |
| **C** | 1.5 | 1.5 | 2 | 2 | 1 | 1 | > 64 |
| **D** | 3 | 2 | 2 | 3 | 0.75 | 1 | > 64 |
| **E** | 1.5 | 1.5 | 1.5 | 1.5 | 2 | --- | 4 |
| **F** | 2 | 1 | 1 | 2 | 1.5 | --- | 3 |
| **G** | 1 | 0.75 | 0.75 | 0.75 | 0.75 | --- | 1.5 |
| **H** | 2 | 2 | 3 | 6 | 2 | 4 | >64 |
| **I** | 2 | 2 | 2 | 8 | 2 | 2 | >64 |

The result presented in **table 7** show the strong antifungal activity of the selected compounds of the invention compared to prior art compound erucylphosphocholine (ErPC).

### IV) In vitro drug activity assay of selected alkyl phospholipid derivatives against different protozoa

In vitro activity of selected alkyl phospholipid derivative compounds against different protozoa was assessed as follows.

Murine (CD1) peritoneal macrophages were harvested 24 hours after starch induction and dispensed into 96-well plates at a concentration of 4 x 10⁵ mL. After 24 hours, the cells were infected with Trypanosoma cruzi Tulahuan LAC-Z amastigotes (grown and cultured according to Buckner et al., Infection and Immunity 1999, 67(1): 403-409). 24 hours later, the infected cells were exposed to the test compounds (compounds of the invention as well as known prior art compounds) for 3 days and 50 µL of 500 µM CPRG-1% Nonidet P-40 was added to each well. The plates were read after 2 to 5 hours at 570 nm (Buckner et al., Antimicrobial Agents and Chemotherapy 1996, 40(11): 2592-2597). ED₅₀ values were calculated with Msxlfit (IDBS).

Trypanosoma brucei rhodesiense STIB900 bloodstream form trypomastigotes were maintained in HMI-18 medium (Hirumi H, Hirumi K, J Parasitol. 1989, 75(6):985-989) with 15% heat-inactivated fetal calf serum at 37°C, 5% CO2, humidified atmosphere. Trypomastigotes were washed and resuspended in fresh medium at a concentration of 2 x 10⁵ mL. Test compounds were given to the trypomastigotes and plates were incubated for 72 hours at 37°C, 5% CO2, humidified atmosphere. At 72 hours the plates were assessed microscopically before Alamar Blue was added (Raz et al., Acta Trop. 1997, 68: 139-147). Plates were read after 5 to 6 hours at EX/EM 530/585 nm with a filter cutoff at 550 nm. ED₅₀ values were calculated with Msxlfit (IDBS).

Plasmodium falciparum strain K1 and strain 3D7 parasites were cultured according to Trager W and Jensen JB (Science 1976, 193: 673-675). In vitro drug susceptibility assays with the test compounds were carried out according to Korsinczky M et al. (Antimicrobial agents and chemotherapy 2000, 44(8): 2100-2108) and Fivelman QL et al. (Antimicrobial agents and chemotherapy 2004, 48(11): 4097-4102) using a ³[H]-hypoxanthine radioisotope method. ED₅₀ values were calculated with Msxlfit (IDBS).

The following protozoa were assessed:
**A)** Trypanosoma cruzi (Tulahuan-LACZ amstigotes in PEM, Lorente SO et al., Antimicrobial agents and chemotherapy 2004, 48(8): 2937-2950; Buckner et al., Infection and Immunity 1999, 67(1): 403-409; Buckner et al., Antimicrobial Agents and Chemotherapy 1996, 40(11): 2592-2597)
**B)** Trypanosoma brucei rhodesiense (strain STIB900, Lorente SO et al., Antimicrobial agents and chemotherapy 2004, 48(8): 2937-2950; Habtemariam S, BMC Pharmacology 2003, 3:6)
**C)** Plasmodium falciparum (strain K1, Korsinczky M et al., Antimicrobial agents and chemotherapy 2000, 44(8): 2100-2108; Thaitong S and Beale GH, Trans. R. Soc. Trop. Med. Hyg. 1981, 75: 271-273; Trager W and Jensen JB, Science 1976, 193: 673-675)
**D)** Plasmodium falciparum (strain 3D7, Mu et al., PLoS Biology 2005, 3(10): e335)

Following **table 8** displays the results (ED₅₀ values in µg/mL) of the *in vitro* activity assay obtained for selected alkyl phospholipid derivative compounds (**compounds 1, 2, 3, 4, 5**) against different protozoa.

**Table 8: In vitro drug activity assay results for different protozoa (ED₅₀ values for a number of selected exemplary compounds)**

| **compound** | **protozoon** | **ED₅₀** (µg/mL) |
|---|---|---|
| **no. 1** | A | 1,71 |
| **no. 1** | B | 18,80 |
| **no. 1** | C | 6,93 |
| **no. 1** | D | 9,97 |
| **no. 2** | A | 0,50 |
| **no. 2** | B | 17,50 |
| **no. 2** | C | 19,36 |
| **no. 2** | D | 2,21 |
| **no. 3** | A | 0,33 |
| **no. 3** | B | 25,03 |
| **no. 3** | C | 8,03 |
| **no. 3** | D | 2,25 |
| **no. 4** | A | 1, 93 |
| **no. 4** | B | 12,29 |
| **no. 4** | C | 5,44 |
| **no. 4** | D | 15,32 |
| **no. 5** | A | 1,43 |
| **no. 5** | B | 14,20 |
| **no. 5** | C | 21,26 |
| **no. 5** | D | 16,31 |
| **benznidazole** | A | 0,22 |
| **miltefosine** | A | 0,63 |

## Claims

1. Use of an alkyl phospholipid derivative according to formula I 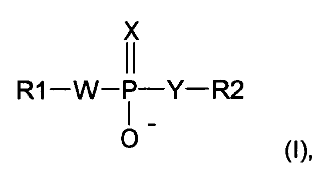 wherein:
W, X, Y independently are selected from the group consisting of: "oxygen atom, sulphur atom";
R1 is "-[(CR3R4)ₘ-Z]ₙ-R5";
R2 is "-(CR6R7)ₚ-R8";
R3 and R4 are independently from each other selected from the group consisting of "hydrogen atom; substituted or unsubstituted C1-C12alkyl, substituted or unsubstituted (C1-C12alkyl)_{q}-A-(C1-C18alkyl)ᵣ, -OH, substituted or unsubstituted -C(O)-(C8-C30alkyl), substituted or unsubstituted -OC(O)-C8-C30alkyl), substituted or unsubstituted -NHCO-(C1-C12alkyl), substituted or unsubstituted -N(C1-C12alkyl)CO-(C1-C12alkyl)";
or optionally R3 and R4 together form a substituted or unsubstituted saturated, partially unsaturated or aromatic heterocyclic ring system of 3, 4, 5, 6, 7 or 8 ring atoms containing at least one heteroatom selected from the group consisting of: "oxygen atom, sulfur atom";
R5 is independently selected from the group consisting of: "substituted or unsubstituted C8-C30alkyl, substituted or unsubstituted -C(O)-(C8-C30alkyl), substituted or unsubstituted steroid moiety;
R6 and R7 are independently from each other selected from the group consisting of "hydrogen atom, -OH, halogen atom, -F, -Cl, -Br, -I, -CN, C1-C6alkyl";
or optionally R6 and R7 together form a substituted or unsubstituted.saturated, partially unsaturated or aromatic ring system of 3, 4, 5, 6 or 7 carbon atoms;
or optionally if p is 1, "-(CR6R7)ₚ-" can also be a substituted or unsubstituted saturated, partially unsaturated or aromatic ring system of 3, 4, 5, 6 or 7 carbon atoms
formed together by R6 and R7;
R8 is selected from the group consisting of: "-VR9R10R11; substituted or unsubstituted heterocycle", where the heterocycle is (i) a 5-, 6- or 7-membered saturated, partially unsaturated or aromatic monocyclic carbon atom ring system with at least one heteroatom selected from the group consisting of: "nitrogen atom, oxygen atom, sulphur atom, arsenic atom", and with the proviso that at least one heteroatom is a quaternary nitrogen atom or a quaternary arsenic atom, or (ii) a 7-, 8-, 9-, 10-, 11- or 12-membered saturated, partially unsaturated or aromatic bicyclic carbon atom ring system with at least one heteroatom selected from the group consisting of: "nitrogen atom, oxygen atom, sulphur atom, arsenic atom", and with the proviso that at least one heteroatom is a quaternary nitrogen atom or a quaternary arsenic atom, or (iii) a tropin moiety, where two or more ring atoms of the heterocycle can be additionally linked via an alkylene-bridge, and where the heterocycle if substituted is substituted with at least one R12, which in case of two or more R12 are independently from each other identical, partly identical or different;
R9, R10, R11, R12 are independently from each other selected from the group consisting of: "hydrogen atom, substituted or unsubstituted C1-C18alkyl, substituted or unsubstituted C3-C8cycloalkyl, substituted or unsubstituted (C1-C12alkyl)ₛ-B-(C1-C12alkyl)ₜ-C-(C1-C12alkyl)ᵤ, substituted or unsubstituted aryl, substituted or unsubstituted heteroaryl, substituted or unsubstituted alkoxy, -OH, halogen, -F, -Cl, -Br, -I, =O, -C(O)O-(C1-C12alkyl), -C(O)O-(C3-C8cycloalkyl), -C(O)O-aryl, -C(O)O-heteroaryl, -C(O)O-heterocyclyl, -C(O)-(C1-C12alkyl), -C(O)-(C3-C8cycloalkyl), -C(O)-aryl, -C(O)-heteroaryl, -C(O)-heterocyclyl", and optionally two substituents R12 can together form a substituted or unsubstituted saturated, partially unsaturated or aromatic ring system of 3, 4, 5, 6 or 7 carbon atoms;
Z is independently selected from the group consisting of "oxygen atom; sulphur atom";
V is independently selected from the group consisting of "nitrogen atom, arsenic atom";
A, B, C are independently from each other selected from the group consisting of
"oxygen atom; sulphur atom; S(O₂)";
m independently is 1, 2 or 3;
n independently is 0, 1, 2, 3, 4, 5, 6, 7, 8, 9 or 10 and preferably is 0, 1, 2, or 3;
p independently is 0, 1, 2, 3, 4, 5 or 6, and preferably is 0, 1, 2 or 3;
q, r, s, t, u independently from each other are 0 or 1;
for the manufacture of a medicament for the treatment or prophylaxis of diseases and/or pathophysiological conditions in mammals that are caused by microorganisms.

2. The use of an alkyl phospholipid derivative according to formula (I) as claimed in claim 1, where
R1 is R5,
n is 0.

3. The use of an alkyl phospholipid derivative according to formula (I) as claimed in claim 1, where
m is 2 or 3,
n is 1 or 2.

4. The use as claimed in any of claims 1 to 3, where the alkyl phospholipid derivative is selected from the group consisting of:
"**Compound 1** 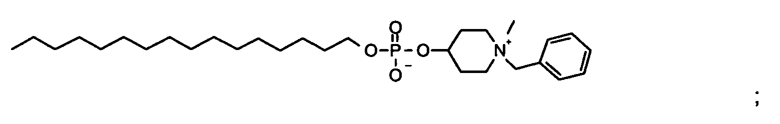
**Compound 2** 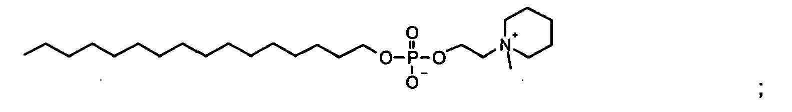
**Compound 3** 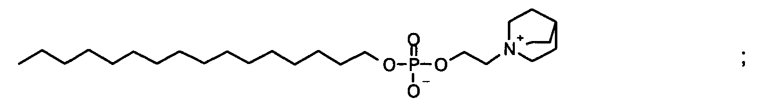
**Compound 4** 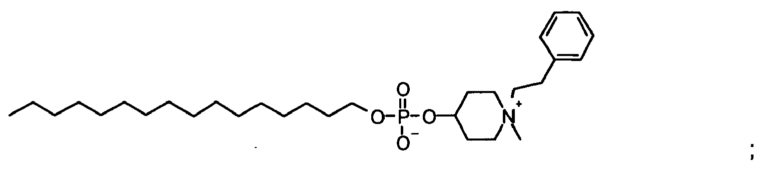
**Compound 5** 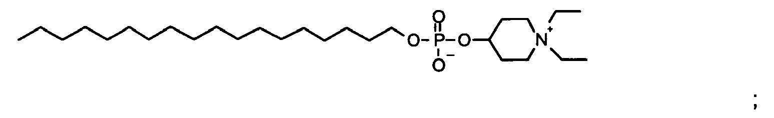
**Compound 6** 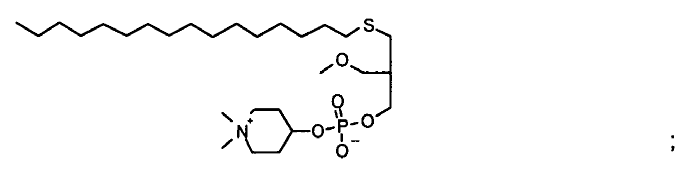
**Compound 7** 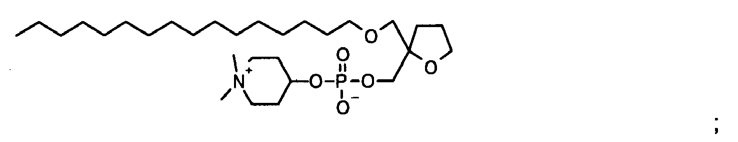
**Compound 8** 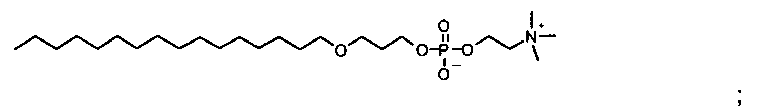
**Compound 9** 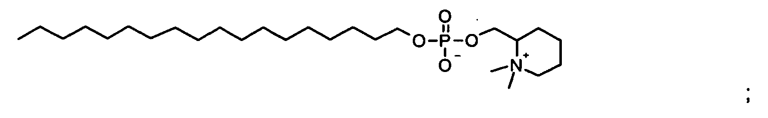
**Compound 10** 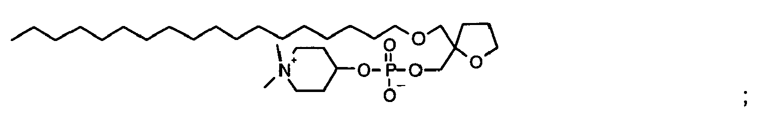
**Compound 11** 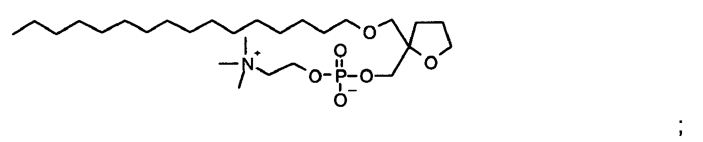
**Compound 12** 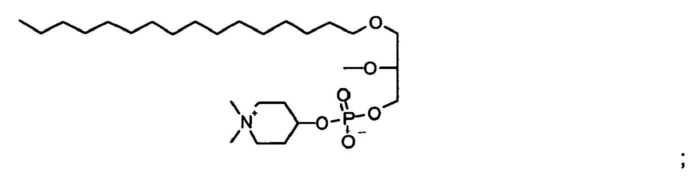
**Compound 13** 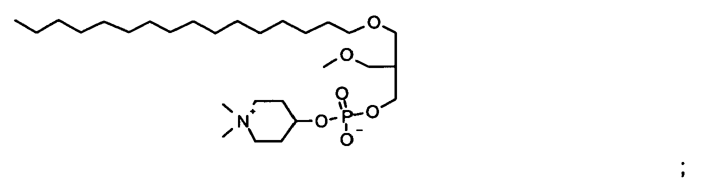
**Compound 14** 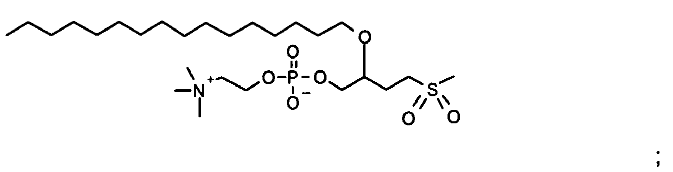
**Compound 15** 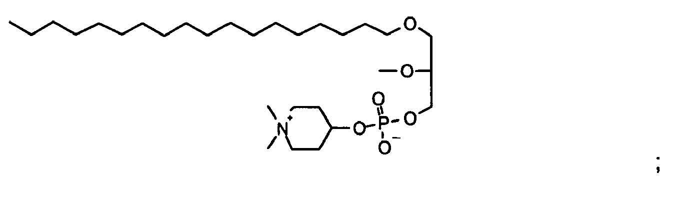
**Compound 16** 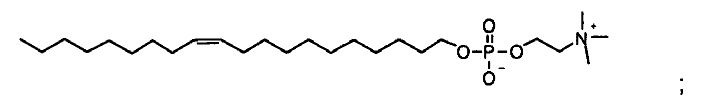
**Compound 17** 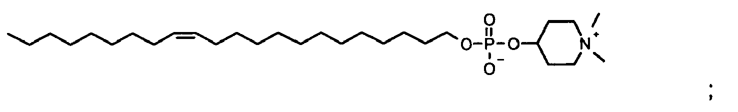
**Compound 18** 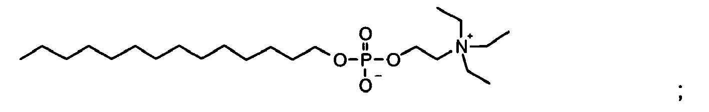
**Compound 19** 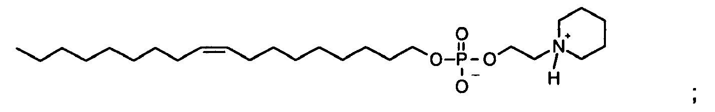
**Compound 20** 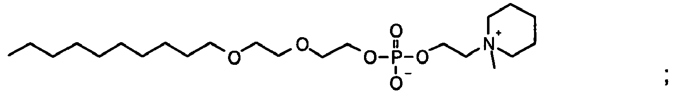
**Compound 22**

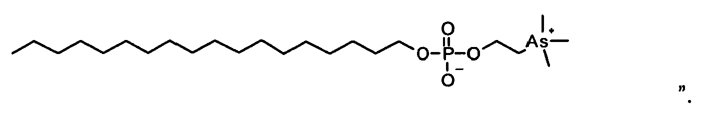

5. The use as claimed in any of claims 1 to 4, where the microorganism is selected from the group consisting of "fungus, protozoon, bacterium and/or virus".

6. The use as claimed in any of claims 1 to 5, where the microorganism is a fungus and selected from the group consisting of "Absidia spp., Acremonium spp., Alternaria spp., Aspergillus spp., Bipolaris spp., Candida spp., Cladophialophora spp., Cladosporium spp., Coccidioides spp., Coniothyrium spp., Cryptococcus spp., Cunninghamella spp., Curvularia spp., Epidermophyton spp., Exophiala spp., Exserohilum spp., Fonsecaea spp., Fusarium spp., Histoplasma spp., Lacazia spp., Lasiodiplodia spp., Leptosphaeria spp., Madurella spp., Microsporum spp., Mucor spp., Mucorales spp., Neotestudina spp., Ochroconis spp., Onychocola spp., Paecilomyces spp., Paracoccidioides spp., Penicillium spp., Phialophora spp., Pseudallesheria spp., Pyrenochaeta spp., Rhizomucor spp., Rhizopus spp., Scedosporium spp., Scopulariopsis spp., Scytalidium spp., Sporothrix spp., Trichophyton spp. and/or Wangiella spp." and preferably is selected from the group consisting of "Absidia spp., Aspergillus spp., Bipolaris spp., Candida spp., Cryptococcus spp., Cunninghamella spp., Exophiala spp., Fusarium spp., Paecilomyces spp., Rhizopus spp. and/or Scedosporium spp.".

7. The use as claimed in claim 6, where the alkyl phospholipid derivative is selected from the group consisting of: "**Compound 1, 2, 3, 4, 5, 6, 7, 8, 9, 10, 11, 12, 13, 14,15,16,17,18, 19, 20** and/or **compound 22**".

8. The use as claimed in any of claims 1 to 5, where the microorganism is a protozoon and selected from the group consisting of "Acanthamoeba spp., Amoeba spp., Babesia spp., Balantidium spp., Cryptosporidium spp., Cyclospora spp., Dientamoeba spp., Echinamoeba spp., Endolimax spp., Entamoeba spp., Enterocytozoon spp., Giardia spp., Hartmanella spp., Isospora spp., Jodamoeba spp., Lamblia spp., Leishmania spp., Microsporidium spp., Naegleria spp., Nosema spp., Paramecium spp., Paramoeba spp., Penumocystis spp., Plasmodium spp., Sarcocystis spp., Tetrahymena spp., Toxoplasma spp., Trichomonas spp. and/or Trypanosoma spp." and preferably is selected from the group consisting of "Leishmania spp., Plasmodium spp., Toxoplasma spp. and/or Trypanosoma spp.".

9. The use as claimed in claim 8, where the alkyl phospholipid derivative is selected from the group consisting of: "**Compound 1, 3, 4, 5, 6, 7, 8, 10, 11, 12, 13, 14, 15, 18, 19** and/or **compound 20**".

10. The use as claimed in any of claims 1 to 5, where the microorganism is a bacterium and selected from the group consisting of "gram-positive bacterium and/or gram-negative bacterium".

11. The use as claimed in claim 10, where the bacterium is selected from the group consisting of "Acinetobacter spp., Actinobacillus spp., Actinomyces spp., Aeromonas spp., Agrobacterium spp., Alcaligenes spp., Anaplasma spp., Aquifex spp., Bacillus spp., Bacteroides spp., Bifidobacterium spp., Bordetella spp., Borrelia spp., Bradyrhizobium spp., Branhamella spp., Brucella spp., Buchnera spp., Burkholderia spp., Campylobacter spp., Capnocytophaga spp., Cardiobacterium spp., Caulobacter spp., Chlamydia spp., Chlamydophila spp., Chlorobium spp., Citrobacter spp., Clostridium spp., Corynebacterium spp., Coxiella spp., Deinococcus spp., Ehrlichia spp., Eikenella spp., Enterobacter spp., Enterococcus spp., Erysipelothrix spp., Escherichia spp., Francisella spp., Fusobacterium spp., Gardnerella spp., Gemella spp., Haemophilus spp., Heliobacter spp., Kingella spp., Kitasatospora spp., Klebsiella spp., Lactobacillus spp., Legionella spp., Leptospira spp., Listeria spp., Mannheimia spp., Mesorhizobium spp., Moraxella spp., Morganella spp., Mycobacterium spp., Mycoplasma spp., Neisseria spp., Neorickettsia spp., Nitrosomonas spp., Nocardia spp., Oceanobacillus spp., Orientia spp., Paracoccus spp., Pasteurella spp., Peptostreptococcus spp., Plesiomonas spp., Porphyromonas spp., Prevotella spp., Propionibacterium spp., Proteus spp., Providencia spp., Pseudomonas spp., Psychobacter spp., Ralstonia spp., Rhodobacter spp., Rhodococcus spp., Rickettsia spp., Salmonella spp., Serratia spp., Shewanella spp., Shigella spp., Spirillum spp., Staphylococcus spp., Stenotrophomonas spp., Streptobacillus spp., Streptococcus spp., Streptomyces spp., Synechococcus spp., Synechocystis spp., Tannerella spp., Thermoanaerobacter spp., Thermotoga spp., Treponema spp., Tropheryma spp., Ureaplasma spp., Veillonella spp., Vibrio spp., Wigglesworthia spp., Wolbachia spp., Xanthomonas spp., Xylella spp., Yersinia spp. and/or Zymomonas spp." and preferably is selected from the group consisting of "Bacteroides spp., Branhamella spp., Chlamydia spp., Escherichia spp., Haemophilus spp., Klebsiella spp., Mycobacterium spp., Mycoplasma spp., Proteus spp., Pseudomonas spp., Serratia spp., Staphylococcus spp. and/or Streptococcus spp.".

12. The use as claimed in any of claims 10 to 11, where the alkyl phospholipid derivative is selected from the group consisting of: "**Compound 1, 2, 3, 4, 5, 6, 7, 8, 9, 10, 11, 12, 13, 14, 15, 16, 17, 18, 19** and/or **compound 20**".

13. The use as claimed in any of claims 1 to 5, where the microorganism is a virus and selected from the group consisting of "DNA virus; dsDNA virus, ssDNA virus; RNA virus; dsRNA virus; (+)ssRNA virus; (-)ssRNA virus; DNA/RNA reverse transcribing virus; ssRNA-RT virus and/or dsDNA-RT virus".

14. The use as claimed in claim 13, where the virus is selected from the group consisting of "adenovirus type 1, 2, 3, 5, 11, 21, adenovirus, alphavirus, arbovirus, arenavirus, borna disease virus, bunyavirus, calicivirus, California encephalitis virus, Colorado tick fever virus, coronavirus cowpox virus, coxsackie type A virus, coxsackie type B virus, coxsackie virus type A-16, A-24, Coxsackie virus type B1, B2, B3, B4, B5, cytomegalovirus (CMV), deltavirus, dengue virus, Ebola virus, echovirus, EEE virus, enterovirus type 7, 70, Epstein-Barr virus (EBV), filovirus, flavivirus, foot and mouth disease virus, FSME virus, hantavirus type Hantaan, Seoul, Dobrava (Belgrade), Puumala. Sin Nombre, Black Creek Canal, Bayou, New York-1, hantavirus, hepadnavirus, hepatitis A virus, hepatitis B virus, hepatitis C virus, hepatitis D virus, hepatitis E virus, hepatitis F virus, hepatitis G virus, herpes simplex virus (HSV), herpes simplex virus type 1 and 2 (HSV-1, HSV-2), herpesvirus, HIV, HIV-1, HIV-2, human papilloma virus (HPV), human T cell leukemia virus, human T-cell lymphotrophic virus type I and II (HTLV-I, -II), influenza virus, influenza virus type A (H5N1) and (H3N2), influenza virus type A, B, C, Japanese encephalitis virus, JC virus, juninvirus, Kaposi's sarcoma-associated virus, LaCross virus, Lassavirus, lentivirus, lymphocytic choriomeningitis virus, machupovirus, Marburg virus, measles virus, Molluscum virus, mumps virus, Norwalk virus, orfvirus, orthomyxovirus, papovavirus, parainfluenza virus type 1, 2, 3, parainfluenza virus, paramyxovirus type 1, 2, 3, 4, paramyxovirus, parvovirus B19, parvovirus, picornavirus, poliovirus, poxvirus, rabies virus, Rabies virus, reovirus, respiratory syncytial virus, rhabdovirus, rhinoviruses, rotavirus, Rubella virus, rubeola virus, rubivirus, SARS virus, Simian virus 40, SLE virus, togavirus, Torque teno virus, vaccinia virus, varicella zoster virus, variola virus, Vicia faba endornavirus, WEE virus, West Nile virus and/or Yellow fever virus".

15. The use as claimed in any of claims 13 to 14, where the alkyl phospholipid derivative is selected from the group consisting of "**Compound 1, 2, 3, 4, 5, 6, 7, 8, 10, 11, 12, 13, 14, 15, 16, 18, 19** and/or **compound 20**".

16. The use as claimed in any of claims 1 to 15, where the disease and/or pathophysiological condition is selected from the group consisting of "aspergillosis, blastomycosis, candidiasis, chromoblastomycosis, coccidioidomycosis, cryptococcosis, dermatomycosis, dermatophytosis, histoplasmosis, lobomycosis, mucormycosis, mycetoma, mycotic keratitis, oculomycosis, onychomycosis, otomycosis, paracoccidiomycosis, phaeohyphomycosis, piedra, pityriasis versicolor, rhinosporidiosis, sporotrichosis, tinea barbae, tinea capitis, tinea corporis, tinea cruris, tinea favosa, tinea nigra, tinea pedis, tinea unguium, zygomycosis; African trypanosomiasis, American trypanosomiasis, amoebiasis, amoebic dysentery, amoebic keratitis, amoebic meningoencephalitis, amoebic vaginitis, babesiosis, chagas disease, coccidiosis, cryptosporidiosis, cutaneous leishmaniasis, cyclosporiasis, dientamoebiasis, entamoebiasis, giardiasis, isosporiasis, lambliasis, leishmaniasis, malaria, malaria quartana, malaria tertiana, malaria tropica, microsporidosis, mucocutaneous leishmaniasis, pneumocystosis, sarcosporidosis, sleeping sickness, toxoplasmosis, trichomoniasis, trypanosomiasis, visceral leishmaniasis, actimomycosis, acute epiglottitis, acute otitis media, acute purulent (septic) arthritis, acute purulent meningitis, anthrax, appendicitis, bacillary dysentery, bacteremia, black death, borderline leprosy, borreliose, botulism, breast abscesses, bronchitis, brucellosis, bubonic plague, carbuncles, cellulitis, cephalic tetanus, cerebritis, cervicitis, Cholera, conjunctivitis, cutaneous anthrax, cystitis, dermatitis, diarrhea, empyema, encephalitis, endocarditis, enteric fever, enteritis, enterocolitis, epididymitis, erysipelas, erysipelothricosis, exfoliation, extrapulmonary tubercolosis, food poisining, furuncles, gas gangrene, gastritis, gastroenteritis, gastrointestinal tract (GI) infections, gastrointestinal tubercolosis, genitourinary tubercolosis, glomerulonephritis, hematogenous or hymphohematogenous tuberculosis, impetigo, intra-abdominal infections, laryngitis, lepromatous leprosy, leprosy, leptospirosis, listeriosis, Localized tetanus, Lyme disease, mastitis, melioidosis, meningitis, meningoencephalitis, miliary tubercolosis, myonecrosis, nausea, necrotizing enteritis, neonatal listeriosis, neonatal sepsis, nocardiosis, ophthalmitis, osteomyelitis, osteomyelitis, otitis media, otitis, pancreatitis, parotitis, pericarditis, peritonitis, pertussis, Pestis, pharyngitis, plague, pneumonia, postpartum sepsis, primary listeremia, proctitis, prostatis, puerperal sepsis, pulmonary anthrax, pulmonary tubercolosis, Pustular or bullous skin subcutaneous abscesses, pyleonephritis, pyoderma, Rabbit/Deer Fly Fever, rat-bite fever, relapsing fever, rheumatic fever, rhinithis, rhomboencephalitis, salmonellosis, salpingitis, scalded skin syndrome, scarlet fever, sepsis, septic arthritis, septic thrombophlebitis, septicemia, shigellosis, sinusitis, skin infections, stitch abscesses, syphilis, tetanus, tick/recurrent/famine fever, tonsillitis, Toxic shock syndrome, tracheobronchitis, treponematosis, tubercolosis, tuberculoid leprosy, tuberculous lymphadenitis, tuberculous meningitis, tuberculous pericarditis, tuberculous peritonitis, tularemia, typhoid fever, ulcus, undulant/Malta/ Mediterranean/Gibraltar Fever, urethritis, urinary tract infections (UTIs), vomitting, wound infections, acute febrile respiratory disease (AFRD), acute hemorrhagic conjunctivitis, acute hemorrhagic cystitis, acute pharyngoconjunctival fever (APC), acute posterior ganglionitis, acute respiratory disease (ARD), AIDS, arbovirus encephalitis, aseptic meningitis, borna disease, Bornholm disease (pleurodynia), breakbone/dandy fever, bronchiolitis, bronchitis, Burkitt's lymphoma, California encephalitis, Castleman's disease, cervical cancer, chickenpox, Chikungunya disease, Colorado tick fever, common cold, conjunctivitis, Cowpox, Creutzfeldt-Jakob disease, Croup, cytomegalic inclusion disease, dengue, dengue hemorrhagic fever, Devil's grip (pleurodynia), Eastern equine encephalitis, Ebola hemorrhagic fever, Ebola virus infection, encephalomyelitis, epidemic keratoconjunctivitis (EKC), epidemic nephrosonephritis, erythema infectiosum, fatal familial insomnia, fifth disease, flue, foot and mouth disease (hand-foot-mouth disease), gastroenteritis, geniculate zoster, genital herpes, genital warts, German measles, Gerstmann-Straussier-Scheinker disease, gingivostomatitis, Hantaan-Korean hemorrhagic fever, hantavirus hemorrhagic fever, hantavirus pulmonary syndrome (HPS), hemorrhagic fever with renal syndrome (HFRS), hepatitis A, hepatitis B, hepatitis C, hepatitis D, hepatitis E, herpangina, herpes labialis, herpes zoster, herpetic stomatitis, HIV infection, Hodgkin's disease, HTLV-I-associated myelopathy, hydrophobia, infectious myocarditis, infectious pericarditis, influenza, Japanese encephalitis, jungle (sylvatic) yellow fever, Junin Argentinian hemorrhagic fever, Kaposi's sarcoma, keratitis, keratoconjunctivitis, Korean Hemorrhagic Fever, kuru, LaCrosse encephalitis, laryngitis, laryngotracheobronchitis (types 1 and 2), Lassa hemorrhagic fever, leukemia, lymphocytic choriomeningitis, lymphoma, Machupo Bolivian hemorrhagic fever, Marburg hemorrhagic fever, Mayaro disease, measles, meningoencephalitis, Molluscum contagiosum, mononucleosis, mononucleosis-like syndrome, multifocal leukoencephalopathy, mumps, nasopharyngeal carcinoma, nausea, neonatal herpes, nephropathia epidemica, ophthalmic herpes zoster, orchitis, orf, parainfluenza, parotitis, pharyngitis, pharyngoconjunctival fever, pleurodynia, pneumonia, polio, poliomyelitis, progressive multifocal leukencephalopathy (PML), rabies, roseola infantum, rubella, rubella panencephalitis, sclerosing panencephalitis, severe acute respiratory syndrome (SARS), shingles (zoster), slapped cheek disease (erythema infectiosum), smallpox, soeola, St. Louis encephalitis, temporal lobe encephalitis, tracheobronchitis, transmissable spongiform encephalopathies, tropical spastic paraparesis, urban yellow fever, urethritis, varicella, verrucae, vomitting, warts, Western equine encephalitis, Yellow fever, zona and/or zoster as well as their different forms and subforms" and preferably is selected from the group consisting of "trypanosomiasis, American trypanosomiasis, chagas disease, leishmaniasis, cutaneous leishmaniasis, mucocutaneous leishmaniasis, visceral leishmaniasis, malaria, malaria tropica, toxoplasmosis, acute otitis media, bronchitis, dermatitis, encephalitis, endocarditis, gastritis, gastroenteritis, gastrointestinal tract (GI) infections, laryngitis, meningitis, otitis media, pericarditis, pharyngitis, pneumonia, sepsis, sinusitis, skin infections, tuberculosis and/or urinary tract infections (UTIs) as well as their different forms and subforms".

17. The use of an alkyl phospholipid derivative selected from the group consisting of "**Compound 1, 2, 3, 4, 5, 6, 7, 8, 9, 10, 11, 12, 13, 14, 15, 16, 17, 18, 19, 20** and/or **compound 22**" for the manufacture of a medicament for the treatment of tumors in mammals.

18. The use as in any of claims 1 to 17, where the mammal is selected from the group consisting of "human, domestic animals, cattle, livestock, pets, cow, sheep, pig, goat, horse, pony, donkey, hinny, mule, hare, rabbit, cat, dog, guinea pig, hamster, rat, mouse" and preferably is human.

19. The use as claimed in any of claims 1 to 18, where such medicament comprises at least one additional pharmacologically active substance.

20. The use as claimed in any of claims 1 to 18, where the medicament is applied before and/or during and/or after treatment with at least one additional pharmacologically active substance.

21. The use as claimed in any of claims 19 to 20, where such additional pharmacologically active substance is selected from the group consisting of "benznidazole (N-Benzyl-2-nitroimidazol-1-yl-acetamide); nifurtimox [3-Methyl-4-(5-nitrofurfurylidenamino)tetrahydro-1,4-thiazine-1,1-dioxide]; amphotericin B ((1R, 3S, 5R, 6R, 9R, 11R, 15S, 16R, 17R, 18S, 19E, 21E, 23E, 25E, 27E, 29E, 31E, 33R, 35S, 36R, 37S)-33-[(3-Amino-3,6-dideoxy-beta-D-mannopyranosyl)oxy]-1, 3, 5, 6, 9, 11, 17, 37-octahydroxy-15,16,18-trimethyl-13-oxo-14,39-dioxabicyclo[33.3.1]-nonatriaconta-19, 21, 23, 25, 27, 29, 31-heptaene-36-carboxylic acid]; liposomal amphotericin B and/or paromomycin [O-2-amino-2-deoxy-alpha-D-glucopyranosyl-(1-4)-O-[O-2,6-diamino-2,6-dideoxy-beta-L-idopyranosyl-(1-3)-beta-D-ribofuranosyl-(1-5)]-2-deoxy-D-streptamine]".

22. The use as claimed in any of claims 19 to 21, where the alkyl phospholipid derivative is selected from the group consisting of "miltefosine (hexadecylphosphocholine), perifosine (octadecyl-1,1-dimethyl-piperidino-4-yl-phosphate) and/or erucylphosphocholine [(13Z)-docosenylphosphocholine]".

23. The use as claimed in any of claims 19 to 22, where the alkyl phospholipid derivative and the additional pharmacologically active substance are applied as a pharmaceutical kit.
